# FASCICULE DE BREVET EUROPEEN

(11) **EP 1 161 562 B1**
(45) Date de publication et mention de la délivrance du brevet: **27.05.2009**
(21) Numéro de dépôt: 00910960.4
(22) Date de dépôt: 16.03.2000
(51) Int. Cl.: C12Q 1/68, G01N 33/569

(54) **SEQUENCES DELETEES CHEZ M. BOVIS BCG/M. BOVIS OU M. TUBERCULOSIS, PROCEDE DE DETECTION DES MYCOBACTERIES UTILISANT CES SEQUENCES ET VACCINS**
DELETIERTE SEQUENZEN IN M. BOVIS BCG / M. BOVIS ODER M. TUBERCULOSIS, VERFAHREN ZUR ERKENNUNG VON MYCOBAKTERIEN MIT DIESEN SEQUENZEN UND VAKZINE
DELETED SEQUENCES IN M. BOVIS BCG/M. BOVIS OR M. TUBERCULOSIS, METHOD FOR DETECTING MYCOBACTERIA USING SAID SEQUENCES AND VACCINES

(30) Priorité: 16.03.1999 FR 9903250
(43) Date de publication de la demande: 12.12.2001
(73) Titulaire: INSTITUT PASTEUR, 75015 Paris (FR)
(72) Inventeur: COLE, Stewart, F-92140 Clamart (FR); GORDON, Stéphen, F-75015 Paris (FR); BUCHRIESER-BROSCH, Roland, F-75013 Paris (FR); BILLAULT, Alain, F-77690 Roissy-en-Brie (FR); GARNIER, Thierry, F-95120 Ermont (FR)
(74) Mandataire: Warcoin, Jacques
(86) Numéro de dépôt international: PCT/FR2000/000637
(87) Numéro de publication internationale: WO 2000/055362

(56) Documents cités:
- EP-A- 0 461 045
- WO-A-99/54487
- MAHAIRAS G G ET AL: "MOLECULAR ANALYSIS OF GENETIC DIFFERENCES BETWEEN MYCOBACTERIUM BOVIS BCG AND VIRULENT M. BOVIS" JOURNAL OF BACTERIOLOGY,US,WASHINGTON, DC, vol. 178, no. 5, - mars 1996 (1996-03) page 1274-1282 XP000647583 ISSN: 0021-9193 cité dans la demande
- M HARBOE ET AL: "B-cell epitopes and Quantification of ESAT-6 protein of Mycobacterium tuberculosis" INFECTION AND IMMUNITY,US,AMERICAN SOCIETY FOR MICROBIOLOGY. WASHINGTON, vol. 66, no. 2, - février 1998 (1998-02) page 717-723-723 XP002104630 ISSN: 0019-9567
- HARBOE ET AL: "Evidence for occurence of the ESAT-6 protein in Mycobacterium tuberculosis and virulent Mycobacterium bovis and for its absence in Mycobacterium bovis BCG" INFECTION AND IMMUNITY,US,AMERICAN SOCIETY FOR MICROBIOLOGY. WASHINGTON, vol. 64, no. 1, - janvier 1996 (1996-01) page 16-22-22 XP002105205 ISSN: 0019-9567 cité dans la demande
- LAGRANDERIE M.R.R. ET AL.,: "Comparison of immune response of mice immunized with five different mycobacterium bovis BCG vaccine strains" INFECTION AND IMMUNITY, vol. 64, no. 1, - janvier 1996 (1996-01) pages 1-9, XP002126619
- BROSCH ET AL: "USE OF A MYCOBACTERIUM TUBERCULOSIS H37Rv BACTERIAL ARTIFICIAL CHROMOSOME LIBRARY FOR GENOME MAPPING, SEQUENCING, AND COMPARATIVE GENOMICS" INFECTION AND IMMUNITY,US,AMERICAN SOCIETY FOR MICROBIOLOGY. WASHINGTON, vol. 66, no. 5, - mai 1998 (1998-05) page 2221-2229-2229 XP002104659 ISSN: 0019-9567 cité dans la demande
- PHILIPP W.J. ET AL.,: "Physical mapping of mycobacterium bovis BCG pasteur reveals differences from the genome map of mycobacterium tuberculosis H37Rv and from M.bovis" MICROBIOLOGY, vol. 142, - 1996 pages 3135-3145, XP002118720 cité dans la demande
- COLE S T ET AL: "Deciphering the biology of Mycobacterium tuberculosis from the complete genome sequence" NATURE,GB,MACMILLAN JOURNALS LTD. LONDON, vol. 393, - 11 juin 1998 (1998-06-11) page 537-544 XP002087941 ISSN: 0028-0836 cité dans la demande
- HARBOE M ET AL: "HOMOLOGY BETWEEN THE MPB70 AND MPB83 PROTEINS OF MYCOBACTERIUM BOVIS BCG" SCANDINAVIAN JOURNAL OF IMMUNOLOGY,GB,OXFORD, vol. 42, no. 1, - 1 juillet 1995 (1995-07-01) page 46-51 XP000611303 ISSN: 0300-9477
- HORWITZ M.A. ET AL.,: "Protective immunity against tuberculosis induced by vaccination with major extracellular proteins of mycobacterium tuberculosis" PROC. NATL. ACAD. SCI. USA, vol. 92, - février 1995 (1995-02) pages 1530-1534, XP002126618 cité dans la demande
- Gordon S.V. et al., Mol. Microbiol., vol. 32,3, 05-1999; 643-655 Identification of variable regions in the genomes of tubercle bacilli using bacterial artificial chromosome arrays XP002143568

## Description

La présente invention a pour objet la mise en évidence de séquences nucléotidiques permettant notamment de distinguer, en terme de diagnostic, une immunisation résultant d'une vaccination par le BCG d'une infection par *M. tuberculosis.* Les séquences en question sont spécifiques soit de *M. bovis* BCG/ *M. bovis,* soit de *M. tuberculosis.* La présente invention a également pour objet un procédé de détection des séquences en question, un procédé pour la détection d'anticorps générés par les produits d'expression de ces séquences ainsi que les kits pour mettre en oeuvre ces procédés. Enfin, la présente demande de brevet a pour objet de nouveaux vaccins.

Le taux important de mortalité et de morbidité causé par *Mycobacterium tuberculosis,* l'agent étiologique de la tuberculose, génère la nécessité de développer de nouveaux vaccins et des traitements chimio-thérapeutiques toujours plus courts. En effet, l'apparition de souches de *M. tuberculosis* résistantes aux anti-tuberculeux et le risque accru chez les patients immunodéprimés, par exemple chez les patients atteints du sida, de développer une tuberculose, rend nécessaire la mise au point de méthodes rapides, spécifiques et fiables pour le diagnostic de la tuberculose et la mise au point de nouveaux vaccins. Le vaccin BCG conventionnel dérive d'une souche de *Mycobacterium bovis* qui a été atténuée par des passages en série répétés sur de l'agar de bile pomme de terre-glycérinox (Calmette, 1927 ; Bloom and Fine, 1994). Cependant, malgré presque 50 ans d'utilisation mondiale, la raison de l'atténuation de *M. bovis* BCG est encore inconnue. Des questions persistent quant à la protection conférée par le vaccin contre la tuberculose pulmonaire, avec une efficacité comprise entre 0 et 80 % (Fine, 1994). De plus, de nombreuses sous-souches de BCG existent et offrent des niveaux variés de protection contre la tuberculose dans un modèle de souris (Lagranderie et al., 1996). L'atténuation de la souche d'origine de *M. bovis* peut avoir été causée par des mutations dans le génome du bacille qui ont été sélectionnées durant les passages en série de la souche, mutations qui sont restées stables dans le génome. Cependant, comme la souche de *M. bovis* d'origine est perdue, la comparaison directe de cette dernière avec *M. bovis* BCG est impossible. Malgré cela, l'identification de différences génétiques entre *M. bovis, M. bovis* BCG et *M. tuberculosis* est susceptible de révéler des localisations dont l'altération aurait pu conduire à l'atténuation de *M*. *bovis* BCG.

L'ADN de *M. tuberculosis* a plus de 99,9 % d'homologie avec l'ADN des autres membres du complexe tuberculeux (*M. bovis, M microti, M. africanum*). Bien que très apparentées, ces souches peuvent être différenciées sur la base de leur gamme d'hôte, de leur virulence pour l'Homme et de leurs caractéristiques physiologiques (Heifets and Good, 1994). Comme dans le cas de l'atténuation du BCG, la base génétique pour les différences phénotypiques entre les bacilles tuberculeux est largement inconnue. Cependant, la richesse de l'information contenue dans la séquence génomique de *M*. *tuberculosis* H37Rv donnait lieu à penser que les variations génétiques entre les souches allaient être mises à jour (Cole et al., 1998). La comparaison génomique présente un outil puissant pour de telles recherches puisque les génomes entiers peuvent être étudiés de préférence à l'étude des gènes dans leur individualité. Une précédente étude comparative de *M. bovis* et *M. bovis* BCG par hybridation génomique soustractive a montré que trois régions, désignées RD1, RD2 et RD3 étaient délétées dans *M. bovis* BCG comparativement à *M*. *bovis* (Mahairas et al., 1996). Cependant, le rôle, le cas échéant, de ces régions dans l'atténuation de *M. bovis* BCG n'a pas été clairement établi. De façon similaire, d'autres études des différences génomiques entre *M. bovis, M. bovis* BCG et *M. tuberculosis* ont montré que de nombreuses localisations polymorphiques existaient entre ces souches (Philipp et al., 1996). Bien que la nature exacte de ces polymorphismes n'ait pas été éclaircie, des analyses supplémentaires ont révélé qu'un polymorphisme était dû à la délétion de 12,7 kb dans *M. bovis* et BCG comparativement à *M*. *tuberculosis* (Brosch et al., 1998). A partir de là, il apparaît qu'il existe deux classes de délétion : celles qui sont absentes de BCG mais présentes dans *M. bovis* et *M. tuberculosis* et celles qui sont absentes de *M. bovis* et BCG mais présentes dans *M. tuberculosis.*

La banque de chromosome artificiel bactérien (BAC) de *M. tuberculosis* H37Rv déposée à la CNCM sous le n° 1-1945 le 19 novembre 1997. et décrite dans la demande WO9954487 témoigne de la connaissance complète de la séquence génomique de *M. tuberculosis* et présente un potentiel en tant qu'outil pour les applications postgénomiques telles que les comparaisons génomiques (Brosch et al., 1998). Afin de pousser plus avant les investigations dans les différences génomiques entre *M. tuberculosis* et *M. bovis* BCG, les inventeurs ont préparé une banque BAC de *M. bovis* BCG déposée le 30 juin 1998 à la CNCM sous le n° 1-2049 et décrite dans la demande WO9954487. Ce type de banque présente en effet certains avantages. Premièrement, le système BAC peut maintenir de larges inserts de DNA mycobactériens, jusqu'à 120 kb. Les 4,36 Mb du génome de *M. bovis* BCG pouvaient dont être représentées dans 50 à 60 clones, simplifiant le stockage et la manipulation de la banque. Deuxièmement, le système BAC peut permettre en toute confiance de répliquer les inserts sans générer de réarrangement ou de délétion dans les clones. De là, les altérations de l'insert ne peuvent pas être à l'origine d'erreur pour la variation dans le génome. Troisièmement, le positionnement des clones BAC sur le chromosome de *M. bovis* BCG est susceptible de générer une carte de clones se chevauchant ce qui devrait permettre la comparaison directe des segments locaux sur le génome de *M*. *tuberculosis* et de *M. bovis* BCG, tout en étant une ressource intéressante pour le séquençage du génome de *M. bovis* BCG.

La construction d'une banque BAC de *M. bovis* BCG-Pasteur (I-2049) est décrite ci-après ainsi que son utilisation, en conjonction avec la banque BAC de *M. tuberculosis* H37Rv (I-1945), en tant qu'outil pour les comparaisons génomiques. Avec cette approche, les inventeurs ont pu identifier de nouvelles délétions et insertions entre les bacilles tuberculeux ce qui permet d'avoir un aperçu dans deux génomes de la dynamique et de la différentiation dans le complexe de *M*. *tuberculosis.*

La voie principale pour extraire l'information biologique à partir du génome est la comparaison entre les génomes. La technologie des bio-puces ou « chips d'ADN » (Chee et al., 1996 ; DeRisi et al., 1997) décrite par exemple dans les brevets n° WO97/02357 et n° WO97/29212 permet d'effectuer les alignements et de sélectionner les séquences d'intérêt . Par ailleurs, la disponibilité d'un jeu minimum de clones de BAC pour les génomes de *M. bovis* BCG et *M. tuberculosis* H37Rv a procuré aux inventeurs des outils prêts à l'emploi pour les susdites études comparatives. La banque de BAC de *M. bovis* BCG contient plus de 1 500 clones avec une taille moyenne des inserts d'environ 75 kb. 57 clones couvrent le génome de BCG incluant un fragment *Hind*III de 120 kb qui était absent de la banque de BAC de *M. tuberculosis.* La construction des puces de BAC à partir de la banque de *M. bovis* BCG devait permettre aux inventeurs d'étendre leurs études comparatives concernant le bacille tuberculeux. Ces fragments peuvent s'hybrider avec de l'ADN génomique à partir d'isolats cliniques de *M*. *tuberculosis* ou des souches épidémiques pour identifier d'autres délétions ou réarrangements, et de là, permettre un nouvel aperçu concernant la plasticité du génome ainsi que l'identification des gènes et des produits des gènes qui peuvent être impliqués dans la virulence.

A l'issue d'expérimentations reportées ci-après, les inventeurs ont identifié 10 localisations ou loci qui sont absents dans *M. bovis* BCG comparativement à *M. tuberculosis.* Des hybridations avec l'ADN génomique de *M. bovis* ont révélé que 7 de ces loci étaient également délétés dans *M. bovis* comparativement à *M*. *tuberculosis.* Ainsi, dans le texte ci-après, à chaque fois qu'il sera question de caractéristiques communes au génome de *M. bovis* BCG et à celui de *M. bovis,* il sera indiqué qu'il s'agit du « génome de *M. bovis* BCG / *M. bovis* ».

Il s'est ensuite avéré que 3 des délétions spécifiques apparaissant dans *M*. *bovis* BCG étaient identiques aux régions RD1, RD2 et RD3 définies par l'équipe de Stover (Mahairas et al., 1996). Ainsi, en gardant la précédente nomenclature, les inventeurs ont appelé les 7 autres délétions du génome de *M. bovis* BCG / *M. bovis,* RD4, RD5, RD6, RD7, RD8, RD9 et RD10.

D'autres délétions se sont révélées être spécifiques du génome de *M. tuberculosis* étant entendu que les séquences « correspondantes » étaient présentes chez *M. bovis* BCG *l M. bovis ;* elles ont été appelées RvD1 et RvD2 (Tableaux 1 et 2).

Les délétions RD5-RD10, RvD1 et RvD2 ont permis aux inventeurs d'identifier de manière approfondie la dynamique du génome dans le bacille tuberculeux et ont donné des indications relatives aux bases génétiques de la différentiation phénotypique du complexe. L'identification de RvD1 et RvD2 en tant que délétions du génome de *M. tuberculosis* H37Rv montre que le processus de délétion ne fonctionne pas dans un seul sens, et la perte d'informations peut donc avoir lieu à la fois dans les souches bovines et dans les souches humaines. On constate que 8 des 10 délétions détectées sont localisées dans une région du chromosome où la terminaison de la réplication a probablement lieu.

Les inventeurs ont ensuite, au sein de chaque région délétée, mis en évidence plusieurs ORFs (ou cadres ouverts de lecture) ou gènes et ils ont tenté de déterminer la fonction putative de chacun d'eux (Tableau 1).

La présente demande décrit des séquences nucléotidiques délétées du génome de *M. bovis* BCG / *M. bovis* et présentes dans le génome de *M. tuberculosis* ou inversement choisies parmi les ORFs et gènes suivants : Rv2346c, Rv2347c, Rv2348c, *p*/*cC*, *plcb, p*/*cA,* Rv2352c, Rv2353c, Rv3425, Rv3426, Rv3427c, Rv3428c, Rv1964. Rv1965. *mce3,* Rv1967, Rv1968, Rv1969, *lprm,* Rv1971, Rv1972, Rv1973, Rv1974, Rv1975, Rv1976c, Rv1977, *ephA,* Rv3618, Rv3619c, Rv3620c, Rv3621c, Rv3622c, *lpqG, cobL,* Rv2073c, Rv2074, Rv2075, *echAl,* Rv0223c, RvD1-ORF1, RvD1-ORF2, RvD1-Rv2024c, RvD2-plcD RvD2-ORF1, RvD2-ORF2, RvD2-ORF3, RvD2-Rv1758.

Par « séquence nucléotidique » selon la présente invention, on entend aussi bien un ADN double brin, un ADN simple brin que des produits de transcription desdits ADN.

Plus particulièrement, les séquences nucléotidiques ci-dessus énumérées sont regroupées en régions nucléotidiques selon la répartition suivante :
- RD5: Rv2346c, Rv2347c, Rv2348c, *p*/*cC, plcB, plcA,* Rv2352c, Rv2353c,
- RD6 : Rv3425, Rv3426, Rv3427c, Rv3428c,
- RD7: Rv1964, Rv1965, *mce3,* Rv1967, Rv1968, Rv1969, *lprM,* Rv1971, Rv1972, Rv1973, Rv1974, Rv1975, Rv1976c, Rv1977, .
- RD8 : *ephA,* Rv3618, Rv3619c, Rv3620c, Rv3621c, Rv3622c, *lpqG,*
- RD9 : *cobL,* Rv2073c, Rv2074, Rv2075,
- RD10 : *echAl,* Rv0223c,
- RvD1:RvD1-ORF1,RvD1-ORF2, RvD1-Rv2024c
- RvD2 : RvD2-plcD, RvD2-ORF1, RvD2-ORF2, RvD2-ORF3, RvD2-Rv1758.

De façon intéressante, 3 des délétions (RD5, RD6 et RD8) contiennent 6 gènes codant pour des protéines PE et PPE. Comme il a été suggéré que ces protéines ont un rôle éventuel dans la variation antigénique (Cole et al., 1998), on peut en déduire que ces loci peuvent représenter des sites d'hypervariabilité entre les souches tuberculeuses.

Au moins 9 protéines susceptibles d'être exportées ou exposées à la surface sont codées par RD4 à RD10, ce qui indique que ces polypeptides ont peut-être un rôle important dans la reconnaissance immunitaire du bacille. Il a en effet été montré que des polypeptides sécrétés peuvent avoir un rôle de stimulateur potentiel dans le système immunitaire et ils sont susceptibles de jouer un rôle d'antigènes connus pour intervenir pendant l'étape précoce de l'infection (Elhay et al., 1998 ; Horwitz et al., 1995 ; Rosenkrands et al., 1998).

Le fait que RD5 et RD6 contiennent des gènes codant pour des protéines appartenant à la famille de ESAT-6 dont 14 sont organisées en 11 loci distincts est particulièrement significatif (F. Tekaia, S. Gordon, T. Garnier, R. Brosch, B.G. Barrell and S.T. Cole, soumis). ESAT-6 est un antigène à cellule T majeur qui semble être sécrété par le bacille tuberculeux virulent d'une manière indépendante du peptide signal (Harboe et al., 1996). Il s'accumule dans le milieu extracellulaire pendant les phases précoces de croissance et son gène est localisé dans RD1, une région qui est délétée du génome de *M. bovis* BCG (Mahairas et al., 1996 ; Philipp et al., 1996). 3 des 10 régions RD contiennent ainsi des gènes de la famille de ESAT-6, ce qui indique que d'autres sites de gènes ESAT-6 peuvent aussi donner lieu à des délétions ou des réarrangements.

La séquence génomique de *M. tuberculosis* H37Rv a par ailleurs révélé la présence de 4 gènes hautement apparentés codant pour des enzymes de phospholipase C appelées *plcA, plcB, plcC* et *p*/*cD* (Cole et al., 1998). La phospholipase C a été reconnue comme facteur de virulence important dans un certain nombre de bactéries incluant *Clostridium perfringens, Listeria monocytogenes* et *Pseudomonas aeruginosa* où elle joue un rôle intracellulaire dans la dissémination des cellules bactériennes, dans la survivance intracellulaire et dans la cytolyse (Titball, 1993). La délétion RD5 englobe 3 gènes (*plcA, plcB* et *plcC*), cette région étant absente de *M. bovis, M. bovis* BCG et *M. microti.* La mise en évidence de l'activité de la phopholipase C dans *M. tuberculosis, M. microti* et *M. bovis* mais pas dans *M*. *bovis* BCG, a été précédemment décrite (Johansen et al., 1996 ; Wheeler et Ratledge, 1992) ainsi que le rôle des enzymes codées par *plcA* et *plcB* (également connues sous la dénomination *mpcA* et *mpcB*) dans l'hydrolyse à la fois de la phosphatidylcholine et de la sphingomyéline. Les niveaux de l'activité de phospholipase C détectés dans *M. bovis* sont très inférieurs à ceux observés dans *M. tuberculosis* qui sont en concordance avec la perte de *plcABC,* l'activité de la sphingomyélinase étant encore détectable. Les données de séquences présentées ici montrent que la phospholipase dans sa pleine longueur est codée par le gène *plcD* dans *M. bovis* BCG-Pasteur et que son importante similarité de séquence avec les produits de *plcA* et *plcB* indique qu'elle est probablement dotée à la fois d'une activité phospholipase et d'un activité sphingomyélinase. Il est donc probable que *plcD* puisse être responsable de l'activité résiduelle de phospholipase C dans les souches présentant la délétion RD5, tel que *M. bovis,* bien qu'il soit difficile de relier cette interprétation à l'absence observée de phospholipase C en dépit de la présence de sphingomyélinase dans la souche de *M. bovis* BCG utilisée dans d'autres études (Johansen et al., 1996 ; Wheeler et Raledge, 1992). Les études de l'expression avec le gène *plcD* cloné devraient clarifier ce point.

Le gène *mce* a été décrit par l'équipe de Riley comme codant pour une protéine putative de *M. tuberculosis* de type invasine, dont l'expression dans *E*. *coli* permet l'invasion des cellules HeLa (Arruda et al., 1993). Trois autres protéines Mce ont été identifiées comme parties du projet de séquençage du génome avec leur gène occupant la même position dans quatre grands opérons très conservés comprenant au moins huit gènes (Cole et al., 1998 ; Harboe et al., 1996). Il est difficile de déduire les effets de la perte de *mce3* (RD7) sur *M. bovis, M. microti* et *M. bovis* BCG du fait que les trois copies de *mce* restantes pourraient complémenter toute perte d'activité, à moins que les opérons ne soient exprimés différentiellement. Cependant, il est intéressant de noter que RD7 est absent de certains membres du complexe de *M. tuberculosis* qui ne sont pas virulents pour l'Homme suggérant que RD7 puisse jouer un rôle spécifique dans la maladie humaine.

Le génome de *M. tuberculosis* H37Rv code également pour six protéines (EphA-F) qui montrent une similarité avec des hydrolases époxydes tandis qu'au moins 21 hydratases énoyle CoA (EchA1-21) et de multiples aldéhydes déshydrogénases sont présentes (Cole et al., 1998). La perte de *ephA* (RD8), *echAl* et l'aldéhyde déhydrogénase codée par Rv0223c (RD10) chez *M. bovis* BCG / *M bovis* peut donc être compensée par d'autres enzymes bien que la spécificité du substrat des enzymes de *M*. *tuberculosis* soit inconnue. Les hydrolases époxydes sont généralement considérées comme des enzymes de détoxification ; un récent rapport a encore montré qu'elles jouent un rôle dans l'activation des leucotoxines (Moghaddam et al., 1997), un acide gras toxique produit par les leucocytes qui sont impliqués dans le syndrome de détresse respiratoire chez l'adulte. Cependant, la question de savoir si les hydrolases époxydes de *M*. *tuberculosis* peuvent modifier chimiquement des chimiokines hôtes est sans réponse. Alternativement, elles peuvent jouer un rôle dans la détoxification lipidique des produits de peroxydation générés par les radicaux oxygènes à partir de macrophages activés.

RD9 est une région délétée des génomes de *M*. *africanum, M. bovis, M. bovis* BCG et *M*. *microti* comparativement à *M. tuberculosis.* En conséquence, en opposition avec les autres régions RD, la localisation de *M. africanum* est proche de *M*. *bovis,* ce qui indique la présence de cette souche entre *M. tuberculosis* et *M. bovis* (Heifets et Good, 1994). De façon similaire, la région D4 peut différencier *M. microti* des souches bovines (Tableau 2).

Les protéines codées par RD4 à RD10 peuvent donc présenter des antigènes intéressants, permettant la discrimination d'individus vaccinés par le BCG de patients infectés par *M. tuberculosis.*

La présente demande décrit également un procédé de détection et d'identification discriminante de *M. bovis BCG* et *M. bovis* de *M. tuberculosis* dans un échantillon biologique, comprenant les étapes suivantes:
a) isolement de l'ADN à partir de l'échantillon biologique à analyser ou obtention d'un ADNc à partir de l'ARN de l'échantillon biologique,
b) détection des séquences d'ADN de la mycobactérie présente dans ledit échantillon biologique,
c) analyse desdites séquences.

Ainsi la présente invention a pour objet un procédé de détection et d'identification permettant de discriminer *M. bovis* BCG et *M. bovis* de *M. tuberculosis* dans un échantillon biologique, comprenant les étapes suivantes :
a) isolement de l'ADN à partir de l'échantillon biologique à analyser ou obtention d'un ADNc à partir de l'ARN de l'échantillon biologique,
b) détection des séquences d'ADN de la mycobactérie présente dans ledit échantillon biologique,
c) comparaison desdites séquences avec les séquences nucléotidiques absentes des génomes de *M. bovis* BCG et de *M. bovis* et présentes dans le génome de *M*. *tuberculosis* choisies parmi les ORFs et gènes suivants : Rv2346c, Rv2347c, Rv2348c, *plcC, plcB, plcA,* Rv2352c, Rv2353c, Rv3425, Rv3426, Rv3427c, Rv3428c, Rv1964, Rv1965, *mce3,* Rv1967, Rv1968, Rv1969, *lprM,* Rv1971, Rv1972, Rv1973, Rv1974, Rv1975, Rv1976c, Rv1977, *ephA,* Rv3618, Rv3619c, Rv3620c, Rv3621c, Rv3622c, *lpgG, cobL,* Rv2073c, Rv2074. Rv2075c, *echAl,* Rv0223c, tels que définis dans le tableau 1.
d) comparaison desdites séquences avec les séquences nucléotidiques présentes dans les génomes de *M. bovis* BCG et de *M. bovis* et absentes du génome de *M. tuberculosis* choisies parmi les ORFs et gènes suivants : RvD1-ORF1, RvD1-ORF2, RvD1-Rv2024C, RvD2-plcD, RvD2-ORF1, RvD2-ORF2, RvD2-ORF3, RvD2-Rv1758, la séquence nucléotidique de RvD1 ayant été déposée sous le n° Y18605 et la séquence nucléotidique de RvD2 sous le n° Y18606 dans la base de données EMBL.

De préférence, les séquences nucléotidiques telles que définies aux étapes c) et d) sont regroupées en régions nucléotidiques RD5 à RD10 et RvD1 et RvD2 selon la répartition suivante :
- RD5: Rv2346c, Rv2347c, Rv2348c, *plcC, plcB, plcA,* Rv2352c, Rv2353c,
- RD6 : Rv3425, Rv3426, Rv3427c, Rv3428c,
- RD7 : Rv1964, Rv1965, *mce3,* Rv1967, Rv1968, Rv1969, *lprM,* Rv1971, Rv1972, Rv1973, Rv1974, Rv1975, Rv1976c, Rv1977,
- RD8 : *ephA,* Rv3618, Rv3619c, Rv3620c, Rv3621c, Rv3622c, *lpqG,*
- RD9 : *cobL,* Rv2073c, Rv2074, Rv2075c,
- RD10 : *echAl,* Rv0223c,
- RvD1 : RvD1-ORF1, RvD1-ORF2, RvD1-Rv2024c,
- RvD2 : RvD2-plcD, RvD2-ORF1, RvD2-ORF2, RvD2-ORF3, RvD2-Rv1758.

De préférence, dans le cadre de la présente invention, l'échantillon biologique est constitué par un fluide, par exemple du sérum humain ou animal, du sang, une biopsie, du liquide broncho-alvéolaire ou du liquide pleural.

L'analyse des séquences recherchées peut par exemple être réalisée par une électrophorèse sur gel d'agarose. Si l'on observe la présence d'un fragment d'ADN migrant à l'endroit attendu, on peut conclure que l'échantillon analysé contenait de l'ADN de mycobactérie. Cette analyse peut également être réalisée par la technique d'hybridation moléculaire en utilisant une sonde nucléique. Cette sonde sera avantageusement marquée par un élément non radioactif (sonde froide) ou radioactif.

Avantageusement, la détection des séquences d'ADN des mycobactéries sera réalisée au moyen de séquences nucléotidiques complémentaires desdites séquences d'ADN. A titre d'exemple, il pourra s'agir de sondes nucléotidiques marquées ou non marquées ; il pourra également s'agir d'amorces en vue d'une amplification.

La technique d'amplification utilisée peut être la PCR mais également d'autres techniques alternatives comme la technique SDA (Strand Displacement Amplification) ou technique d'amplification à déplacement de brins, la technique TAS (Transcription-based Amplification System), la technique NASBA (Nucleic Acid Sequence Based Amplification) ou la technique TMA (Transcription Mediated Amplification).

Les amorces conformes à l'invention ont une séquence nucléotidique choisie dans le groupe comprenant SEQ ID N° 1, SEQ ID N° 2, SEQ ID N° 3, SEQ ID N° 4, SEQ ID N° 5, SEQ ID N° 6, SEQ ID N° 7, SEQ ID N° 8, SEQ ID N° 9, SEQ ID N° 10, SEQ ID N° 11 SEQ ID N° 12, SEQ ID N° 13, SEQ ID N° 14, SEQ ID N° 15, SEQ ID N° 16, SEQ ID N° 17 et SEQ ID N° 18 avec:
- le couple SEQ ID N° 1 / SEQ ID N° 2 spécifique de RD4,
- le couple SEQ ID N° 3 / SEQ ID N° 4 spécifique de RD5,
- le couple SEQ ID N° 5 / SEQ ID N° 6 spécifique de RD6,
- le couple SEQ ID N° 7 / SEQ ID N° 8 spécifique de RD7,
- le couple SEQ ID N° 9 / SEQ ID N° 10 spécifique de RD8,
- le couple SEQ ID N° 11 1 SEQ ID N° 12 spécifique de RD9,
- le couple SEQ ID N° 13 / SEQ ID N° 14 spécifique de RD 10,
- le couple SEQ ID N° 15 / SEQ ID N° 16 spécifique de RvD1, et
- le couple SEQ ID N° 17 / SEQ ID N° 18 spécifique de RvD2,

Dans une variante, la demande décrit également un procédé de détection et d'identification discriminante de *M. bovis* BCG et *M*. *bovis* de *M. tuberculosis* dans un échantillon biologique comprenant les étapes suivantes :
a) mise en contact de l'échantillon biologique à analyser avec au moins un couple d'amorces tel que défini ci-dessus, l'ADN contenu dans l'échantillon ayant été, le cas échéant, préalablement rendu accessible à l'hybridation,
b) amplification de l'ADN de la mycobactérie,
c) mise en évidence de l'amplification des fragments d'ADN.

Les fragments amplifiés peuvent être identifiés par une électrophorèse en gel d'agarose ou de polyacrylamide, par une électrophorèse capillaire ou encore par une technique chromatographique (filtration sur gel, chromatographie hydrophobe ou chromatographie échangeuse d'ions). La spécification de l'amplification peut être contrôlée par hybridation moléculaire en utilisant des sondes, des plasmides contenant ces séquences ou leur produit d'amplification.

Les fragments nucléotidiques amplifiés peuvent être utilisés comme réactif dans des réactions d'hybridation afin de mettre en évidence la présence, dans un échantillon biologique, d'un acide nucléique cible de séquences complémentaires à celles desdits fragments nucléotidiques amplifiés.

Ces sondes et amplicons peuvent être marqués ou non par des éléments radioactifs ou par des molécules non radioactives telles que des enzymes ou des éléments fluorescents.

La présente invention a également pour objet un kit pour la détection et l'identification discriminante de *M. bovis* BCG et *M. bovis* de *M*. *tuberculosis* dans un échantillon biologique comprenant les éléments suivants :
a) au moins un couple d'amorces tel que défini ci-dessus,
b) les réactifs nécessaires pour effectuer une réaction d'amplification d'ADN,
c) éventuellement, les éléments nécessaires permettant de vérifier ou comparer la séquence et/ou la taille du fragment amplifié, par exemple des réactifs nécessaires à une électrophorèse ou une chromatographie, ou des sondes nécessaires à une hybridation moléculaire.

En effet, dans le cadre de la présente invention, en fonction du couple d'amorces utilisé, on peut obtenir des résultats très différents. Ainsi l'utilisation d'amorces internes à la délétion, telles qu'elles sont décrites dans la présente invention pour RD4, RD5 et RD8, fait qu'aucun produit d'amplification n'est détectable chez *M. bovis* BCG. Cependant, l'utilisation d'amorces externes à la zone de délétion ne donne pas nécessairement le même résultat, en ce qui concerne par exemple la taille du fragment amplifié, en fonction de l'importance de la zone délétée chez *M. bovis* BCG. Ainsi, l'utilisation du couple d'amorces SEQ ID N° 5 / SEQ ID N° 6 pour la détection de RD6 est susceptible de donner lieu à un amplicon chez *M. bovis* BCG d'environ 3 801 pb alors que la mise en oeuvre du couple d'amorces SEQ ID N° 11 / SEQ ID N° 12 pour la détection de RD9 donnera lieu chez *M. bovis* BCG à un amplicon d'environ 1 018 pb.

L'invention a également pour objet l'utilisation d'au moins un couple d'amorces tel que défini ci-dessus pour l'amplification de séquences d'ADN de *M. bovis* BCGet, *M. bovis* ou *M. tuberculosis.*

L'intérêt de l'utilisation de plusieurs couples d'amorces sera bien évidemment de croiser les résultats obtenus avec chacun d'eux pour affiner le résultat de l'analyse. En effet, lorsqu'il est indiqué, dans le cadre de la présente invention, que certaines délétions sont spécifiques de *M. bovis* BCG / *M. bovis,* cela n'est pas tout à fait exact puisque certaines d'entre elles sont également retrouvées chez *M. microti* OV254, chez *M. tuberculosis* CSU#93 et chez *M. africanum* ainsi que chez certains isolats cliniques (Tableau 2). Ainsi, l'utilisation du couple d'amorces SEQ ID N° 1 / SEQ ID N° 2 spécifique de la région RD4 ne donnera pas lieu à des amplicons de taille normale avec *M. bovis* BCG / *M. bovis* dans l'échantillon biologique. En revanche si le couple d'amorces utilisé est SEQ ID N° 5 / SEQ ID N° 6 spécifique de RD6 et que des amplicons de taille normale ne sont pas retrouvés, il ne sera pas possible, à partir de ce seul résultat, de discriminer entre la présence, dans l'échantillon biologique, de *M. bovis* BCG / *M. bovis, M. microti* OV254 et *M*. *tuberculosis* CSU#93.

La discrimination sera plus radicale quand il s'agira de déterminer si la mycobactérie présente dans l'échantillon biologique à analyser est *M. bovis* BCG / *M. bovis* ou *M*. *tuberculosis* H37Rv car les couplés d'amorces SEQ ID N° 15 / SEQ ID N° 16 et SEQ ID N° 17 / SEQ ID N° 18 ne sont spécifiques que de *M. tuberculosis* H37Rv. Par conséquent, l'absence d'amplicon de taille normale lors de l'utilisation de l'un ou l'autre de ces couples d'amorces pourra être considérée comme significative de la présence de *M. tuberculosis* H37Rv dans l'échantillon biologique analysé.

La présente demande décrit également les produits d'expression de tout ou partie des séquences nucléotidiques délétées du génome de *M. bovis* BCG / *M. bovis* et présentes dans *M. tuberculosis* ou inversement telles qu'énumérées dans le Tableau 1.

Par « produit d'expression » on entend toute protéine, polypeptide ou fragment polypeptidique résultant de l'expression de tout ou partie des susdites séquences nucléotidiques et de préférence présentant ou moins l'une des caractéristiques suivantes :
- capacité à exporter ou sécréter par une mycobactérie et ou être induit ou réprimé lors de l'infection par la mycobactérie, et/ou
- capacité à induire, réprimer ou moduler directement ou indirectement, un facteur de virulence de mycobactérie, et /ou
- capacité à induire une réaction d'immunogénicité dirigée contre une mycobactérie, et/ou
- capacité à être reconnu par un anticorps spécifique de mycobactérie.

En effet, la présente invention a également pour objet un procédé de détection discriminante *in vitro* d'anticorps dirigés contre *M. bovis* BCG et *M. bovis* ou d'anticorps dirigés contre *M. tuberculosis* dans un échantillon biologique, comprenant les étapes suivantes :
a) mise en contact de l'échantillon biologique avec au moins un produit d'expression de tout ou partie des séquences nucléotidiques absentes des génomes de *M. bovis* BCG et de *M. bovis* et présentes dans *M. tuberculosis* ou présentes dans les génomes de *M. bovis.* BCG et de *M. bovis* et absentes du génome de *M. tuberculosis* telles que définies aux étapes 1) et 2) ci après :
   1) comparaison desdites séquences avec les séquences nucléotidiques absentes des génomes de *M. bovis* BCG et de *M. bovis* et présentes dans le génome de *M. tuberculosis* choisies parmi les ORFs et gènes suivants : Rv2346c, Rv2347c, Rv2348c, *plcC, plcB, plcA,* Rv2352c, Rv2353c, Rv3425, Rv3426, Rv3427c, Rv3428c, Rv1964, Rv1965. *mce3,* Rv1967, Rv1968. Rv1969, *lprM,* Rv1971, Rv1972, Rv1973, Rv1974, Rv1975, Rv1976c, Rv1977, *ephA,* Rv3618, Rv3619c, Rv3620c, Rv3621c, Rv3622c, *lpqG, cobL,* Rv2073c, Rv2074, Rv2075c, *echAl,* Rv0223c, tels que définis dans le tableau 1, et
   2) comparaison desdites séquences avec les séquences nucléotidiques présentes dans les génomes de *M. bovis* BCG et de *M. bovis* et absentes du génome de *M. tuberculosis* choisies parmi les ORFs et gènes suivants: RvD1-ORF1, RvD1-ORF2, RvD1-Rv2024c, RvD2-plcD, RvD2-ORF1, RvD2-ORF2, RvD2-ORF3, RvD2-Rv1758, la séquence nucléotidique de RvD1 ayant été déposée sous le n° Y18605 et la séquence nucléotidique de RvD2 sous le n° Y18606 dans la base de données EMBL ;
b) mise en évidence du complexe antigène - anticorps formé.

De préférence les séquences nucléotidiques telles que définies à l'étape a) dudit procédé sont regroupées en régions nucléotidiques RD5 à RD10 et RvD1 et RvD2 selon la répartition suivante :
- RD5 : Rv2346c, Rv2347c, Rv2348c, *plcC, plcB, plcA,* Rv2352c, Rv2353c; tels que définis dans le tableau 1
- RD6 : Rv3425, Rv3426, Rv3427c, Rv3428c, tels que définis dans le tableau 1
- RD7 : Rv1964, Rv1965, *mce3,* Rv1967, Rv1968, Rv1969, *lprM,* Rv1971, Rv1972, Rv1973, Rv1974, Rv1975, Rv1976c, Rv1977, tels que définis dans le tableau 1
- RD8 : *ephA,* Rv3618, Rv3619c, Rv3620c, Rv3621c, Rv3622c, *lpqG,* tels que définis dans le tableau 1
- RD9 : *cobL,* Rv2073c, Rv2074, Rv2075c, tels que définis dans le tableau 1
- RD10 : *echAl,* Rv0223c, tels que définis dans le tableau 1
- RvD1 : RvD1-ORF1, RvD1-ORF2, RvD1-Rv2024c, la séquence nucléotidique de RvD1 ayant été déposée sous le n° Y18605 dans la base de données EMBL
- RvD2: RvD2-plcD, RvD2-ORF1, RvD2-ORF2, RvD2-ORF3, RvD2-Rv1758, la séquence nucléotidique de RvD2 ayant été déposée sous le n° Y18606 dans la base de données EMBL.

La demande décrit également un procédé de détection discriminante d'une vaccination avec *M. bovis* BCG ou d'une infection par *M. tuberculosis* chez un mammifère, comprenant les étapes suivantes :
a) préparation d'un échantillon biologique contenant des cellules, plus particulièrement des cellules du système immunitaire dudit mammifère et plus particulièrement encore, des cellules T,
b) incubation de l'échantillon biologique de l'étape a) avec au moins un produit d'expression conforme à la présente invention,
c) détection d'une réaction cellulaire indiquant une sensibilisation préalable du mammifère audit produit, notamment la prolifération cellulaire et/ou la synthèse de protéines telles que l'interféron gamma.

Ainsi, l'invention a également pour objet un procédé de détection *in vitro* permettant de discriminer une vaccination avec *M. bovis* BCG d'une infection par *M. tuberculosis* chez un mammifère, comprenant les étapes suivantes :
a) incubation de cellules dudit mammifère, plus particulièrement des cellules du système immunitaire dudit mammifère et plus particulièrement encore des cellules T avec au moins un produit d'expression de tout ou partie des séquences nucléotidiques absentes des génomes de *M. bovis* BCG et de *M*. *bovis* et présentes dans *M. tuberculosis* ou présentes dans les génomes de *M. bovis* BCG et de *M. bovis* et absentes du génome de *M. tuberculosis* telles que définies aux étapes 1) et 2) ci-après :
   1) comparaison desdites séquences avec les séquences nucléotidiques absentes des génomes de *M. bovis* BCG et de *M. bovis* et présentes dans le génome de *M*. *tuberculosis* choisies parmi les ORFs et gènes suivants : Rv2346c, Rv2347c, Rv2348c, *plcC, plcB, plcA,* Rv2352c, Rv2353c, Rv3425, Rv3426, Rv3427c, Rv3428c, Rv1964, Rv1965, *mce3,* Rv1967, Rv1968, Rv1969, *lprM,* Rv1971, Rv1972, Rv1973, Rv1974, Rv1975, Rv1976c, Rv1977, *ephA,* Rv3618, Rv3619c, Rv3620c, Rv3621c, Rv3622c, *lpgG, cobL,* Rv2073c, Rv2074, Rv2075c, *echAl,* Rv0223c, tels que définis dans le tableau 1, et
   2) comparaison desdites séquences avec les séquences nucléotidiques présentes dans les génomes de *M. bovis* BCG et de *M. bovis* et absentes du génome de *M. tuberculosis* choisies parmi les ORFs et gènes suivants : RvD1-ORF1, RvD1-ORF2, RvD1-Rv2024c, RvD2-*plcD*, RvD2-ORF1, RvD2-ORF2, RvD2-ORF3, RvD2-Rv1758, la séquence nucléotidique de RvD1 ayant été déposée sous le n° Y18605 et la séquence nucléotidique de RvD2 sous le n° Y18606 dans la base de données EMBL : et
b) détection d'une réaction cellulaire indiquant une sensibilisation préalable du mammifère audit produit, notamment la prolifération cellulaire et/ou la synthèse de protéines telles que l'interféron gamma.

De préférence, les séquences nucléotidiques telles que définies à l'étape a) dudit procédé sont regroupées en régions nucléotidiques RD5 à RD 10 et RvD1 et RvD2 selon la répartition suivante :
- RD5: Rv2346c, Rv2347c, Rv2348c, *plcC, plcB, plcA,* Rv2352c, Rv2353c, tels que définis dans le tableau 1
- RD6 : Rv3425, Rv3426, Rv3427c, Rv3428c, tels que définis dans le tableau 1
- RD7: Rv1964, Rv1965, *mce3,* Rv1967, Rv1968, Rv1969, *lprM,* Rv1971, Rv1972, Rv1973, Rv1974, Rv1975, Rv1976c, Rv1977, tels que définis dans le tableau 1
- RD8 : *ephA,* Rv3618, Rv3619c, Rv3620c, Rv3621c, Rv3622c, *lpqG,* tels que définis dans le tableau 1
- RD9 : *cobL,* Rv2073c, Rv2074, Rv2075c, tels que définis dans le tableau 1
- RD10 : *echAl,* Rv0223c, tels que défini dans le tableau 1
- RvD1 : RvD1-ORF1, RvD1-ORF2, RvD1-Rv2024c, la séquence nucléotidique de RvD1 ayant été déposée sous le n° Y18605 dans la base de données EMBL
- RvD2 : RvD2-*plcD,* RvD2-ORF1, RvD2-ORF2, RvD2-ORF3, RvD2-Rv1758, la séquence nucléotidique de RvD2 ayant été déposée sous le n° Y18606 dans la base de données EMBL.

La prolifération cellulaire pourra être mesurée par exemple par incorporation de ³H-Thymidine.

La demande décrit également un kit pour le diagnostic *in vitro* d'une infection par *M. tuberculosis* chez un mammifère éventuellement préalablement vacciné avec *M. bovis* BCG comprenant :
a) un produit d'expression conforme à la présente invention,
b) le cas échéant, les réactifs pour la constitution du milieu propice à la réaction immunologique,
c) les réactifs permettant la détection des complexes antigène - anticorps produits par la réaction immunologique,
d) le cas échéant, un échantillon biologique de référence (témoin négatif) dépourvu d'anticorps reconnus par ledit produit,
e) le cas échéant, un échantillon biologique de référence (témoin positif) contenant une quantité prédéterminée d'anticorps reconnus par ledit produit.

Ainsi, la présente demande décrit également un kit pour le diagnostic *in vitro* d'une infection par *M. tuberculosis* chez un mammifère éventuellement préalablement vacciné avec *M. bovis* BCG, comprenant :
a) un produit d'expression de tout ou partie des séquences nucléotidiques absentes des génomes de *M. bovis* BCG et de *M. bovis* et présentes dans *M. tuberculosis* ou présentes dans les génomes de *M. bovis* BCG et de *M. bovis* et absentes du génome de *M*. *tuberculosis* telles que définies aux étapes 1) et 2) ci-après :
   1) comparaison desdites séquences avec les séquences nucléotidiques absentes des génomes de *M. bovis* BCG et de *M. bovis* et présentes dans le génome de *M. tuberculosis* choisies parmi les ORFs et gènes suivants : Rv2346c, Rv2347c, Rv2348c, *plcC, plcB, plcA,* Rv2352c, Rv2353c, Rv3425, Rv3426, Rv3427c, Rv3428c, Rv1964, Rv1965, *mce3,* Rv1967, Rv1968, Rv1969, *lprM,* Rv1971, Rv1972, Rv1973, Rv1974, Rv1975, Rv1976c, Rv1977, *ephA,* Rv3618, Rv3619c, Rv3620c, Rv3621c, Rv3622c, *lpqG, cobL,* Rv2073c, Rv2074, Rv2075c, *echAl,* Rv0223c, tels que définis dans le tableau 1, et
   2) comparaison desdites séquences avec les séquences nucléotidiques présentes dans les génomes de *M. bovis* BCG et de *M. bovis* et absentes du génome de *M. tuberculosis* choisies parmi les ORFs et gènes suivants : RvD1-ORF1, RvD1-ORF2, RvD1-Rv2024c, RvD2-*plcD,* RvD2-ORF1, RvD2-ORF2, RvD2-ORF3, RvD2-Rv1758, la séquence nucléotidique de RvD1 ayant été déposée sous le n° Y18605 et la séquence nucléotidique de RvD2 sous le n° Y18606 dans la base de données EMBL,
b) le cas échéant, les réactifs pour la constitution du milieu propice à la réaction immunologique,
c) les réactifs permettant la détection des complexes antigène - anticorps produits par la réaction immunologique,
d) le cas échéant, un échantillon biologique de référence (témoin négatif) dépourvu d'anticorps reconnus par ledit produit,
e) le cas échéant, un échantillon biologique de référence (témoin positif) contenant une quantité prédéterminée d'anticorps reconnus par ledit produit.

De préférence les séquences nucléotidiques telles que définies dans la partie a) dudit kit sont regroupées en régions nucléotidiques RD5 à RD10 et RvD1 et RvD2 selon la répartition suivante :
- RD5: Rv2346c, Rv2347c, Rv2348c, *plcC, plcB, plcA,* Rv2352c, Rv2353c, tels que définis dans le tableau 1
- RD6 : Rv3425, Rv3426, Rv3427c, Rv3428c, tels que définis dans le tableau 1
- RD7: Rv1964, Rv1965, *mce3,* Rv1967, Rv1968, Rv1969, *lprM,* Rv1971, Rv1972, Rv1973, Rv1974, Rv1975, Rv1976c, Rv1977, tels que définis dans le tableau 1
- RD8 : *ephA,* Rv3618, Rv3619c, Rv3620c, Rv3621c, Rv3622c, *lpqG,* tels que définis dans le tableau 1
- RD9 : *cobL,* Rv2073c, Rv2074, Rv2075c, tels que définis dans le tableau 1
- RD10 : *echAl,* Rv0223c, tels que définis dans le tableau 1 RvD1 : RvD1-ORF1, RvD1-ORF2, RvD1-Rv2024c, la séquence nucléotidique de RvD1 ayant été déposée sous le n° Y18605 dans la base de données EMBL
- RvD2 RvD2-*plcD,* RvD2-ORF1, RvD2-ORF2, RvD2-ORF3, RvD2-Rv1758, la séquence nucléotidique de RvD2 ayant été déposée sous le n° Y18606 dans la base de données EMBL.

Les réactifs permettant la détection des complexes antigène - anticorps peuvent porter un marqueur ou être susceptibles d'être reconnus à leur tour par un réactif marqué, plus particulièrement dans le cas où l'anticorps utilisé n'est pas marqué.

L'invention a également pour objet des anticorps mono- ou polyclonaux, leurs fragments ou anticorps chimériques, capables de reconnaître spécifiquement un produit d'expression de tout ou partie des séquences nucléotidiques absentes des génomes de *M. bovis* BCG et de *M. bovis* et présentes dans *M. tuberculosis* ou présentes dans les génomes de *M. bovis* BCG et de *M. bovis* et absentes du génome de *M. tuberculosis* telles que définies aux étapes 1) et 2) ci-après :
1) comparaison desdites séquences avec les séquences nucléotidiques absentes des génomes de *M. bovis* BCG et de *M. bovis* et présentes dans le génome de *M. tuberculosis* choisies parmi les ORFs et gènes suivants : Rv2346c, Rv2347c, Rv2348c, *p*/*cC, plcB, plcA,* Rv2352c, Rv2353c, Rv3425, Rv3426, Rv3427c, Rv3428c, Rv1964, Rv1965, *mce3,* Rv1967, Rv1968, Rv1969, *lprM,* Rv1971, Rv1972, Rv1973, Rv1974, Rv1975, Rv1976c, Rv1977, *ephA,* Rv3618, Rv3619c, Rv3620c, Rv3621c, Rv3622c, *lpqG, cobL,* Rv2073c, Rv2074, Rv2075c, *echAl,* Rv0223c, tels que définis dans le tableau 1, et
2) comparaison desdites séquences avec les séquences nucléotidiques présentes dans les génomes de *M. bovis* BCG et de *M. bovis* et absentes du génome de *M. tuberculosis* choisies parmi les ORFs et gènes suivants : RvD1-ORF1, RvD1-ORF2, RvD1-Rv2024c, RvD2-*plcD*, RvD2-ORF1, RvD2-ORF2, RvD2-ORF3, RvD2-Rv1758, la séquence nucléotidique de RvD1 ayant été déposée sous le n° Y18605 et la séquence nucléotidique de RvD2 sous le n° Y18606 dans la base de données EMBL.

De préférence, lesdites séquences nucléotidiques sont regroupées en régions nucléotidiques RD5 à RD10 et RvD1 et RvD2 selon la répartition suivante :
- RD5 : Rv2346c, Rv2347c, Rv2348c, *plcC, plcb, plcA,* Rv2352c, Rv2353c, tels que définis dans le tableau 1
- RD6 : Rv3425, Rv3426, Rv3427c, Rv3428c, tels que définis dans le tableau 1
- RD7: Rv1964, Rv1965, *mce3,* Rv1967, Rv1968, Rv1969, *lprM,* Rv1971, Rv1972, Rv1973, Rv1974, Rv1975, Rv1976c, Rv1977, tels que définis dans le tableau 1
- RD8 : *ephA,* Rv3618, Rv3619c, Rv3620c, Rv3621c, Rv3622c, *lpqG,* tels que définis dans le tableau 1
- RD9 : *cobL,* Rv2073c, Rv2074, Rv2075c, tels que définis dans le tableau 1
- RD10 : *echAl,* Rv0223c, tels que définis dans le tableau 1
- RvD1 : RvD1-ORF1, RvD1-ORF2, RvD1-Rv2024c, la séquence nucléotidique de RvD1 ayant été déposée sous le n° Y18605 dans la base de données EMBL
- RvD2 : RvD2-*plcD,* RvD2-ORF1 , RvD2-ORF2, RvD2-ORF3, RvD2-Rv1758, la séquence nucléotidique de RvD2 ayant été déposée sous le n° Y18606 dans la base de données EMBL.

La présente invention concerne donc également un procédé pour la détection de la présence d'un antigène permettant de discriminer un antigène de *M. bovis* BCG et *M. bovis* d'un antigène de *M. tuberculosis* dans un échantillon biologique comprenant les étapes suivantes :
a) mise en contact de l'échantillon biologique avec un anticorps conforme à l'invention,
b) mise en évidence du complexe antigène - anticorps formé.

L'invention concerne également le kit pour la détection discriminante de la présence d'un antigène de *M. bovins* BCG et *M. bovis* par rapport à un antigène de *M. tuberculosis* dans un échantillon biologique comprenant les éléments suivants :
a) un anticorps conforme à l'invention,
b) les réactifs pour la constitution du milieu propice à la réaction immunologique,
c) les réactifs permettant la détection des complexes antigène - anticorps produits par la réaction immunologique.

Les réactifs ci-dessus mentionnés sont bien connus de l'homme du métier qui n'aura aucune difficulté à les adapter au contexte de la présente invention.

L'invention a également pour objet une composition immunogène caractérisée en ce qu'elle consiste en au moins un produit d'expression des séquences nucléotidiques absentes des génomes de *M*. *bovis* BCG et de *M. bovis* et présentes dans *M. tuberculosis* ou présentes dans les génomes de *M*. *bovis* BCG et de *M. bovis* et absentes du génome de *M. tuberculosis* telles que définies aux étapes 1) et 2) ci-après :
1) comparaison desdites séquences avec les séquences nucléotidiques absentes des génomes de *M*. *bovis* BCG et de *M. bovis* et présentes dans le génome de *M*. *tuberculosis* choisies parmi les ORFs et gènes suivants : Rv2346c, Rv2347c, Rv2348c, *p*/*cC, plcB, p*/*cA,* Rv2352c, Rv2353c, Rv3425, Rv3426, Rv3427c, Rv3428c, Rv1964, Rv1965, *mce3,* Rv1967, Rv1968, Rv1969, *lprM,* Rv1971, Rv1972, Rv1973, Rv1974, Rv1975, Rv1976c, Rv1977, *ephA,* Rv3618, Rv3619c, Rv3620c, Rv3621c, Rv3622c, *lpqG, cobL,* Rv2073c, Rv2074, Rv2075c, *echAl,* Rv0223c, tels que définis dans le tableau 1, et
2) comparaison desdites séquences avec les séquencesnucléotidiques présentes dans les génomes de *M. bovis* BCG et de *M. bovis* et absentes du génome de *M. tuberculosis* choisies parmi les ORFs et gènes suivants : RvD1-ORF1, RvD1-ORF2, RvD1-Rv2024c, RvD2 *plcD*, RvD2-ORF1, RvD2-ORF2, RvD2-ORF3, RvD2-Rv1758, la séquence nucléotidique de RvD1 ayant été déposée sous le n° Y18605 et la séquence nucléotidique de RvD2 sous le n° Y18606 dans la base de données EMBL.

De préférence, lesdites séquences nucléotides sont regroupées en régions nucléotidiques RD5 à RD10 et RvD1 et RvD2 selon la répartition suivante :
- RD5 : Rv2346c, Rv2347c, Rv2348c, *p*/*cC, plcB, plcA,* Rv2352c, Rv2353c, tels que définis dans le tableau 1
- RD6 : Rv3425, Rv3426, Rv3427c, Rv3428c, tels que définis dans le tableau 1
- RD7 : Rv1964, Rv1965, *mce3,* Rv1967, Rv1968, Rv1969, *lprM,* Rv1971, Rv1972, Rv1973, Rv1974, Rv1975, Rv1976c, Rv1977, tels que définis dans le tableau 1
- RD8 : *ephA,* Rv3618, Rv3619c, Rv3620c, Rv3621c, Rv3622c, /*pqG,* tels que définis dans le tableau 1
- RD9 : *cobL,* Rv2073c, Rv2074, Rv2075c, tels que définis dans le tableau 1
- RD10 : *echAl,* Rv0223c, tels que définis dans le tableau 1
- RvD1 : RvD1-ORF1, RvD1-ORF2, RvD1-Rv2024c, la séquence nucléotidique de RvD1 ayant été déposée sous le n° Y18605 dans la base de données EMBL
- RvD2 *:* RvD2-*plcD,* RvD2-ORF1, RvD2-ORF2, RvD2-ORF3, RvD2-Rv1758, la séquence nucléotidique de RvD2 ayant été déposée sous le n° Y18606 dans la base de données EMBL.

Avantageusement, la composition immunogène conforme à l'invention entre dans la composition d'un vaccin lorsqu'elle est présentée en association avec un véhicule pharmaceutiquement acceptable et éventuellement avec un ou plusieurs adjuvant(s) de l'immunité tel que l'alun ou un représentant de la famille des muramylpeptides ou encore d'adjuvant incomplet de Freund.

La présente demande décrit également un vaccin comprenant au moins un produit d'expression de tout ou partie des séquences nucléotidiques absentes des génomes de *M. bovis* BCG et de *M. bovis* et présentes dans *M. tuberculosis* ou présentes dans les génomes de *M. bovis* BCG et de *M. bovis* et absentes du génome de *M. tuberculosis* telles que définies aux étapes 1) et 2) ci-après :
1) comparaison desdites séquences avec les séquences nucléotidiques absentes des génomes de *M. bovis* BCG et de *M. bovis* et présentes dans le génome de *M. tuberculosis* choisies parmi les ORFs et gènes suivants : Rv2346c, Rv2347c, Rv2348c, *plcC, plcB, plcA;* Rv2352c, Rv2353c, Rv3425, Rv3426, Rv3427c, Rv3428c, Rv1964, Rv1965, *mce3,* Rv1967, Rv1968, Rv1969, *lprM,* Rv1971, Rv1972, Rv1973, Rv1974, Rv1975, Rv1976c, Rv1977, *ephA,* Rv3618, Rv3619c, Rv3620c, Rv3621c, Rv3622c, *lpqG, cobL,* Rv2073c, Rv2074, Rv2075c, *echAl,* Rv0223c, tels que définis dans le tableau 1, et
2) comparaison desdites séquences avec les séquences nucléotidiques présentes dans les génomes de *M. bovis* BCG et de *M. bovis* et absentes du génome de *M*. *tuberculosis* choisies parmi les ORFs et gènes suivants : RvD1-ORF1, RvD1 -ORF2, RvD1-Rv2024c, RvD2-*plcD*, RvD2-ORF1, RvD2-ORF2, RvD2-ORF3, RvD2-Rv1758, la séquence nucléotidique de RvD1 ayant été déposée sous le n° Y18605 et la séquence nucléotidique de RvD2 sous le n° Y 18606 dans la base de données EMBL,
en association avec un véhicule pharmaceutiquement compatible et, le cas échéant, un ou plusieurs adjuvants de l'immunité appropriés.

De préférence, lesdites séquences nucléotidiques sont regroupées en régions nucléotidiques RD5 à RD10 et RvD1 et RvD2 selon la répartition suivante :
- RD5: Rv2346c, Rv2347c, Rv2348c, *plcC, plcB, plcA,* Rv2352c, Rv2353c, tels que définis dans le tableau 1
- RD6 : Rv3425, Rv3426, Rv3427c, Rv3428c, tels que définis dans le tableau 1
- RD7: Rv1964, Rv1965, *mce3,* Rv1967, Rv1968, Rv1969, *lprM,* Rv1971, Rv1972, Rv1973, Rv1974, Rv1975, Rv1976c, Rv1977, tels que définis dans le tableau 1
- RD8 : *ephA,* Rv3618, Rv3619c, Rv3620c, Rv3621c, Rv3622c, *lpqG,* tels que définis dans le tableau 1
- RD9 : *cobL,* Rv2073c, Rv2074, Rv2075c, tels que définis dans le tableau 1
- RD10 : *echA1,* Rv0223c, tels que définis dans le tableau 1
- RvD1 : RvD1-ORF1, RvD1-ORF2, RvD1-Rv2024c, la séquence nucléotidique de RvD1 ayant été déposée sous le n° Y18605 dans la base de données EMBL
- RvD2 : RvD2-*plcD,* RvD2-ORF1 , RvD2-ORF2, RvD2-ORF3, RvD2-Rv1758, la séquence nucléotidique de RvD2 ayant été déposée sous le n° Y18606 dans la base de données EMBL.

Les connaissances standards sur l'évolution du complexe de *M. tuberculosis* sont basées sur l'hypothèse que *M. tuberculosis* est dérivé de *M. bovis* (Sreevatsan et al., 1997). Cependant, la distribution de RD1 à RD10 parmi le complexe tuberculeux suggère qu'une évolution linéaire de *M. tuberculosis* à partir de *M. bovis* est trop simpliste. Il apparaît en effet, de façon plus probable, que les deux bacilles sont dérivés d'une souche-mère commune, que les délétions reflètent donc l'adaptation des bacilles à leur niche particulière, c'est-à-dire que la perte de RD4 à RD10 a probablement aidé *M. bovis* à devenir un agent pathogène des bovins plus puissant que *M. tuberculosis.* Des études génomiques fonctionnelles détermineront quel rôle ces délétions jouent dans la différentiation phénotypique du complexe tuberculeux.

Enfin, les inventeurs ont mis en évidence, toujours par la comparaison du BAC de *M. tuberculosis* H37Rv et du BAC de *M. bovis* BCG deux duplications dans le génome de *M. bovis* BCG-Pasteur, appelées DU1 et DU2. Il s'agit de duplications de régions de plusieurs dizaines de kilobases qui semblent être absentes à la fois de la souche type de *M. bovis* et de *M. tuberculosis* H37Rv. La mise en évidence de ces deux duplications a été faite suite à une digestion des mêmes clones pour chaque BAC avec *Hind*III et une analyse sur gel d'électrophorèse en champ pulsé (PFGE). Ces observations ont été confirmées par hybridation de l'ADN chromosomique digéré issu de *M. bovis* BCG, de la souche type de *M*. *bovis* et *M. tuberculosis* H37Rv avec des sondes sélectionnées couvrant les régions dupliquées. Des amorces spécifiques pour les régions réarrangées ont été préparées et testées sur l'ADN génomique à partir d'isolats additionnels de *M. bovis* BCG et *M. tuberculosis.*

Il a été déterminé que DU1 et DU2 étaient présentes chez trois souches de *M. bovis* BCG y compris chez *M. bovis* BCG-Pasteur et absentes de trois autres sous-souches de *M. bovis* BCG.

Ces deux duplications sont également absentes de la souche type de *M. bovis* et de *M. tuberculosis* H37Rv.

Ainsi, toujours dans le cadre de la présente invention relativement à la détection discriminante de *M. bovis* ou *M. tuberculosis,* l'invention a également pour objet un procédé de détection et d'identification discriminante de *M. bovis* BCG ou *M. tuberculosis* dans un échantillon biologique comprenant les étapes suivantes :
- la digestion par une enzyme de restriction d'au moins une partie du génome de la mycobactérie présente dans l'échantillon biologique à analyser, et
- l'analyse des fragments de restriction ainsi obtenus.

La digestion par une enzyme de restriction peut en effet être réalisée soit sur l'intégralité du génome de la mycobactérie, soit sur un ou plusieurs clones de la banque réalisée à partir du génome en question.

Préférentiellement, l'enzyme de restriction utilisée dans le cadre du susdit procédé est *Hin*dIII.

En ce qui concerne l'analyse des fragments de restriction, celle-ci peut consister à compter lesdits fragments et/ou à déterminer leur longueur. En effet, comme ceci est expliqué ci-après, la digestion par *Hin*dIII de *M*. *bovis* BCG donne lieu à un fragment de plus que ceux obtenus après digestion par *Hin*dIII du génome de *M. tuberculosis* H37Rv. Le nombre des fragments ainsi obtenus peut également être complété par la détermination de leur longueur. Ceci peut être réalisé au moyen de techniques bien connues de l'homme du métier, par exemple sur un gel d'électrophorèse en champ pulsé (PFGE). Il a ainsi pu être déterminé que le fragment supplémentaire apparaissant après digestion par *Hin*dIII du génome de *M*. *bovis* BCG-Pasteur présentait une taille d'environ 29 kb.

Une autre façon d'analyser les fragments de restriction résultant de la digestion enzymatique du génome de la mycobactérie telle que ci-dessus décrite, consiste à mettre en contact lesdits fragments avec au moins une sonde appropriée, couvrant par exemple la région dupliquée, dans des conditions d'hybridation afin d'identifier ensuite le nombre et la taille des fragments qui ont hybridé. Les sondes utilisées à cette fin peuvent être marquées ou non marquées selon les techniques bien connues de l'homme du métier.

Ainsi, la sonde peut être obtenue par amplification de l'ADN génomique avec les amorces choisies dans le groupe SEQ ID N° 31, SEQ ID N° 32, SEQ ID N° 33 OU SEQ ID N° 34 avec le couple :
- SEQ ID N° 31 / SEQ ID N° 32 spécifique de DU 1
- SEQ ID N° 33 / SEQ ID N° 34 spécifique de DU2

On peut aussi analyser les fragments en effectuant une amplification des fragments obtenus avec des amorces choisies dans le groupe SEQ ID N° 19, SEQ ID N° 20, SEQ ID N° 21, SEQ ID N° 22, SEQ ID N° 23, SEQ ID N° 24, SEQ ID N° 25, SEQ ID N° 26, SEQ ID N° 27 et SEQ ID N° 28 avec :
- SEQ ID N° 19, SEQ ID N° 20 / SEQ ID N° 21 spécifiques de JDU1
- SEQ ID N° 22, SEQ ID N° 24 / SEQ ID N° 23, SEQ ID N° 25 spécifiques de JDU2A
- SEQ IDN ° 26 / SEQ ID N° 27, SEQ ID N° 28 spécifiques de JDU2B

Enfin, on peut également amplifier les fragments obtenus avec des amorces choisies dans le groupe SEQ ID N° 35, SEQ ID N° 36, SEQ ID N° 37 et SEQ ID N° 38 spécifiques de DU1 puis les analyser par séquençage.

### LEGENDES DES FIGURES

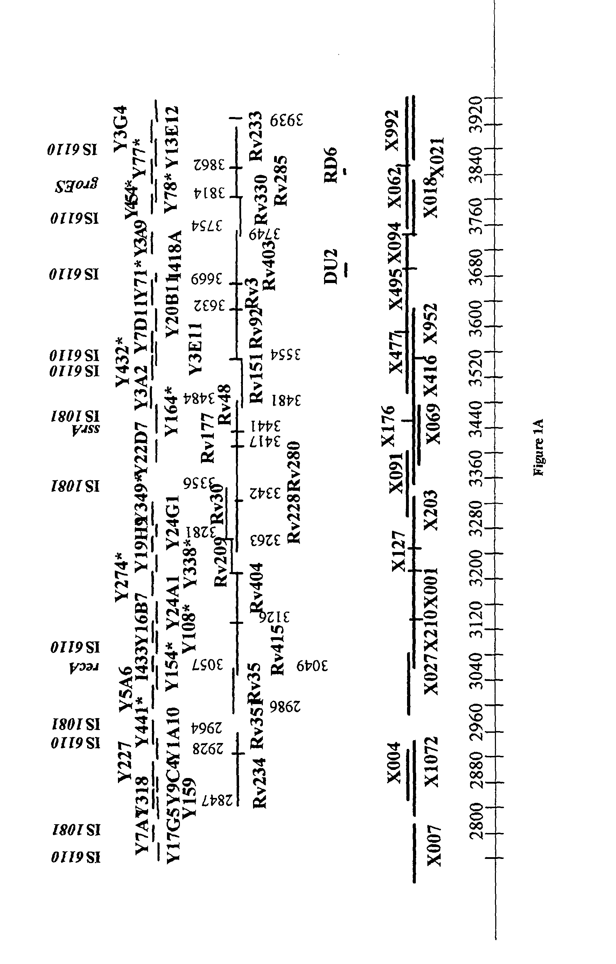

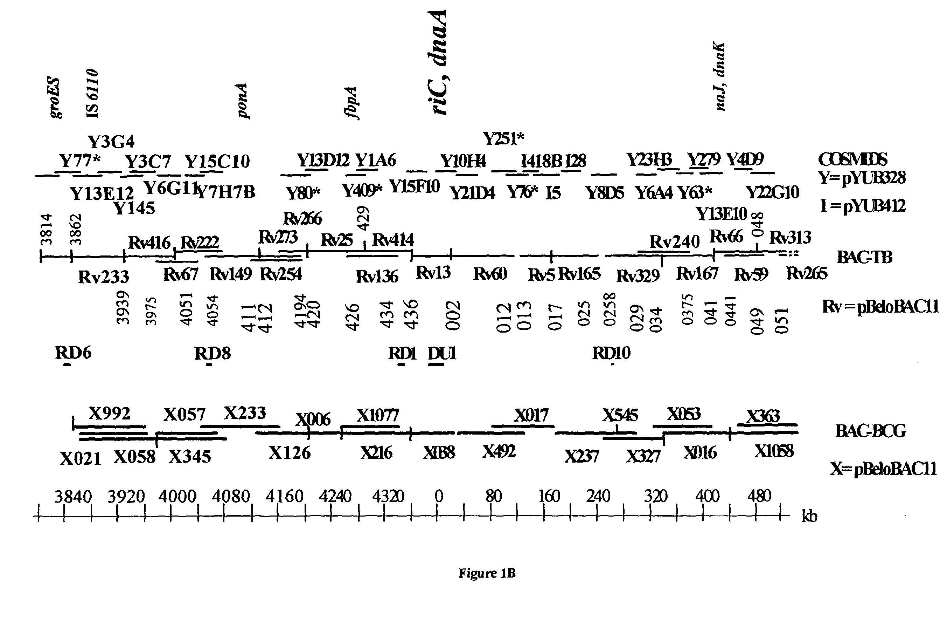

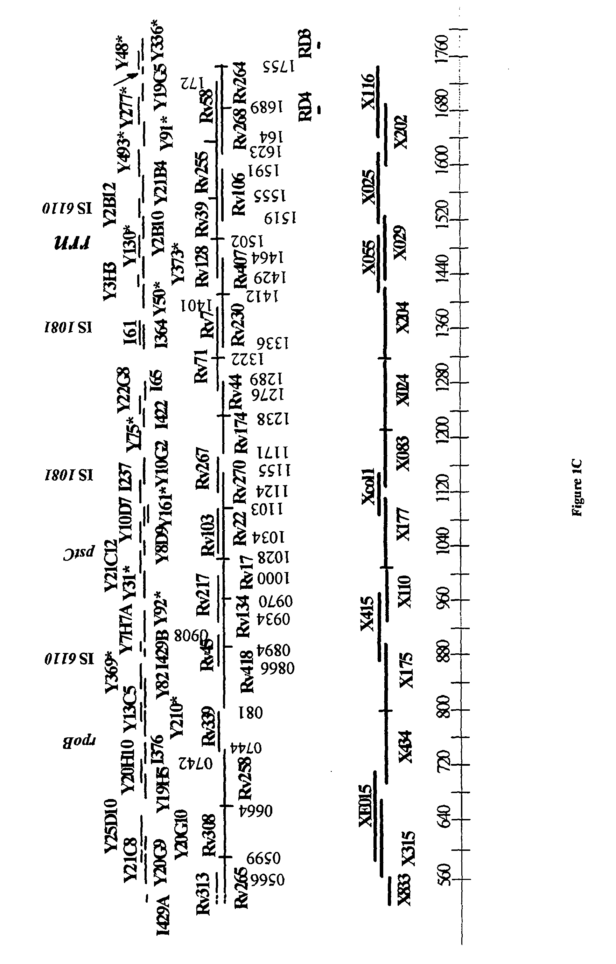

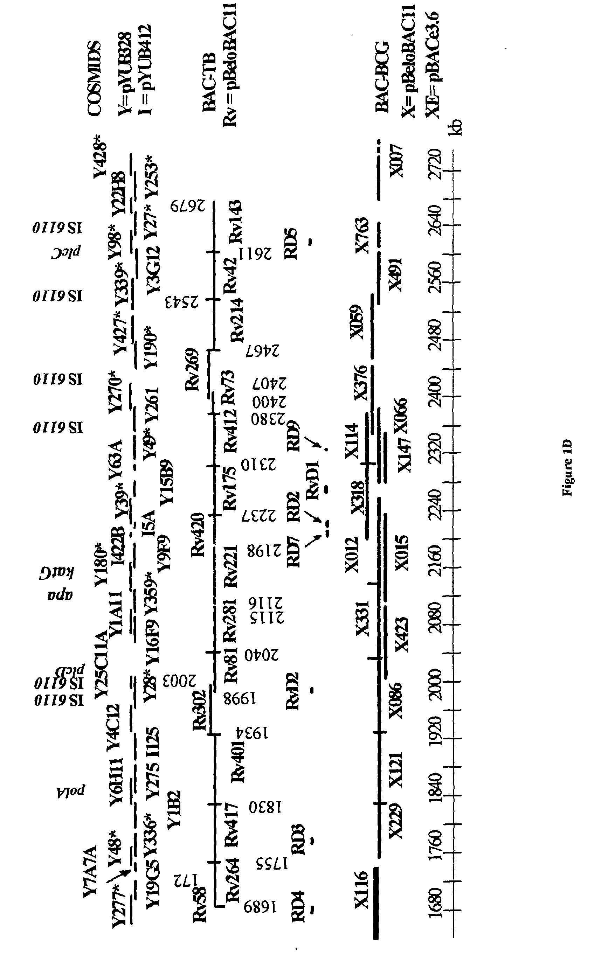

**FIGURES 1A à 1D**: Carte du BAC de *Mycobacterium bovis* BCG Pasteur superposée au BAC de *M. tuberculosis* H37Rv et aux cartes de cosmide (ces figures doivent se lire de gauche à droite et de haut en bas, la figure 1A en haut à gauche, la figure 1B en haut à droite, la figure 1C en bas à gauche et la figure 1D en bas à droite).

Les clones «X» correspondent aux clones dans pBeloBAC11 de *M*. *bovis* BCG, les clones « XE » correspondent aux clones dans pBACe3.6 de *M. bovis* BCG, les clones « Rv » correspondent aux clones dans pBeloBAC11 de *M. tuberculosis,* les clones « Y » correspondent aux clones dans le cosmide pYUB328 de *M. tuberculosis* et les clones « I » correspondent aux clones dans le cosmide pYUB412 de *M. tuberculosis.* La localisation de chaque région de délétion est montrée sur la carte. Les barres d'échelle indiquent la position sur le génome de *M. tuberculosis.*

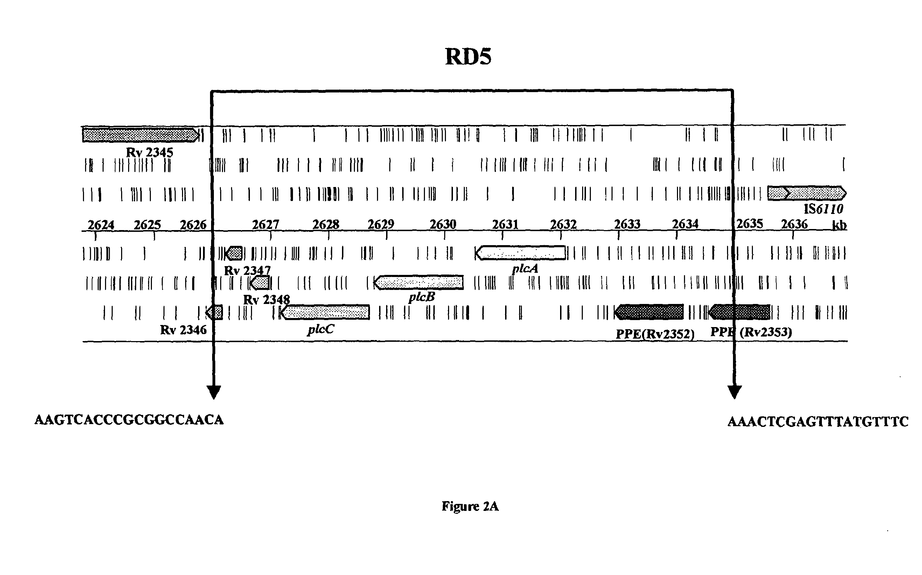

### FIGURES 2A à 2F : Vue générale des régions délétées RD5-RD10.

Les régions délétées à partir du génome de *M. tuberculosis* sont délimitées par des flèches avec une séquence flanquant chaque délétion. Les ORF (cadres ouverts de lecture) sont représentés par des boîtes « dirigées » montrant le sens de la transcription comme précédemment décrit (Cole et al., 1998). Les fonctions putatives et les familles des ORF sont décrites dans le Tableau 3. Les codons stop sont indiqués par de petites barres verticales.

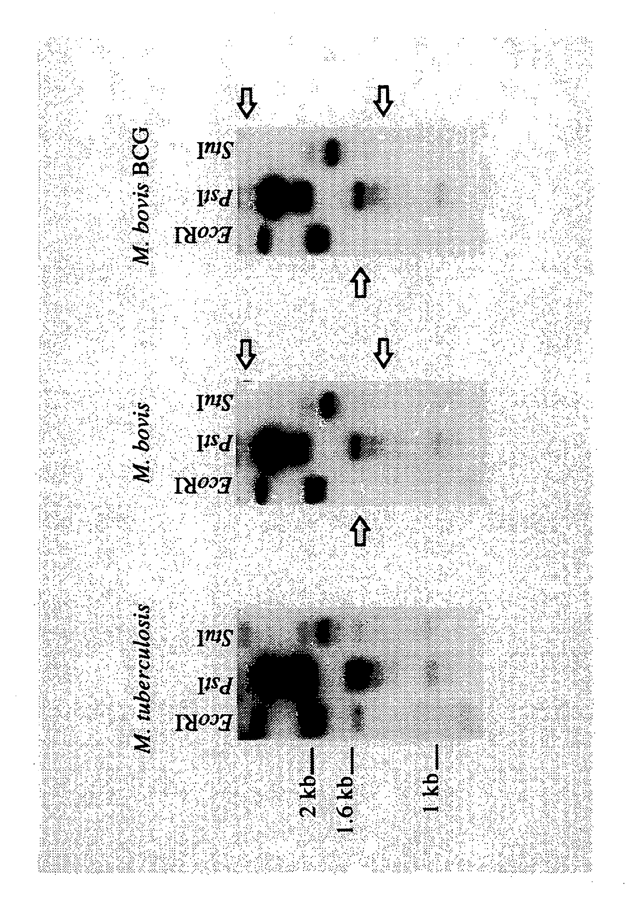

### FIGURE 3 : Détection de la délétion RD5.

Des digestions du clone Rv143 du BAC avec les endonucléases *Eco*RI*, Pst*I et *Stu*I ont révélé que des fragments de 1,5 kb (*Eco*RI), 1,5 kb (*Pst*I)*,* 1,3 et 2,7 kb (*Stu*I) ne montraient aucune liaison avec des sondes d'ADN de *M. bovis* ou de *M. bovis BCG* (les bandes manquantes sont indiquées par des flèches). La taille en kilobases (kb) est indiquée sur la gauche.

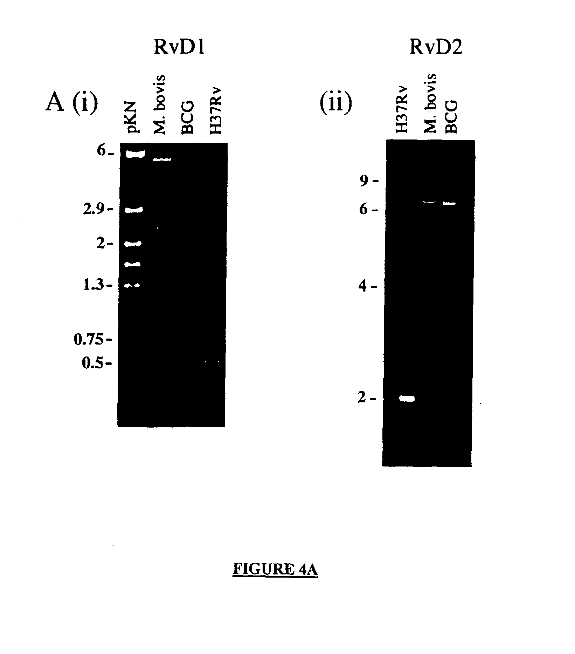

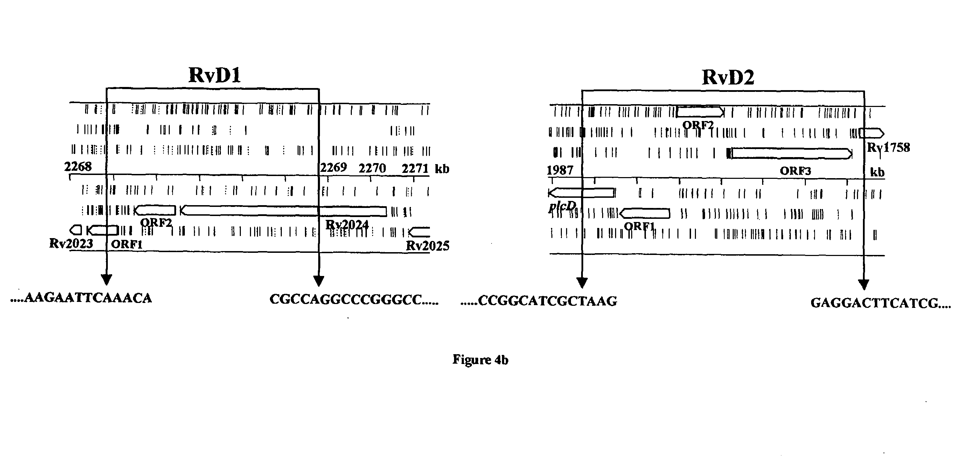

### FIGURE 4 : Les régions RvD1 et RvD2.

A. Polymorphisme de taille dans des amplicons générés par des amorces flanquant (i) RvD1 et (ii) RvD2. Des réactions de PCR ont été réalisées au moyen du kit GeneAmp XL PCR (Perkin Elmer) avec des matrices d'ADN de *M. tuberculosis* H37Rv, *M*. *bovis* et *M. bovis* BCG Pasteur en combinaison avec des amorces décrites dans le Tableau 3. La taille en kilobases est indiquée à gauche de chaque image.
B. Structure des ORF des loci de RvD1 et RvD2. La séquence des deux loci a été déterminée à partir de *M. bovis* BCG Pasteur, la séquence flanquante dans *M*. *tuberculosis* H37Rv étant montrée. Les fonctions putatives des ORF sont décrites dans le Tableau 1 avec des barres verticales représentant les codons stop.

### FIGURE 5 : Région dupliquée DU1 dans M. bovis BCG-Pasteur comparativement à la même région dans M. tuberculosis H37Rv

### FIGURE 6 : Région dupliquée DU2 dans M. bovis BCG-Pasteur comparativement à la même région dans M. tuberculosis H37Rv

La présente demande ne se limite pas à la description ci-dessus et sera mieux comprise à la lumière des exemples ci-après qui ne doivent en aucune manière être considérés comme limitant la présente invention.

### EXEMPLES

### I. PROCEDURES ET RESULTATS

### Construction d'une banques BAC de M. bovis BCG-Pasteur

Des tentatives précédentes pour cloner des inserts très larges d'ADN mycobactériens (120-180 kb) dans le vecteur pBeloAC11 se sont soldées par un échec (Brosch et al., 1998). Pour établir si cette détermination de taille était due au vecteur pBeloBAC11, les inventeurs ont testé en parallèle le vecteur pBACe3.6 de BAC qui utilise le système de sélection *sac*B (Lawes et Maloy, 1995 ; Pelicic et al., 1996). Des ligatures réalisées avec des fragments dans des gammes de taille de 50-125 kb ont donné 5 à 10 fois moins de transformants dans pBACe3.6 que les ligatures contrôle utilisant pBeloBAC11 (clones X). La taille d'un insert dans les clones pBACe3.6 était approximativement comprise dans l'intervalle 40-100 kb, similaire à ce qui avait été observé pour pBeloBAC11. Ceci suggère qu'une taille d'environ 120 kb est bien la taille limite supérieure pour la faisabilité du clonage d'ADN mycobactérien.

### Définition du jeu minimum des BACs de BCG

100 clones sélectionnés au hasard à partir des banques de pBeloBAC11 et de pBACe3.6 ont été séquences aux extrémités pour déterminer leur position par rapport au chromosome de *M. tuberculosis* H37Rv (Cole et al., 1998). Ceci a donné un réseau minimum de clones sur le génome mais avec un groupement préférentiel au voisinage de l'unique opéron *rrn,* ce qui a également été observé durant la construction de la carte du BAC de *M. tuberculosis* (Brosch et al., 1998). Pour combler les trous entre les clones positionnés, des amorces de PCR ont été élaborées, sur la base de la séquence du génome complet de *M. tuberculosis,* de façon à cribler des pools de BAC pour des clones spécifiques. En utilisant cette méthodologie, des clones couvrant plus de 98 % du génome ont été isolés et positionnés sur la séquence du génome de *M. tuberculosis.*

Un jeu minimum de 57 clones de BAC de *M. bovis* BCG a été nécessaire pour couvrir le génome (Figure 1). 56 de ces clones proviennent de la banque pBeloBAC11 et 1 provient de la banque de pBACe3.6, à savoir XE015 (à environ 680 kb). Du fait que des expériences antérieures avaient montré que les clones de *M. tuberculosis* basés sur pBeloBAC11 présentaient une exceptionnelle stabilité (Brosch et al., 1998), ces clones ont été préférés au système pBACe3.6 moins caractérisé. Le clone XE015 représente une région pour laquelle les clones de pBeloBAC11 ne pouvaient pas être trouvés. Deux zones d'environ 36-52 kb, couvertes par aucun clone, sont localisées à environ 2 660 kb et environ 2 960 kb sur le génome. Précédemment, l'isolement de cosmides et de clones de BAC de *M. tuberculosis* qui couvraient la région à environ 2 960 kb a posé des problèmes (Brosch et al., 1998) suggérant que cette région pourrait contenir des gènes qui sont préjudiciables à *E. coli.*

### Utilisation des puces BAC pour détecter des délétions dans le génome de M. bovis BCG

Il s'agit de la mise en évidence, à partir de la banque de BAC de *M. tuberculosis* H37Rv, de 63 clones couvrant 97 % du génome (Brosch et al., 1998). L'analyse *in silico* de la séquence du génome de *M. tuberculosis* n révélé que la digestion de ces clones avec soit *Pvu*II ou *Eco*RI donnait lieu à un nombre raisonnable de fragments de restriction pour chaque clone. Les fragments digérés ont migré à travers des gels d'agarose, ont donné lieu à des spots sur des membranes et ont ensuite été hybridés avec de l'ADN génomiques marqué au ³²P de *M*. *bovis* BCG et *M. bovis.* Les fragments de restriction qui n'ont pas hybridé avec les sondes d'ADN ont été considérés comme manquant dans les génomes de *M. bovis* ou BCG. Comme le criblage initial n'employait que deux enzymes, il est possible que d'autres délétions soient passées inaperçues. Cependant, il est probable que toutes les délétions importantes (> 5 kb) ont été détectées par cette approche.

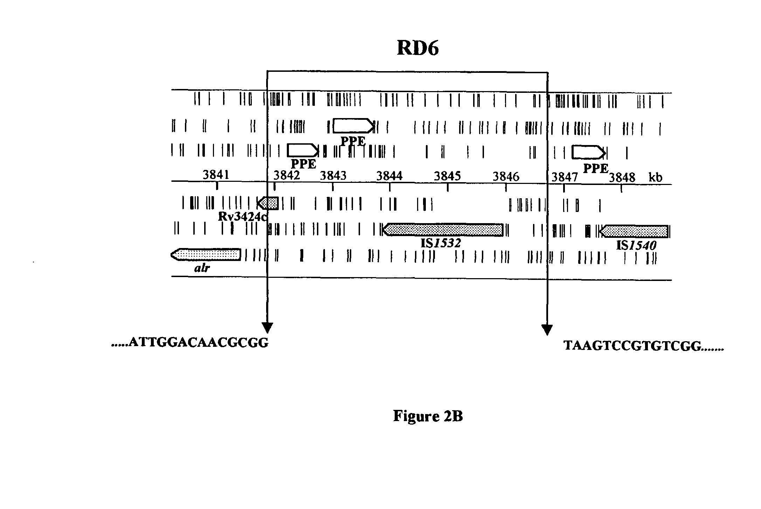

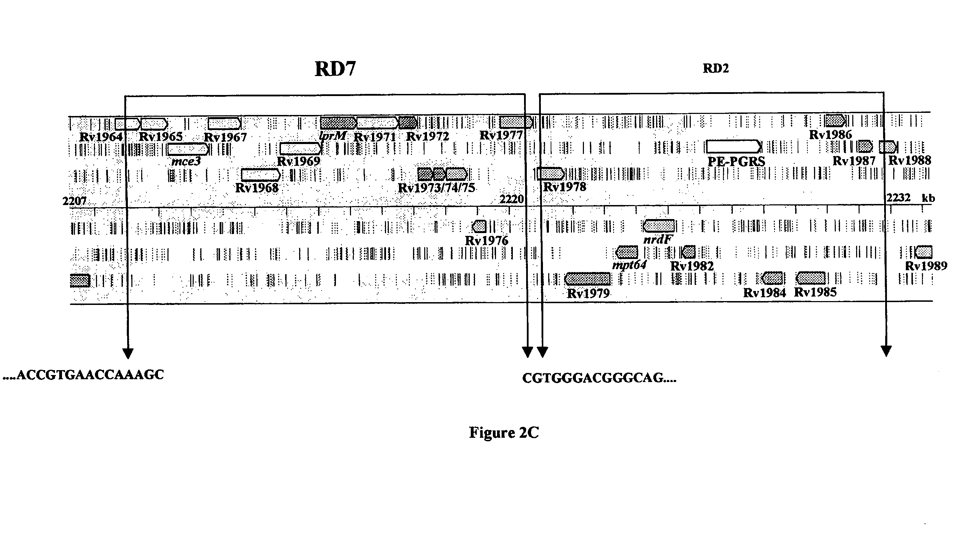

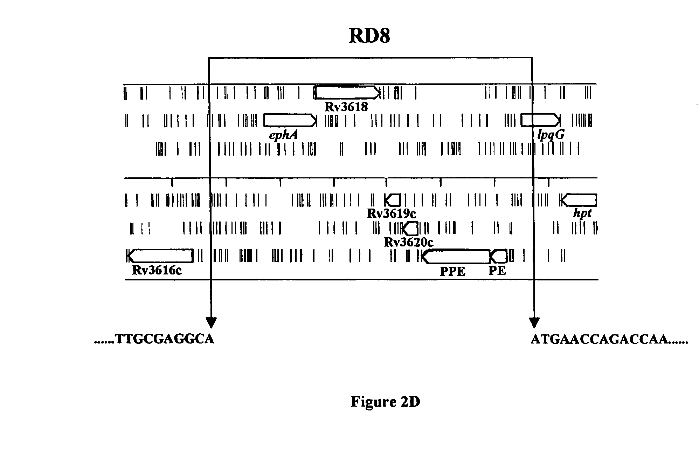

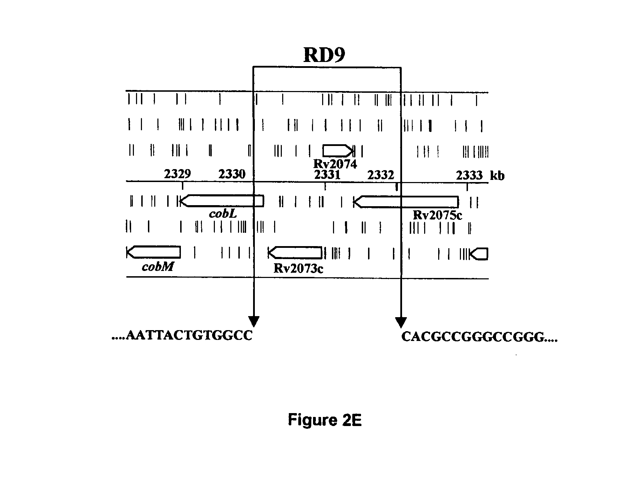

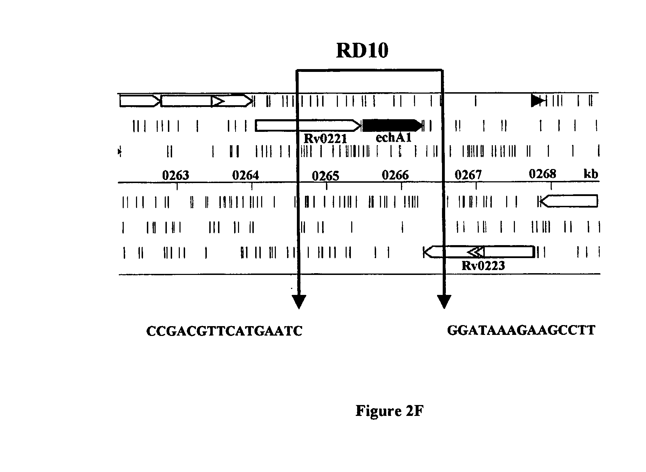

A partir d'une analyse de l'ensemble du génome, 10 loci ont été identifiés qui semblent être absents dans *M. bovis* BCG par rapport à *M. tuberculosis.* Des hybridations avec l'ADN génomique de M*. bovis* ont révélé que 7 de ces loci étaient également délétés dans *M. bovis* par rapport à *M. tuberculosis.* Une analyse plus serrée a révélé que les trois délétions spécifiques de *M. bovis* BCG étaient identiques aux régions RD1-RD3 définies par l'équipe de Stover (Mahairas et al., 1996). En gardant la précédente nomenclature, les 7 délétions *M. bovis* / BCG ont été désignées RD4, RD5, RD6, RD7, RD8, RD9 et RD10 (Figures 1 et 2). Les réactions de séquençage utilisant les clones de BAC correspondant en tant que matrice ont été utilisées pour définir précisément les zones terminales des délétions (Figure 2, Tableau 1).

### RD4

RD4 est une délétion de 12,7 kb précédemment caractérisée comme une région absente de *M. bovis* et *M. bovis* BCG des sous-souches Pasteur, Glaxo et Danemark (Brosch et al., 1998). Parmi les protéines codées par les 11 ORFs, certaines montrent des ressemblances avec des enzymes impliquées dans la synthèse des lipopolysaccharides. Pour déterminer si RD4 était délétée seulement dans les souches bovines. *M*. *africanum, M. microti, M. tuberculosis* CSU#93 et 27 isolats cliniques de *M. tuberculosis* ont été examinés pour la présence du locus (Tableau 2). Des réactions de PCR utilisant des amorces internes à RD4 (Tableau 3) n'ont généré que des produits dans des souches non bovines.

### RD5

RD5 a une taille de 8 964 pb localisées entre les positions génomiques 2626067-2635031 (Figure 3, Tableau 1). La région contenait 8 ORFs (Tableau 1), trois d'entre elles : *plcA, p*/*cB* et *p*/*cC,* codent pour des enzymes de phospholipase C tandis que deux autres codent pour des protéines appartenant respectivement aux familles de ESAT-6 et QILSS (Cole et al., 1998 ; F. Tekaia, S. Gordon, T. Garnier, R. Brosch, B.G. Barrell et S.T. Cole, soumis). L'ORF Rv2352c code pour une protéine PPE qui est un membre de la grande famille des protéines dans *M. tuberculosis* (Cole et al., 1998). Une autre protéine de la famille de PPE (Rv2352c) est tronquée dans *M. bovis* BCG du fait que l'une des délétions des parties terminales est située dans l'ORF. Des recherches dans les bases de données ont révélé qu'un segment de 3 013 pb de RD5 était virtuellement identique au locus *mpt40* précédemment décrit, montré par Pattaroyo et al. comme étant absent chez *M*. *bovis* et *M. bovis* BCG (Leao et al., 1995). Des amorces destinées à amplifier la partie interne de RD5 (Tableau 3) ont été utilisées dans des réactions de PCR avec de l'ADN issu de bacilles tuberculeux variés. Aucun amplicon n'a été produit à partir des matrices de *M. bovis, M. bovis* BCG et *M. microti* (Tableau 2) indiquant que *M. microti* est également dépourvu du locus RD5.

### RD6

RD6 a été cartographiée au niveau de la séquence d'insertion *IS1532.* un élément IS qui est manquant chez *M. microti, M. bovis* et *M. bovis* BCG (Gordon et al., 1998) (Tableau 1). La délimitation de la taille de la délétion a été compliquée par la présence de régions répétées flanquant directement l'élément IS et nécessitant l'utilisation d'amorces en dehors de la région répétée (Tableau 3). Ces amorces ont amplifiées des produits chez *M. bovis* et *M. bovis* BCG qui sont plus petits d'environ 5 kb que l'amplicon de *M. tuberculosis.* La marche à l'aide d'amorce a été utilisée pour localiser précisément les jonctions de délétions et a révélé une délétion de 4 928 b chez *M. bovis* et *M*. *bovis* BCG (position génomique de *M. tuberculosis* 3846807-3841879). A l'instar de l'élément IS*1532*, il a été déterminé que RD6 contenait deux gènes codant pour des protéines PPE (Rv3425 et Rv3426) et une partie de Rv3424c dont la fonction est inconnue (Tableau 1).

### RD7

La délétion RD2 décrite dans Mahairas et al. (Mahairas et al., 1996) a été cartographiée dans le clone Rv420 de *M. tuberculosis* et les résultats obtenus par les inventeurs ont suggéré l'existence d'une délétion supplémentaire chez *M*. *bovis* BCG très proche de RD2. Des hybridations ont été répétées utilisant de l'ADN génomique de *M. bovis* en tant que sonde puisque cette souche contient des séquences RD2, simplifiant ainsi l'identification d'autres fragments délétés. Cette analyse (Figure 2) a révélé une délétion de 12 718 pb chez *M. bovis* BCG par rapport à *M. tuberculosis,* localisée 336 pb en amont de RD2, aux positions 2208003-2220721 sur le génome de *M. tuberculosis.* La région RD7 contient 14 ORFs (Tableau 3). 8 d'entre eux (Rv1964-1971) constituent une partie de l'opéron avec le gène putatif d'invasine *mce3* (Cole et al., 1998). Les ORFs Rv1968, Rv1969, Rv1971, Rv1973 et Rv1975 pourraient coder pour d'éventuelles protéines exportées ou exprimées à la surface puisqu'ils contiennent des séquences signal N-terminales putatives ou des ancrages membranaires. Elles sont toutes membres de la famille Mce et ont des propriétés communes (Tekaia et al., soumis). De façon intéressante, *Mce3* et Rv1968 contiennent le tripeptide « RGD » ou Arg-Gly-Asp, un motif impliqué dans l'attachement cellulaire (Ohno, 1995 ; Relman et al., 1989). Rv1977, qui est tronqué par RD7, code pour une protéine présentant des similarités (38,5 % d'identité sur 275 acides aminés) avec un polypeptide hypothétique et la souche PCC 6803 de *Synechocystis.* Une analyse PCR (Tableau 2) a révélé que RD7 était présent dans 30 isolats cliniques de *M. tuberculosis* ainsi que dans *M*. *africanum* et *M. tuberculosis* CSU#93. Le locus était cependant absent de *M. microti, M. bovis* et *M bovis* BCG.

### RD8

RD8 englobe une région de 5 895 pb positionnée sur la séquence génomique de *M. tuberculosis* à 4556836-4062731. La délétion contient 6 ORFs (Figure 2, Tableau 1) avec un septième ORF : *lpqQ* qui code pour une lipoprotéine tronquée à son extrémité 5' par la délétion. Parmi ces 6 ORFs, Rv3619c et Rv3620c codent pour des membres des familles de ESAT-6 et QILSS (Cole et al., 1998, Harboe et al., 1996 ; F. Tekaia, et al., soumis) et 2 autres ORFs codent pour des protéines PE et PPE. Les 2 autres ORFs, *ephA* et Rv3618, codent respectivement pour une hydrolase époxyde putative et une monooxygénase. L'analyse PCR dirigée contre un segment interne de RD8 (Tableau 2) a révélé que la région était également délétée chez le type sauvage de *M. bovis* et *M. microti.*

### RD9 et RD10

La délétion de 2 030 pb englobée par RD9 couvre 2 ORFs, Rv2037c et Rv2074, qui codent vraisemblablement respectivement pour une oxydoréductase et une protéine inconnue (Tableau 1). 2 ORFs additionnels sont tronqués par RD9: Rv2075c code pour une protéine exportée putative tandis que *cobL* code pour une méthyltransférase précorrine impliquée dans la synthèse de la cobalamine. L'analyse PCR avec des amorces flanquantes (Tableau 3) a révélé que RD9 est également présent chez *M. africanum* et *M. microti* (Tableau 2). RD10 est une délétion de 1 903 pb qui tronque 2 ORFs, *echA1* et Rv0223, qui codent une énoyl CoA hydratase et une aldéhyde déshydrogénase respectivement (Tableau 1). Des réactions de PCR ont révélé que RD10 était absent chez *M. microti* ainsi que chez *M. bovis* et BCG.

### D'autres différences entre M. tuberculosis et BCG

Compte tenu du fait que les génomes des bacilles tuberculeux sont hautement conservés (Sreevatsan et al., 1997), la comparaison locale directe peut être entreprise d'une façon simple et ciblée en examinant les profils d'enzyme de restriction générés à partir des clones de BAC des *M. tuberculosis* et *M. bovis* BCG qui couvrent les mêmes régions. Une cartographie comparative de la zone englobée par le clone X318 a identifié cette zone comme étant très différente des clones correspondants de *M. tuberculosis.* Les données relatives aux séquences terminales à partir du clone X066 ont révélé que si sa séquence terminale SP6 permettait de le positionner à environ 2 380 kb sur la matrice de *M. tuberculosis,* la séquence terminale T7 ne générait aucune similarité significative avec une quelconque séquence de H37Rv, indiquant qu'une extrémité de X066 était interne au segment d'ADN présent dans BCG mais absent de H37Rv. Des amorces de séquençage ont été utilisées pour marcher le long du clone X318 du BAC de BCG (Figure 1) et ont révélé l'insertion à la position 2238724 pb dans le génome de *M. tuberculosis.* Utilisées dans des réactions PCR, les matrices de *M. bovis* BCG et *M. bovis* ont généré des amplicons plus grands d'environ 5 kb que le produit de *M. tuberculosis* H37Rv (Figure 4A). L'insert entier, désigné RvD1, a été séquencé à partir de X318 BCG. L'insert de 5 014 pb prolongait l'ORF, Rv2024c de *M*. *tuberculosis* de 2,8 kb et contenait un ORF additionnel, RvD1-ORF2, de 954 pb (Tableau 1, Figure 4B). RvD1-ORF1 se superpose au point de jonction 5' de la délétion et s'étend à l'intérieur du DNA flanquant. L'analyse FASTA a révélé que RvD1-ORF1 et l'ORF2 codent pour des protéines ne présentant aucune similarité significative avec d'autres protéines dans les bases de données. Rv2024c étendu a montré certaines similarités (36,5 % d'identité sur 946 acides aminés) avec une protéine hypothétique d'*Helicobacter pylori* (n° d'accession 025380). La perte de cette séquence n'a clairement aucune conséquence sur la virulence de *M. tuberculosis* H37Rv puisque cette souche est pleinement virulente chez les modèles animaux. Une analyse PCR spécifique du locus a démontré sa présence dans plusieurs mais pas dans tous les isolats cliniques ainsi que dans toutes les souches BCG testées (Tableau 2).

Un ORF codant pour une phopholipase, *plcD,* est interrompu par *IS6110* dans *M. tuberculosis* H37Rv (Cole et al., 1998). Pour déterminer si *plcD* était intact dans d'autres membres du complexe tuberculeux, des amorces qui flanquaient le site d'insertion *IS6110* (Tableau 3) ont été utilisées dans des réactions de PCR avec *M. bovis, M. bovis* BCG et *M. tuberculosis* H37Rv. Ceci a révélé un polymorphisme au locus *plcD* où les amplicons de *M. bovis* et de *M. bovis* BCG étaient d'environ 5 kb plus grands que le produit de H37Rv (Figure 4A). Cette délétion d'environ 5 kb dans le génome de *M*. *tuberculosis* H37Rv par rapport à *M*. *bovis* BCG a été appelée RvD2. Le séquençage du clone X086 du BAC de *M. bovis* BCG a révélé que RvD2 était positionné entre les bases 1987699-19890045 dans le génome de *M. tuberculosis.* La région comprend 6,5 kb et contient 3 ORFs codant pour une protéine inconnue, une oxydoréductase et une protéine membranaire, et elle prolonge le gène *plcD* pour coder pour un produit de 514 acides aminés (Figure 4B, Tableau 1).

### II. DONNES EXPERIMENTALES

### Souches bactériennes et plasmides

Les souches du complexe de *M*. *tuberculosis* (*Mycobacterium africanum, Mycobacterium microti, Mycobacterium tuberculosis, Mycobacterium bovis* et *Mycobacterium bovis* BCG) et sous-souches de *M. bovis* BCG (Danemark, Glaxo, Russe, Japonais, Pasteur et Moreau) ont été obtenus à partir des stocks de laboratoires (Unité de G.M.B., Institut Pasteur). *Mycobacterium tuberculosis* CSU#93 a été reçu de John BELISLE, Department of Microbiology, Colorado State University, Fort Collins, CO 80523. Des isolats cliniques non épidémiques de *M. tuberculosis* ont été fournis par Beate HEYM, Hopital Ambroise Paré, 9 avenue Charles de Gaulle, 92104 BOULOGNE CEDEX, FRANCE. Les vecteurs de BAC pBeloBAC11 (Kim et al., 1996) et PBACe3.6 (Genbank n° d'accession U80929) ont été donnés par H. SHIZUYA, Department of Biology, California Institute of Technology, Pasadena, CA, et P. de JONG, Roswell Park Cancer Institute, Human Genetics Department, Buffalo, NY, respectivement. Les vecteurs et les recombinants dérivés ont été maintenus dans *E*. *coli* DH10B.

### Préparation de l'ADN génomique

La préparation de l'ADN génomique dans des cubes d'agarose à partir de *M. bovis* BCG Pasteur a été réalisée comme indiqué précédemment (Philipp et al., 1996 ; Philipp et al., 1996) mais avec deux digestions à la protéinase K pendant 24 h chacune, plutôt qu'une digestion de 48 h. Les cubes ont été stockés dans 0,2 M EDTA à 4°C et lavés 2 fois dans 50 ml de Tris-EDTA (pH 8)/Triton X-100 (0,1 %) à 4°C pendant 1 h, ensuite lavés 2 fois dans 50 ml d'un tampon d'enzyme de restriction Triton X-100 (0,1 %) pendant 1 h à température ambiante avant utilisation.

### Construction de la banque BAC

Un vecteur d'ADN a été préparé comme précédemment indiqué (Woo et al., 1994). Des digestions partielles avec *Hin*dIII et *EcoRI* de l'ADN dans l'agarose, pour le clonage dans pBeloBAC11 et pBACe3.6 respectivement, et ensuite une migration en champ électrique homogène à contour serré (CHEF) ont été réalisées comme précédemment décrit (Brosch et al., 1998). 5 zones, 50-75 kb, 75-100 kb, 100-125 kb, 125-150 kb et 150-170 kb ont été excisées à partir des gels d'agarose et stockées dans du TE à 4°C. Des ligatures avec les vecteurs pBeloBAC11 et pBACe3.6 et une transformation dans *E. coli* DH10B ont été réalisées comme précédemment décrit (Brosch et al., 1998). Les transformants pBeloBAC11 ont été sélectionnés sur de l'agar LB contenant 12,5 µg/ml de chloramphénicol, 50 µg/ml de X-gal et 25 µg/ml d'IPTG, et ont été criblés avec des colonies recombinantes blanches. Les transformants pBACe3.6 ont été sélectionnés sur de l'agar LB contenant 12,5 µg/ml de chloramphénicol et 5 % de sucrose. Les clones recombinants ont été repiqués, en deux exemplaires, dans des plaques microtitre à 96 puits contenant un milieu 2xYT avec 12,5 µg/ml de chloramphénicol et ont été incubés toute la nuit à 37°C. Un volume égal de glycérol à 80% a alors été ajouté aux puits et une plaque a été conservée à -80°C en tant que plaque maîtresse. La plaque restante a été utilisée pour faire des ensembles de clones à des fins de criblage (voir ci-dessus).

### Préparation d'ADN à partir de recombinants et détermination de la taille des inserts

Un recombinant portant un plasmide d'ADN a été préparé à partir de 40 ml de culture et a été mis en croissance sur le milieu 2xYT contenant 12,5 µg/ml de chloramphénicol comme précédemment décrit (Brosch et al., 1998). 100-200 ng d'ADN ont été digérés avec *Dra*I (Gibco-BRL) et les produits de restriction ont été séparés sur un gel d'électrophorèse en champ pulsé (PFGE) avec un appareil CHEF de LKB-Pharmacia utilisant un gel d'agarose à 1 % (poids/volume) et une impulsion de 4 secondes pendant 15 h à 6,25 V/cm. Des marqueurs PFGE de taille moyenne et inférieure (New England Biolabs) ont été utilisés en tant que taille standard. Les tailles des inserts ont été estimées après coloration au bromure d'éthidium et visualisation avec de la lumière UV.

### Réactions de séquençage

Des réactions de séquençage ont été réalisées comme précédemment indiqué (Brosch et al., 1998). Pour des clones isolés à partir de la banque de pBeloBAC11, les amorces SP6 et T7 ont été utilisées pour séquencer les extrémités des inserts, tandis que pour les clones pBACe3.6, les amorces dérivées du vecteur ont été utilisées. Les réactions ont été chargées sur des gels de polyacrylamide à 6 % et une électrophorèse a été réalisée avec un séquenceur d'ADN automatique 373A ou 377 (Applied Biosystems) pendant 10 à 12 h. Les réactions ont donné généralement entre 300 et 600 pb de séquences lisibles.

### Puces BAC

Les clones qui se superposaient à partir de la banque pBeloBAC11 de *M. tuberculosis* H37Rv (Brosch et al., 1998) ont été sélectionnés de telle façon que 97 % du génome de *M. tuberculosis* étaient représentés. L'ADN préparé à partir de ces clones a été digéré avec *Eco*RI (Gibco-BRL) ou PvuII (Gibco-BRL) et a été mis à migrer dans des gels d'agarose à 0,8 %, de 25 cm de longueur, à un faible voltage pendant 12 à 16 h. Après coloration et visualisation aux UV, les gels d'agarose ont été traités avec la méthode Southern standard et les ADN ont été transférés sur des membranes de nitrocellulose Hybond-C Extra (Amersham). L'ADN a été fixé à la membrane par chauffage à 80°C pendant 2 h. L'ADN génomique de *M. tuberculosis* H37Rv, *Mycobacterium bovis* ATCC 19210 et *M*. *bovis* BCG Pasteur a été marqué avec [α- ³²P]dCTP au moyen du kit Prime-It II (Stratagene). Les sondes ont été purifiées à travers une colonne P10 (Biorad) avant utilisation. Des hybridations ont été réalisées comme précédemment décrit (Philipp et al., 1996). Les sondes marquées purifiées ont été mises en solution dans une solution de 5xSSC (1xSSC est 0,5 M de chlorure de sodium ; 0,015 M de citrate de sodium), et 50 % (poids/volume) formamide. L'hybridation a été réalisée à 37°C, et les membranes ont été lavées pendant 15 mn à température ambiante dans du 2xSSC/0,1 % SDS et ensuite dans du 1xSSC/0,1% SDS et finalement dans du 0,1 xSSC/0,1 % SDS. Les résultats ont été interprétés à partir des autoradiogrammes. En général, il a été difficile de visualiser sur les autoradiogrammes les fragments de moins de 1 kb, surtout après utilisation répétée des membranes. Les fragments supérieurs à 1 kb ont donné des résultats plus clairs. Les clones qui sont apparus comme contenant des fragments sans contrepartie dans *M. bovis* BCG ont été repiqués pour des analyses ultérieures. La séquence génomique a permis l'établissement de cartes de restriction dans le but de déterminer les zones de délétion suspectées, permettant de sélectionner des enzymes donnant la meilleure résolution des régions. Des clones pouvaient ainsi être digérés avec une seconde gamme d'enzymes (généralement *Pst*I et *Stu*I*,* avec *Eco*RI inclus en tant que contrôle) et hybridés pour obtenir une taille plus précise de la délétion. Les amorces de séquençage qui flanquent les délétions ont ainsi été désignées et utilisées dans les réactions de séquençage avec le BAC correspondant de *M. bovis* BCG utilisé comme matrice.

### Analyses PCR

Les amorces utilisées dans les réactions de PCR sont listées dans les Tableaux 3 et 4. Les réactions pour des produits attendus de moins de 3 kb ont été réalisées avec une polymérase *Taq* standard (Boehringer Mannheim). Les réactions mettaient en oeuvre 5 µl de tampon 10xPCR (100 mM de β-mercaptoéthanol, 600 mM de Tris HCl, pH 8,8), 20 mM de Mach, 170 mM de (NH₄)₂SO₄, 5 µl de mélange nucléotidique à 20 mM, 0,2 µM de chaque amorce, 10-50 ng de matrice d'ADN, du DMSO à 10 %, 0,5 unité de polymérase *Taq* et de l'eau distillée stérile à 50 µl. Les cycles thermiques ont été réalisés avec un amplificateur PTC-100 (MJ Inc.) avec une étape de dénaturation initiale de 90 secondes à 95°C suivie par 35 cycles de 30 secondes à 95°C, 1 min à 55°C et 2 min à 72°C.

Les réactions de PCR susceptibles de donner lieu à des produits supérieurs à 3 kb ont été réalisées au moyen du kit de PCR GeneAmp XL (Perkin Elmer). Les réactions ont été lancées selon les instructions des fabricants, avec 0,8 mM de Mg(OAc)₂; 0,2 µM de chaque amorce et 10-30 ng de matrice d'ADN par réaction. Les cycles thermiques étaient réalisés à 96°C pendant 1 mn, suivis ensuite par 15 cycles en 2 étapes à 94°C pendant 15 secondes et 70°C pendant 7 mn, suivis par 20 cycles en 2 étapes à 94°C pendant 15 secondes et 70°C pendant 8 mn plus 15 secondes par cycle.

### Analyse informatique

Les données concernant les séquences ont été transférées du séquenceur automatisé ABI373A aux stations de travail Digital ou Sun et éditées au moyen du logiciel TED à partir de l'ensemble Staden. Les séquences éditées ont été comparées à la base de données des inventeurs concernant *M. tuberculosis* (H37Rv.dbs) pour déterminer les positions relatives des séquences terminales sur la séquence du génome de *M. tuberculosis.* Avec cette méthode, une cartographie des clones BAC de *M. bovis* BCG a été construite en utilisant la séquence de *M. tuberculosis* H37Rv comme matrice.

Pour faire de la comparaison génomique, des digestions *in silico* au moyen d'enzymes de restriction ont été réalisées avec le logiciel NIP (Nucleotide Interprétation Program) à partir de l'ensemble Staden. Le programme Display and Analysis (DIANA) du Centre Sanger, Cambridge, UK, a été utilisé pour interpréter les données de séquence.

### Numéros d'accession des séquences d'ADN

Les séquences nucléotidiques qui flanquent chaque locus RD dans *M. bovis* BCG ont été déposées dans la base de données EMBL. Les numéros d'accession pour RD5, RD6, RD7, RD8, RD9 et RD10 sont respectivement AJ007300, AJ131209, AJ007301, AJ131210, Y181604 et AJ 132559. Les séquences de RvD1 et RvD2 dans *M. bovis* BCG ont été déposées sous les n° Y18605 et Y18606 respectivement.

### Mise en évidence de la région dupliquée DU1

DU1 a été la première région dupliquée observée quand les bandes de digestion par *Hin*dIII du clone X038 du BAC de BCG et du clone Rv13 du BAC de H37Rv ont été comparées. Les deux clones X038 et Rv13 avaient des séquences terminales identiques, s'étendant de la position *Hin*dIII*∼* 4 367 kb au site *Hin*dIII∼ 0 027 kb (via 4411529 b) sur la séquence du génome de *M. tuberculosis* H37Rv (MTBH37RV), englobant l'origine de réplication.

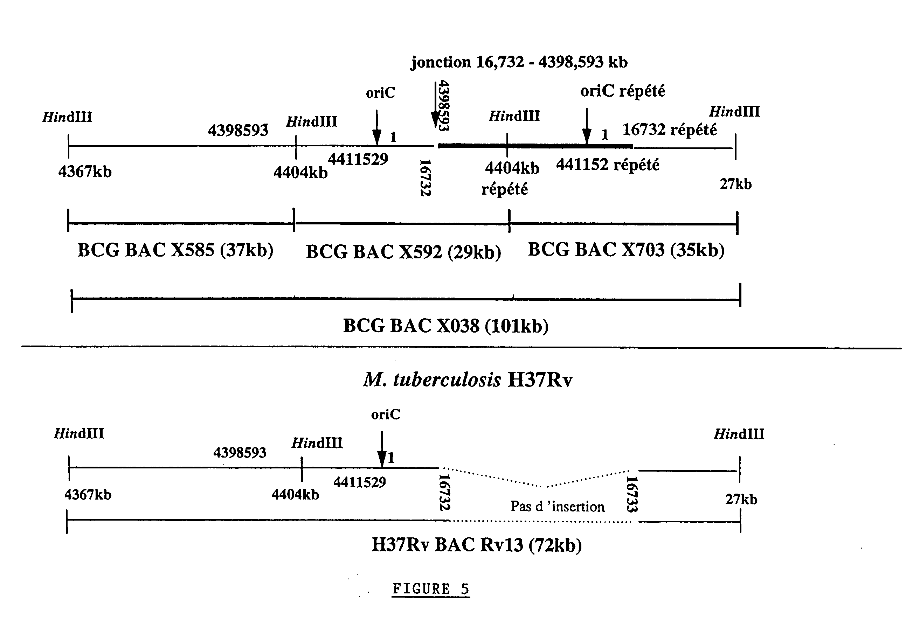

L'analyse *in silico* des sites de restriction *Hin*dIII pour la zone donnée entre - 4 367 kb et - 0 027 kb a révélé un site *Hin*dIII à la position -4 404 kb. En conséquence, la digestion de ces clones devrait montrer deux fragments de restriction plus la bande spécifique du vecteur à environ 8 kb. C'était le cas pour le clone Rv13 de H37Rv. Au contraire, le clone X038 du BAC de BCG montrait trois bandes plus la bande spécifique du vecteur à environ 8 kb, deux d'entre elles étaient identiques au schéma de Rv13. La bande additionnelle présente une taille d'environ 29 kb. Des analyses PFGE supplémentaires utilisant *Dra*I ont révélé que X038 est bien de 29 kb plus long que Rv13. Par un criblage PCR des pools de BAC de BCG utilisant des oligonucléotides sélectionnés, les inventeurs ont pu identifier trois clones X de plus couvrant les parties de cette région génomique dans BCG : X585, X592, X703. La séquence terminale et l'analyse PFGE ont montré que chacun de ces clones contient un insert de taille différente, correspondant aux trois bandes observées dans les résultats de digestion de X038 (Figure 5).

Les séquences terminales sont : X585 (∼ 4 367-4 404 kb) ; X592 (∼ 4 404-4404 kb) ; X703 (- 4 404-0 027 kb). Les séquences ont été répétées deux fois avec les mêmes résultats. Le curieux résultat selon lequel le clone X592 a des extrémités T7 et SP6 dans la même région génomique pouvait être expliqué par une duplication de cette région génomique dans BCG et a donné également l'indication sur la dimension du réarrangement. Des analyses de restriction comparatives additionnelles des clones X585, X592, X703 et X038 avec *Eco*RI ont révélé que X592 et X703 ont le même schéma de restriction à l'exception d'une bande de 10 kb présente dans X703 mais absente de X592. Sur la base de ces résultats, des amorces ont été préparées pour l'amplification de la région de jonction où le segment d'ADN dupliqué joint l'unique région.

L'analyse PCR avec des amorces à 16.000 et à 4398.700 pb (SEQ ID N° 19 et 21) a donné un produit d'une taille attendue à partir du clone X592 et également sur l'ADN génomique de BCG-Pasteur. Le séquençage des produits PCR obtenus directement sur l'ADN de BAC du clone X592 a révélé que la jonction était bien localisée aux bases 16,732/4398,593 comparativement à la séquence génomique de H37Rv et que ce réarrangement génomique résultait en la troncature des gènes *Rv3910* et pknB. Toutefois, dans la mesure où ce réarrangement est une duplication en tandem, des copies intactes des deux gènes pouvaient être présentes dans les régions avoisinantes. L'analyse PCR avec des amorces franquantes des gènes *Rv3920* et pknB a confirmé ceci quand l'ADN génomique du BCG-Pasteur et de *M. tuberculosis* H37Rv ont été utilisés. Une preuve additionnelle du réarrangement a été obtenue en utilisant un fragment PCR de 500 pb englobant la région *oriC* de H37Rv en tant que sonde marquée au ³²P pour hybrider les produits de digestion de l'ADN génomique de *M.* tuberculosis*, M. bovis* et *M. bovis* BCG-Pasteur dans les conditions stringentes décrites précédemment (Philipp et al., 1996). Tandis que dans *M. bovis* et *M. tuberculosis,* une bande d'une taille moyenne d'environ 35 kb a été détectée, dans *M*. *bovis* BCG-Pasteur deux bandes ont hybridé l'une d'environ 35 kb et l'autre de 29 kb. En conclusion, DU1 correspond à une duplication en tandem de 29668 pb qui résulte en une mérodiploïdie pour la région *sigM-pabA* (*Rv3911-Rv0013*).

L'analyse PCR utilisant des amorces à 16,000F (SEQ ID N° 19) ou 16,500F (SEQ ID N° 20) (amorces sens) et à 4398,770R (SEQ ID N° 21) (amorce reverse) sur l'ADN génomique de souches variées de BCG (Pasteur, Glaxo, Cophenhague, Russie, Prague, Japon) a révélé que des produits ont seulement été obtenus à partir de trois souches y compris *M*. *bovis* BCG-Pasteur. Les trois autres sous-souches ont toujours donné des résultats négatifs malgré la confirmation des contrôles positifs.

Comme attendu, la souche type de *M. bovis* et de *M. tuberculosis* H37Rv étaient également toujours négatives. Un résumé des données de cartographie est montré sur la Figure 5.

La région *dnaA-dnaN* est regardée de façon générale comme l'origine de réplication fonctionnelle des mycobactéries dans la mesure où après insertion dans des plasmides dont la propre origine de réplication est manquante, la capacité à se répliquer de façon autonome a été restaurée. Dans la mesure où BCG-Pasteur est diploïde pour la région *dnaA-dnaN,* les Inventeurs ont étudié s'il existait des différences entre les nucléotides des deux copies présentes sur les deux clones BAC X592 et X703. L'analyse de la séquence de l'ADN des BAC en utilisant des amorces de régions flanquantes et internes de la région intergénique *dnaA-dnaN* n'a révélé aucune différence entre les deux copies de la région minimale *oriC.* De plus, ces séquences étaient identiques à celles divulguées dans la littérature pour cette souche de BCG. Cette étude suggère que les deux copies de *oriC* devraient être fonctionnelles.

### Mise en évidence de la région dupliquée DU2

Le deuxième grand réarrangement génomique observé dans le chromosome de *M. bovis* BCG-Pasteur a été trouvé par l'analyse de plusieurs clones de BAC de BCG couvrant une région génomique d'environ 200 kb (3 550-3 750 kb). Leurs tailles évaluées par PFGE n'étaient pas conformes avec celles attendues à partir du génome de H37Rv et des données relatives aux séquences terminales. Les comparaisons directes ont été compliquées par la présence d'un élément IS*6110* dans cette région du chromosome de *M. tuberculosis* H37Rv qui a conduit à une petite délétion RvD5.

Les séquences terminales du BAC X495 étaient toutes deux localisées aux alentours du site *Hin*dIII à 3 594 kb, tandis que les résultats PFGE montraient que le clone a une taille d'environ 106 kb, contenant trois fragments *Hin*dIII*,* d'environ 37,5 kb, environ 37 kb et environ 24 kb en plus du vecteur. La bande de 24 kb était d'environ 2 kb plus longue que le fragment correspondant *Hin*dIII de 22 kb dans Rv403. Cette observation a conduit à l'hypothèse que la région génomique aux alentours de 3 594 kb devait avoir été dupliquée, ceci donnant lieu à l'introduction d'un nouveau site *Hin*dIII là où le clone X495 prend fin. Pour montrer cela, plusieurs amorces dans la région chromosomique de 3 589 kb à 3 594 kb ont été testées pour le séquençage de l'ADN du BAC X495 et une jonction (JDU2A) a été identifiée aux bases 3690124/3590900 relativement à la séquence génomique de H37Rv. Ceci menait à une interruption du gène *lpdA (Rv3303)* mais les résultats de PCR ont indiqué qu'une copie intacte de ce gène est présente dans la région dupliquée.

L'analyse systématique d'autres clones dans le voisinage a permis l'identification de 2 BACs indépendants du BCG (X094 et X1026) qui portaient le même fragment chromosomal 3 594 à 3 749 kb. Bien que les données de séquences terminales suggéraient que ces clones devaient avoir une taille d'environ 155 kb, la taille estimée par des digestions par *HindIII* ou *Dra*I suivies d'une séparation par PFGE était seulement d'environ 100 kb. Cette différence indiquait que les inserts de clones X094 et X1026 s'étendaient probablement des sites répétés *HindIII* à 3 594 kb au site authentique *Hind*III à la position 3 749 kb, et qu'une délétion interne avait eu lieu à l'intérieur de l'unité dupliquée.

Ceci a été confirmé par des expériences d'hybridation dans les conditions stringentes décrites précédemment sur de l'ADN génomique, digéré par *Hind*III*,* de *M. tuberculosis* H37Rv, *M. bovis* et BCG-Pasteur en utilisant de l'ADN du clone X495 radio-marqué. La taille de l'une des bandes qui s'hybridaient avec cet ADN dans les profils *Hind*III de *M. tuberculosis* H37Rv et *M. bovis* était d'environ 22 kb, tandis que la bande correspondante dans BCG était de 24 kb exactement ce qui était observé avec les clones BACs. De plus, les résultats d'hybridation montraient qu'une bande de 34 kb dans le profil *Hind*III du clone X094 s'hybridait également avec l'ADN génomique du clone X495, ce qui confirmait que les clones X094 et X1026 contenaient de l'ADN dupliqué de la région génomique couverte par X495. Des réactions de PCR et la séquence de l'ADN du clone BAC X094 ont permis l'identification d'un second point de jonction JDU2B à une position équivalente à 3 608 471/3 671 535 dans *M. tuberculosis* H37Rv. Ceci confirmait que DU2 résultait d'une duplication directe d'une région de 99 225 pb correspondant aux séquences entre les positions 3 590 900 et 3 690 124 dans le génome de *M*. *tuberculosis* H37Rv, et qu'une délétion interne de 63 064 pb avait ensuite eu lieu. L'unité résiduelle DU2 est ainsi longue de 36 162 pb, ce qui est cohérent avec les données de cartographie, et BCG-Pasteur est diploïde pour les gènes *Rv3213c - Rv3230c,* et *Rv3290c - Rv3302c.*

Finalement, des expériences de PCR, de cartographie par PFGE et de séquençage des séquences terminales avec le BAC X094 ont suggéré que le BCG-Pasteur contenait de l'ADN additionnel dans la région chromosomale du site *Hind*III 3 691 à 3 749 kb. La comparaison directe avec le clone BAC Rv403 de *M. tuberculosis* a permis la mise en évidence de deux sites supplémentaires *Hind*III dans cette région dans la mesure où les fragments *Hind*III de 48 kb présents dans Rv403 (correspondant au fragment de 3 691 à 3 749) étaient représentés par deux bandes de 22 et 36 kb dans BCG. Cette région du chromosome de *M. tuberculosis* H37Rv contient une copie de IS*6110* qui n'est pas flanquée des unités répétées caractéristiques directes de 3 pb. Il est maintenant clair qu'il y avait initialement deux copies de IS*6110* qui ont servi comme substrat pour un événement de recombinaison. Ceci a donné lieu à la délétion d'un segment de 4 kb du génome de *M. tuberculosis* H37Rv (RvD5), qui est toujours présent dans BCG. de même que dans *M. bovis* et les isolats cliniques de *M. tuberculosis.* L'analyse de la séquence de cette région a indiqué que ce fragment de 4 kb contient deux sites *HindIII* et qu'il y manque la séquence IS6110 qui est présente à ce site dans *M. tuberculosis* H37Rv. En utilisant des amorces internes pour RvD5 (Tableau 4), les Inventeurs ont obtenu des amplicons avec l'ADN génomique de toutes les souches de *M. bovis* BCG testées et, la souche de *M. bovis,* de même qu'avec l'ADN de clones X094 et X1026, mais pas avec les souches de *M. tuberculosis* H37Rv et H37Ra.

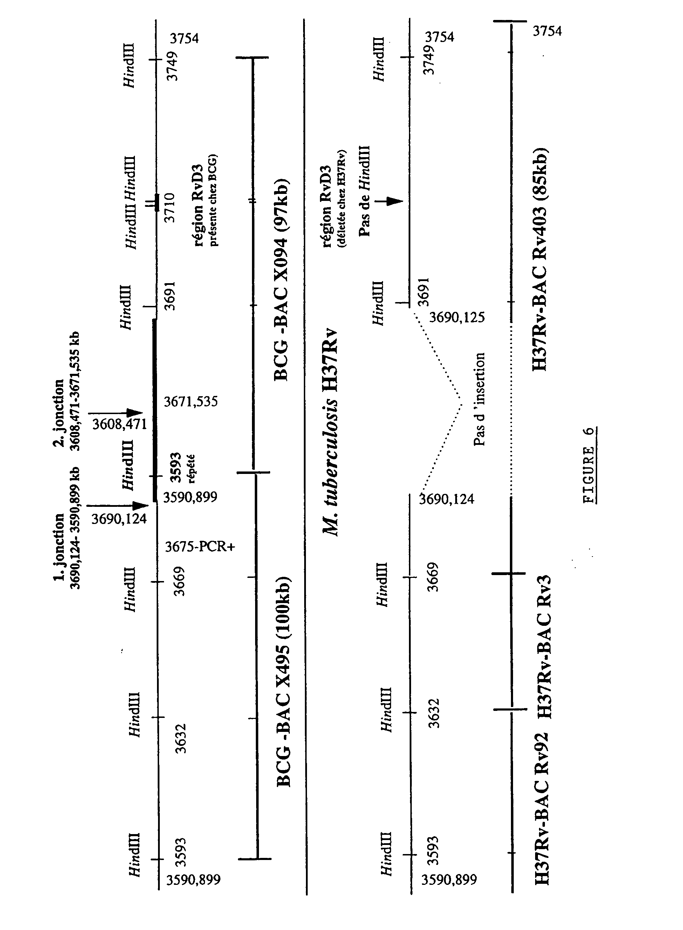

Des expérimentations avec de multiples jeux d'amorces (3689.500F (SEQ ID N° 22) ou 3689.900F (SEQ ID N° 24) (sens) 3591.000R (SEQ ID N° 23), 3591.500R (SEQ ID N° 25) ou 3592.000R (reverse)) pour amplifier la région de jonction au niveau de la base 3690124/3590900 (décrit ci-dessus) dans différentes souches de *M. bovis* BCG ont révélé que des amplicons pouvaient être obtenus seulement à partir de *M. bovis* BCG-Pasteur et à partir de deux autres sous-souches de BCG, tandis que les autres sous-souches de BCG ne donnaient pas d'amplicon. La confirmation des résultats pourra être faite sur des spots *Hin*dIII hybridés avec l'ADN marqué issu de la région 3689500F-3690.000R qui devrait donner lieu à des bandes avec des souches de BCG réarrangées, l'une d'entre elles présente une taille d'environ 24 kb, environ 2 kb de plus que la bande correspondante dans les digestions génomiques de *M. bovis* et de *M. tuberculosis.* La seconde bande d'environ 35 kb devrait seulement être présente dans les souches réarrangées et pas dans *M*. *tuberculosis* H37Rv ou la souche type de *M. bovis* (Figure 6).

Le criblage de clones de 2000 X et XE (Gordon et al., 1999) pour des BACs contenant à la fois les jonctions JDU2A et JDU2B, c'est-à-dire qui couvrent la région complète réarrangée a permis l'identification de trois BACs (X1070, XE377 et XE256) qui ont produit des amplicons avec les deux jeux d'amorces. Les inserts ont été estimés être respectivement d'une taille de 95. 86 et 97 kb. par PFGE. Sur la base de ces résultats de PCR, des données correspondant aux séquences terminales et de la présence de trois fragments chromosomaux *Hind*III de 37, 36 et 24 kb, les Inventeurs ont conclu que le clone X1070 chevauche le clone X495. Pourtant, il contenait un fragment chromosomal *HindIII* de 36 kb qui n'était présent ni dans le clone X495 ni dans le clone X094 et, avec les données de séquences terminales, ceci suggérerait la présence d'une troisième copie du site *HindIII* à 3 594 kb dans la région réarrangée. On a obtenu de nouvelles preuves de ceci quand les clones XE256 et XE377 isolés d'une banque *Eco*R*I* dans pBACe3.6, ont été analysés. En fonction des données des séquences terminales, XE256 s'étend du site *Eco*R*I* à 3 597 kb au site *EcoRI* à 3 713 kb, et XE377 du site *EcoRI* à 3 679 kb au site *Eco*RI *à* 3 715 kb. Le fait que ces clones donnaient de façon répétée des amplicons pour les deux régions de jonction citées JDU2A et JDU2B n'était pas en accord avec leur taille et leurs séquences terminales. Pourtant, ces données étaient cohérentes avec le fait que la région de 36 162 pb de DU2 était présente non seulement comme une mais plutôt en tant que deux copies en tandem. L'hybridation (selon la méthode de Philipp et al., 1996) des fragments d'ADN digéré par *HindIII* des clones XE256, X1070 et XE377 avec une sonde de 0,5 kb de la région génomique 3 675 kb a confirmé les résultats de PCR. Un fragment de 24 kb du clone X1070 s'hybridait, équivalent de celui du clone X495, et un fragment unique de 36 kb qui correspond à une copie additionnelle de DU2 était aussi présent. Deux fragments de 33 et 34 kb du clone XE256 s'hybridaient à la sonde. Le fragment de 33 kb correspond à une région qui s'étend du site *Hind*III présent dans le vecteur adjacent au site *Eco*RI de clonage au site *Hind*III le plus proche dans l'insert mycobactérien, tandis que le fragment de 34 kb est identique à celui également présent dans le clone X094. Le fragment de 33 kb chevauchait en partie le clone X1070 tandis que le fragment de 34 kb *Hind*III était identique à celui présent dans les clones X094 et XE377.

Ces données indiquaient que deux copies en tandem de DU2 existent dans le génome de BCG-Pasteur. Ceci a été confirmé par les hybridations des produits de digestion par *Hind*III de l'ADN génomique de BCG-Pasteur, *M. tuberculosis* H37Rv et *M. bovis* puisque tous s'hybridaient avec la sonde 3 675. Comme attendu, seulement une bande de 22 kb a été observée avec *M. tuberculosis* et *M. bovis* tandis que trois bandes de 24, 34 et 36 kb ont été détectées, par hybridation, dans le génome de BCG-Pasteur. Toutefois, le signal d'hybridation pour le fragment à 36 kb était très faible. Le fait que les bandes de 24 et 36 kb présentes dans le clone de BAC X1070 s'hybrident avec la sonde 3 675 avec la même intensité, alors que celles dans l'ADN génomique de BCG-Pasteur ne le font pas, suggère que seulement une sous-population de la culture de BCG-Pasteur contient la seconde copie de DU2. Ainsi, la différence observée dans l'intensité d'hybridation peut refléter que la seconde copie de DU2 n'a été acquise que récemment et indique que des variants qui contiennent une ou deux copie(s) de DU2 existent probablement dans la même culture de *M*. *bovis* BCG-Pasteur.

On a obtenu des résultats similaires avec des fragments d'ADN génomique digérés par *Xba*I de *M. tuberculosis, M. bovis* et BCG-Pasteur qui s'hybridaient avec la sonde 3 675. Dans la digestion de *M. tuberculosis* H37Rv, la sonde 3 675 s'hybridait avec un fragment de 183 kb (position génomique 3 646 kb à 3 829 kb). Le fragment correspondant de *M. bovis* était d'à peu près 178 kb, cette différence de taille étant due à l'absence de plusieurs éléments d'insertion qui ne sont présents que dans le fragment génomique de 183 kb de *M. tuberculosis* H37Rv. Le produit de digestion par *Xba*I de BCG-Pasteur contenait deux fragments de 215 et 250 kb qui s'hybridaient avec la sonde 3 675. Ces deux fragments correspondaient au fragment de 178 kb observé dans le génome de *M. bovis* augmenté par 36 ou 72 kb en raison de la présence d'une ou de deux copie(s) de DU2. Il est intéressant de noter que le signal d'hybridation pour le fragment de 250 kb était moins intense que le signal obtenu pour le fragment de 215 kb, ce qui confirme les observations précédentes avec les produits de digestion par *Hind*III*.*

Ces observations indiquent que cette région du génome de BCG est encore dynamique et qu'une sous-population de cellules est triploïde pour les gènes *Rv3213c-Rv3230c,* et *Rv3290c-Rv3302c.* Ces données de comparaison entre la séquence du génome de *M. tuberculosis* H37Rv et de BCG-Pasteur indiquent que BCG-Pasteur devrait être triploïde pour au moins 58 gènes, et qu'à un point de leur évolution, leur ancêtre commun contenait des copies dupliquées de 60 gènes supplémentaires qui ont été perdues lorsque la délétion interne à DU2 a eu lieu. De plus, la présence de DU1 et de DU2 et, en particulier la mise en évidence du fait que DU2 soit présente sous la forme de deux copies dans une sous-population de BCG-Pasteur, suggère que le processus de duplication en tandem dans BCG est encore dynamique.

L'invention fournit donc des données qui peuvent permettre de comparer les différentes souches de BCG entre elles. Par ailleurs, l'invention montre l'intérêt d'utiliser les stratégies de cartographie par les BACs en tant que complément du séquençage du génome et permet la mise en évidence des éventuels inconvénients des projets qui ne sont basés que sur le séquençage de clones par la technique « de coup de fusil ». Ainsi, sans cette librairie de BACs, il est très probable que ces réarrangements génomiques complexes présents dans les souches de *M. bovis* BCG n'auraient pas été détectés. C'est donc un avantage de la présente invention que de fournir des données qui permettent la caractérisation et éventuellement les classifications immunogéniques et protectrices, des différentes souches de BCG aujourd'hui utilisées en clinique et pour des applications vaccinales, ainsi que de fournir des éléments qui permettent l'identification spécifique de *M. tuberculosis* par rapport à *M. bovis* et *M. bovis* BCG, ou des éléments qui permettent l'identification spécifique de *M. bovis* BCG par rapport à *M. bovis.* La présente invention fournit ainsi des éléments importants pour l'étude et l'épidémiologie de la tuberculose, ainsi que pour les prochaines études de réarrangements génomiques dans les différentes bactéries. La technique développée dans la présente invention est exemplifiée par les résultats de la présente invention et peut être appliquée à d'autres génomes bactériens et/ou de parasites.

Ainsi, le fait que *M. bovis* BCG-Pasteur et deux autres sous-souches de *M. bovis* BCG ont un jeu complètement dupliqué de gènes responsables de procédés majeurs tels que, entre autres, la division cellulaire et la traduction du signal, comprenant deux origines de réplication, est l'un des aspects surprenant révélé aux inventeurs par cette approche des comparaisons génomiques.

Puisque le matériel biologique est sujet à changements, et étant donné que les essais de vaccination BCG ont donné des résultats très variables de protection (0-80 %), il pourrait être important d'évaluer si cette variation dans l'efficacité de protection peut être attribuée en partie au choix de la sous-souche BCG utilisée.

Il convient donc de mener des investigations complémentaires afin de déterminer s'il existe une corrélation entre les particularités génomiques et les variations phénotypiques parmi les différentes sous-souches de BCG.

Les banques de BAC ont été déposées à la Collection Nationale de Culture de Microorganismes (CNCM), 25 rue du Dr Roux, 75724 PARIS CEDEX 15, France selon les dispositions du Traité de Budapest.
- BAC de *M. tuberculosis* H37Rv: Numéro d'Ordre I1945
- BAC de *M. bovis BCG*: Numéro d'Ordre 12049

**TABLEAU 1: DESCRIPTION DES DELETIONS**

| **DELETIONS** | **ORF/ GENE** | **POSITION* DANS LE GENOME DE *M*. *TUBERCULOSIS* H37Rv** | **TAILLE DU PRODUIT** | **FONCTION PUTATIVE OU FAMILLE** |
|---|---|---|---|---|
| **RDS** | Rv2346c | 2625889-2626170 | 94 aa | Famille de ESAT-6 |
| | Rv2347c | 2626224-2626517 | 98 aa | Famille de QLISS |
| | Rv2348c | 2626655-2626978 | 108 aa | Inconnue |
| | *plcC* | 2627173-2628696 | 508 aa | Phospholipase |
| | *plcB* | 2628782-2630317 | 512 aa | Phospholipase |
| | *plcA* | 2630538-2632073 | 512 aa | Phospholipase |
| | Rv2352c | 2632924-2634096 | 391 aa | Protéine PPE |
| | Rv2353c | 2634529-2635590 | 354 aa | Protéine PPE |
| **RD6** | Rv3425 | 3842235-3842762 | 176 aa | Protéine PPE |
| | Rv3426 | 3843032-3843727 | 232 aa | Protéine PPE |
| | Rv3427c | 3843884-3844636 | 251 aa | Transposase IS*1532* |
| | Rv3428c | 3844737-3845966 | 410 aa | Transposase IS*1532* |
| **RD7** | Rv1964 | 2207698-2208492 | 265 aa | Membranaire intégrale |
| | Rv1965 | 2208505-2209317 | 271 aa | Membranaire intégrale |
| | *Mce3* | 2209325-2210599 | 425 aa | Protéine type invasine motif RGD |
| | Rv1967 | 2210599-2211624 | 342 aa | Protéine exportée |
| | Rv1968 | 2211624-2212853 | 410 aa | Protéine exportée, motif RGD |
| | Rv1969 | 2212853-2214122 | 423 aa | Protéine exportée |
| | *lprM* | 2212853-2214122 | 377 aa | Lipoprotéine |
| | Rv1971 | 2215255-2216565 | 437 aa | Protéine exportée |
| | Rv1972 | 2216590-2217162 | 191 aa | Protéine membranaire |
| | Rv1973 | 2217162-2217641 | 160 aa | Protéine exportée |
| | Rv1974 | 2217657-2218031 | 125 aa | Inconnue |
| | Rv1975 | 2218050-2218712 | 221 aa | Protéine exportée |
| | Rv1976c | 2218845-2219249 | 135 aa | Inconnue |
| | Rv1977 | 2219752-2220795 | 348 aa | Inconnue, signature de liaison Zn |
| **RD8** | *ephA* | 4057730-4058695 | 322 aa | Epoxyde hydrolase |
| | Rv3618 | 4058695-4059879 | 395 aa | Monooxygénase |
| | Rv3619c | 4059984-4060265 | 94 aa | Famille de ESAT-6 |
| | Rv3620c | 4060295-4060588 | 98 aa | Famille de QLISS |
| | Rv3621c | 4060648-4061886 | 413 aa | Protéine PPE |
| | Rv3622c | 4061899-4062195 | 99 aa | Protéine PE |
| | *lpqG* | 4062524-4063243 | 240 aa | Lipoprotéine |
| **RD9** | *cobL* | 2328975-2330144 | 390 aa | Précorrine méthylase |
| | Rv2073c | 2330215-2330961 | 249 aa | Oxidoréductase |
| | Rv2074 | 2330991-2331401 | 137 aa | Inconnue |
| | Rv2075 | 2331417-2332877 | 487 aa | Protéine ou membranaire exportée |
| **RD10** | *echA1* | 265505-266290 | 262 aa | Hydratase énoyl CoA |
| | Rv0223c | 266302-267762 | 487 aa | Aldéhyde déshydrogénase |
| **RvD1** | RvD1- ORF1 | - | 675 aa | Inconnue |
| | RvD1- ORF2 | - | 318 aa | Inconnue |
| | Rv2024c | - | 1606 aa | Inconnue |
| **RvD2** | *plcD* | - | 514 aa | Phospholipase |
| | RvD2- ORF1 | - | 394 aa | Sucre transférase |
| | RvD2- ORF2 | - | 367 aa | Oxidoréductase |
| | RvD2- ORF3 | - | 945 aa | Protéine membranaire |
| | Rv1758 | - | 143 aa | Cutinase |

| | | | | |
|---|---|---|---|---|
| * Telle que définie par Cole et al., Nature, 1998, 393, pages 537-544 | | | | |

**TABLEAU 2: DISTRIBUTION DES DELETIONS PARMI LE COMPLEXE DE M. TUBERCULOSIS**

| **DELETION** | ***M. tuberculosis* H37Rv** | ***M. africanum*** | ***M. bovis*** | ***M. bovis* BCG** | ***M. microti.* OV254** | ***M. tuberculosis* CSU#93** | ***M*. *tuberculosis* ISOLATS CLINIQUES*** |
|---|---|---|---|---|---|---|---|
| RD4 | √ | √ | X | X | √ | √ | 27/27 |
| RD5 | √ | √ | X | X | X | √ | ND |
| RD6 | √ | √ | X | X | X | X | 19/30 |
| RD7 | √ | √ | X | X | X | √ | 30/30 |
| RD8 | √ | √ | X | X | X | √ | ND |
| RD9 | √ | X | X | X | X | √ | 8/8 |
| RD10 | √ | √ | X | X | X | √ | 8/8 |
| RvD1 | X | √ | √ | √ | √ | √ | 5/7 |
| RvD2 | X | √ | √ | √ | √ | √ | 4/7 |

| | | | | | | | |
|---|---|---|---|---|---|---|---|
| ND: Non déterminé : √= la région est présente, X= la région est délétée * Nombre d'isolats cliniques positifs pour la présence de région | | | | | | | |

**TABLEAU 3:AMORCES PCR**

| **DELETION** | **NOM DE L'AMORCE** | **SEQUENCE** | **TAILLE DU PRODUIT ATTENDUE** |
|---|---|---|---|
| RD4* | 277-32F | ACATGTACGAGAGACGGCATGAG | H37Rv: 1031 bp |
| | 277-32R | ATCCAACACGCAGCAACCAG | BCG: Pas de produit |
| RD5* | lcC-B.5P | GATTCCTGGACTGGCGTTG | H37Rv: 1623 bp |
| | lcC-B.3P | CCACCCAAGAAACCGCAC | BCG: Pas de produit |
| RD6 | 78-dell | ACAAAATCGCCTCGTCGCC | H37Rv: 8729 bp |
| | 78-del2 | ACCTGTATTCGTCGTTGCTGACC | BCG: 3801 bp |
| RD7 | v420-flank1.F | GGTAATCGTGGCCGACAAG | H37Rv: 13068 bp |
| | V420-flank2.R | CTTGCGGCCCAATGAATC | BCG: 350 bp |
| RD8* | D8-ephA.F | GTGTGATTTGGTGAGACGATG | H37Rv: 678 bp |
| | D8-ephA.R | GTTCCTCCTGACTAATCCAGGC | BCG: Pas de produit |
| RD9 | TB2329.5F | TCTGCCCGTCGTGCGCGAA | H37Rv: 3048 bp |
| | TB2332.5R | CAGTGGCTCGGCACGCACA | BCG: 1018 bp |
| RD10 | D10-264F | CGCGAAAGAGGTCATCTAAAC | H37Rv: 3024 bp |
| | D10-267R | GATGCTCAAGCCGTGCACC | BCG: 1121 bp |
| RvD1 | Boli2268469.F | GCGCCACAAACGTACTATCTC | H37Rv: 595 bp |
| | Boli2269064.R | GTTTCACCGGCTGTCGTTC | BCG: 5595 bp |
| RvD2 | 28-IS6110B.5' | CCACACCGCAGGATTGGCAAG | H37Rv: 2007 bpt |
| | 28-RHS.2 | TCGAGTGCATGAACGCAACCGAG | BCG: 7456 bp |

| | | | |
|---|---|---|---|
| ***** = Amorces internes à la délétion t = Taille incluant une copie de *IS6110* non présent dans BCG | | | |

**TABLEAU 4: AMORCES POUR L'IDENTIFICATION DES REGIONS DUPLIQUEES**

| **REGION** | **NOM DE L'AMORCE** | **SEQUENCE** |
|---|---|---|
| JONCTION DU1 | TB 16.0F | GAG CCA ACG ATG ATG ATG ACC |
| | TB16.5F | GGT CAC GGT CGG TGT CGT C |
| | TB4398.7R | CAG AAC TGC AGG GGT GGT AC |
| JONCTION DUZA | TB3689.5F | CTA GTT GTT CAG CCG CGT CTT |
| | TB3591.0R | ACC GGG GTG TCG GCC AGT T |
| | TB3689.9F | TCG CGG CCA CCG TGC GTA A |
| | TB3591.5R | GGC GCC TAT GAC TGA TAC CC |
| JONCTION DU2B | TB3608.0F | GAA CAG GGT CGC GGA GTC T |
| | TB3672.0R | TCG AGG AGG TCG AGT CCT GT |
| | TB3671.7R | GGG TTC ATG AGG TGC TAG GG |
| AMORCES DE DETECTION RvD5 | RvD5-intF | GGG TTC ACG TTC ATT ACT GTT C |
| | RvD5-intR | CCT GCG CTT ATC TCT AGC GG |
| SONDE D'HYBRIDATION DU 1 | TB4411.0F | CCG GCC ACT CAC TGC CTT C |
| | TB0.3R | ACG GTA GTG TCG TCG GCT TC |
| SONDE D'HYBRIDATION DU2 (sonde 3 675) | TB3675.0F | CCA ACA CCG TCA ACT ACT CGA |
| | TB3675.5R | ATC GCA GAA CTC CGG CGA CA |
| SEQUENCAGE DE LA REGION dnaA-dnaN | TB1.2F | CGA TCT GAT CGC CGA CGC C |
| | TB 1.5F | TCC GTC AGC GCT CCA AGC G |
| | TB 1.8F | GTC CCC AAA CTG CAC ACC CT |
| | TB2.2R | AAT CCG GAA ATC GTC AGA CCG |

### REFERENCES

1. Arruda, S., Bomfim, G., Knights, R., Huima, B.T. and Riley, L.W. (1993) Cloning of an M. tuberculosis DNA fragment associated with entry and survival inside cells. Science 261: 1454-1457.
2. Bloom, B.R. and Fine, P.E.M. (1994) The BCG experience: Implications for future vaccines against tuberculosis. In Tuberculosis: Pathogenesis, Protection and Control. Bloom, B.R. (eds). Washington D.C.: American Society for Microbiology, pp. 531-557.
3. Brosch, R., Gordon, S.V., Billault, A., Garnier, T., Eiglmeier, K., Soravito, C., Barrell, B.G. and Cole, S.T. (1998) Use of a Mycobacterium tuberculosis H37Rv bacterial artificial chromosome library for genome mapping, sequencing, and comparative genomics. Infect Immun 66: 2221-2229.
4. Calmette, A. (1927) La vaccination contre la tuberculose, 250 p, Paris: Masson et Cie.
5. Chee, M., Yang, R., Hubbell, E., Berno, A., Huang, X.C., Stern, D., Winkler, J., Lockhart, D.J., Morris, M.S. and Fodor, S.P. (1996) Accessing genetic information with high-density DNA arrays. Science 274: 610-614.
6. Cole, S.T., Brosch, R., Parkhill, J., Garnier, T., Churcher, C., Harris, D. et al. (1998) Deciphering the biology of Mycobacterium tuberculosis from the complete genome sequence. Nature 393: 537-544.
7. DeRisi, J.L., Iyer, V.R. and Brown, P.O. (1997) Exploring the metabolic and genetic control of gene expression on a genomic scale. Science 278: 610-614.
8. Elhay, M.J., Oettinger, T. and Andersen, P. (1998) Delayed-type hypersensitivity responses to ESAT-6 and MPT64 from Mycobacterium tuberculosis in the guinea pig. Infect Immun 66: 3454-3456.
9. Fine, P.E.M. (1994) Immunities in and to tuberculosis: implications for pathogenesis and vaccination. In Tuberculosis: Back to the future. Porter, J.D.H. and McAdam, K.P.W.J. (eds). Chichester: John Wiley and Sons Ltd., pp. 53-74.
10. Gordon, S.V., Heym, B., Parkhill, J., Barrell, B.G. and Cole, S.T. (1998) New insertion sequences and a novel repetitive element in the genome of Mycobacterium tuberculosis. Microbiology (in press)
11. Harboe, M., Oettinger, T., Wiker, H.G., Rosenkrands, I. and Andersen, P. (1996) Evidence for occurrence of the ESAT-6 protein in Mycobacterium tuberculosis and virulent Mycobacterium bovis and for its absence in Mycobacterium bovis BCG. Infect Immun 64: 16-22.
12. Heifets, L.B. and Good, R.C. (1994) Current laboratory methods for the diagnosis of tuberculosis. In Tuberculosis: Pathogenesis, Protection and Control. Bllom, B.R. (eds). Washington D.C.: American Society for Microbiology, pp. 85-110.
13. Horwitz, M.A., Lee, B.W., Dillon, B.J. and Harth, G. (1995) Protective immunity against tuberculosis induced by vaccination with major extracellular proteins of Mycobacterium tuberculosis. Proc Natl Acad Sci USA 92: 1530-1534.
14. Johansen, K.A., Gill, R.E. and Vasil, M.L. (1996) Biochemical and molecular analysis of phospholipase C and phospholipase D activity in mycobacteria. Infect Immun 64: 3259-3266.
15. Kim, U.J., Birren, B.W., Slepak, T., Mancino, V., Boysen, C., Kang, H.L., Simon, M.I. and Shizuya, H. (1996) Construction and characterization of a human bacterial artificial chromosome library. Genomics 34: 213-218.
16. Lagranderie, M.R., Balazuc, A.M., Deriaud, E. and Leclerc, C.D. (1996) Comparison of immune responses of mice immunized with five different Mycobacterium bovis vaccine strains. Infect Immun 64: 1-9.
17. Lawes, M. and Maloy, S. (1995) MudSacI, a transposon with strong selectable and counterselectable markers: use for rapid mapping of chromosomal mutations in Salmonella typhimurium. J Bacteriol 177: 1383-1387.
18. Leao, S.C., Rocha, C.L., Murillo, L.A., Parra, C.A. and Patarroyo, M.E. (1995) A species-specific nucleotide sequence of Mycobacterium tuberculosis encodes a protein that exhibits hemolytic activity when expressed in Escherichia coli. Infect Immun 63: 4301-4306.
19. Mahairas, G.G., Sabo, P.J.; Hickey, M.J., Singh, D.C. and Stover, C.K. (1996) Molecular analysis of genetic differences between Mycobacterium bovis BCG and virulent M. bovis. J Bacteriol 178: 1274-1282.
20. Moghaddam, M.F., Grant, D.F., Cheek, J.M., Greene, J.F., Williamson, K.C. and Hammock, B.D. (1997) Bioactivation of leukotoxins to their toxic diols by epoxide hydrolase. Nature Med 3: 562-6.
21. Ohno, S. (1995) Active sites of ligands and their receptors are made of common peptides that are also found elsewhere. J Mol Evol 40: 102-6.
22. Pelicic, V., Reyrat, J.M. and Gicquel, B. (1996) Expression of the Bacillus subtilis sacB gene confers sucrose sensitivity on mycobacteria. J Bacteriol 178: 1197-9.
23. Philipp, W.J., Nair, S., Guglielmi, G., Lagranderie, M., Gicquel, B. and Cole, S.T. (1996) Physical mapping of Mycobacterium bovis BCG Pasteur reveals differences from the genome map of Mycobacterium tuberculosis H37Rv and from M. bovis. Microbiology 142:3135-3145
24. Philipp, W.J., Poulet, S., Eiglmeier, K., Pascopella, L., Balasubramanian, V., Heym, B., Bergh, S., Bloom, B.R., Jacobs, W.J. and Cole, S.T. (1996) An integrated map of the genome of the tubercle bacillus, Mycobacterium tuberculosis H37Rv, and comparison with Mycobacterium leprae. Proc Natl Acad Sci USA 93: 3132-3137.
25. Relman, D.A., Domenighini, M., Tuomanen, E., Rappuoli, R. and Falkow, S. (1989) Filamentous hemagglutinin of Bordetella pertussis: nucleotide sequence and crucial role in adherence. Proc Natl Acad Sci USA 86: 2637-2641.
26. Rosenkrands, I., Rasmussen, P.B., Carnio, M., Jacobsen, S., Theisen, M. and Andersen, P. (1998) Identification and characterization of a 29-kilodalton protein from Mycobacterium tuberculosis culture filtrate recognized by mouse memory effector cells. Infect Immun 66: 2728-2735.
27. Sreevatsan, S., Pan, X., Stockbauer, K.E., Connell, N.D., Kreiswirth, B.N., Whittam, T.S. and Musser, J.M. (1997) Restricted structural genes polymorphism in the Mycobacterium tuberculosis complex indicates evolutionarily recent global dissemination. Proc Natl Acad Sci USA 94: 9869-9874.
28. Titball, R.W. (1993) Bacterial phospholipases C. Microbiological Reviews 57: 347-66.
29. Wheeler, P.R. and Ratledge, C. (1992) Control and location of acyl-hydrolysing phospholipase activity in pathogenic mycobacteria. J Gen Microbiol 138: 825-830*.*
30. Woo, S.S., Jiang, J., Gill, B.S., Paterson, A.H. and Wing, R.A. (1994) Construction and characterization of a bacterial artificial chromosome library of Sorghum bicolor. Nuc Acids Res 22: 4922-31.

## Revendications

1. Procédé de détection et d'identification permettant de discriminer *M. bovis* BCG et *M. bovis* de *M. tuberculosis* dans un échantillon biologique, comprenant les étapes suivantes :
a) isolement de l'ADN à partir de l'échantillon biologique à analyser ou obtention d'un ADNc à partir de l'ARN de l'échantillon biologique,
b) détection des séquences d'ADN de la mycobactérie présente dans ledit échantillon biologique,
c) comparaison desdites séquences avec les séquences nucléotidiques absentes des génomes de *M. bovis* BCG et de *M. bovis* et présentes dans le génome de *M. tuberculosis* choisies parmi les ORFs et gènes suivants : Rv2346c, Rv2347c, Rv2348c, *plcC, plcB, plcA,* Rv2352c, Rv2353c, Rv3425, Rv3426, Rv3427c, Rv3428c, Rv1964, Rv1965, *mce3,* Rv1967, Rv1968, Rv1969, *lprM,* Rv1971, Rv1972, Rv1973, Rv1974, Rv1975, Rv1976c, Rv1977, *ephA,* Rv3618, Rv3619c, Rv3620c, Rv3621c, Rv3622c, *lpqG, cobL,* Rv2073c, Rv2074, Rv2075c, *echAl,* Rv0223c, tels que définis dans le tableau 1.
d) comparaison desdites séquences avec les séquences nucléotidiques présentes dans les génomes de *M. bovis* BCG et de *M. bovis* et absentes du génome de *M. tuberculosis* choisies parmi les ORFs et gènes suivants : RvD1-ORF1, RvD1-ORF2, RvD1-Rv2024c, RvD2-*plcD,* RvD2-ORF1, RvD2-ORF2, RvD2-ORF3, RvD2-Rv1758, la séquence nucléotidique de RvD1 ayant été déposée sous le n° Y18605 et la séquence nucléotidique de RvD2 ayant été déposée sous le n° Y18606 dans la base de données EMBL.

2. Procédé de détection et d'identification selon la revendication 1, **caractérisé en ce que** les séquences nucléotidiques telles que définies aux étapes c) et d) sont regroupées en régions nucléotidiques RD5 à RD10 et RvD1 et RvD2 selon la répartition suivante :
- RD5: Rv2346c, Rv2347c, Rv2348c, *plcC, plcB, plcA,* Rv2352c, Rv2353c,
- RD6 : Rv3425, Rv3426, Rv3427c, Rv3428c,
- RD7: Rv1964, Rv1965, *mce3,* Rv1967, Rv1968, Rv1969, *lprM,* Rv1971, Rv1972, Rv1973, Rv1974, Rv1975, Rv1976c, Rv1977,
- RD8 : *ephA,* Rv3618, Rv3619c, Rv3620c, Rv3621c, Rv3622c, *lpqG,*
- RD9 : *cobL,* Rv2073c, Rv2074, Rv2075c,
- RD10 : *echAl,* Rv0223c,
- RvD1: RvD1-ORF1, RvD1-ORF2, RvD1-Rv2024c,
- RvD2: RvD2-plcD, RvD2-ORF1, RvD2-ORF2, RvD2-ORF3, RvD2-Rv1758.

3. Procédé selon la revendication 1 ou 2, dans lequel la détection des séquences d'ADN de la mycobactérie est réalisée au moyen de séquences nucléotidiques complémentaires desdites séquences d'ADN.

4. Procédé selon la revendication 3, dans lequel la détection des séquences d'ADN de la mycobactérie est réalisée par amplification de ces séquences à l'aide d'amorces.

5. Procédé selon la revendication 4, dans lequel les amorces ont une séquence nucléotidique choisie dans le groupe comprenant SEQ ID N° 3, SEQ ID N° 4, SEQ ID N° 5, SEQ ID N° 6, SEQ ID N° 7, SEQ ID N° 8, SEQ ID N° 9, SEQ ID N° 10, SEQ ID N° 11 SEQ ID N° 12, SEQ ID N° 13, SEQ ID N° 14, SEQ ID N° 15, SEQ ID N° 16, SEQ ID N° 17 et SEQ ID N° 18 avec :
- le couple SEQ ID N° 3 / SEQ ID N° 4 spécifique de RD5,
- le couple SEQ ID N° 5 / SEQ ID N° 6 spécifique de RD6,
- le couple SEQ ID N° 7 / SEQ ID N° 8 spécifique de RD7,
- le couple SEQ ID N° 9 / SEQ ID N° 10 spécifique de RD8,
- le couple SEQ ID N° 11 / SEQ ID N° 12 spécifique de RD9, .
- le couple SEQ ID N° 13 / SEQ ID N° 14 spécifique de RD10,
- le couple SEQ ID N° 15 / SEQ ID N° 16 spécifique de RvD1, et
- le couple SEQ ID N° 17 / SEQ ID N° 18 spécifique de RvD2.

6. Kit pour la détection et l'identification permettant de discriminer *M. bovis* BCG et *M. bovis* de *M. tuberculosis* dans un échantillon biologique comprenant les éléments suivants :
a) au moins un couple d'amorces tel que défini dans la revendication 5,
b) les réactifs nécessaires pour effectuer une réaction d'amplification d'ADN,
c) éventuellement, les éléments nécessaires permettant de vérifier ou comparer la séquence et/ou la taille du fragment amplifié, par exemple des réactifs nécessaires à une électrophorèse ou une chromatographie, ou des sondes nécessaires à une hybridation moléculaire.

7. Utilisation d'au moins un couple d'amorces tel que défini dans la revendication 5 pour l'amplification de séquence d'ADN de *M. bovis* BCG, *M. bovis* ou *M. tuberculosis.*

8. Procédé de détection *in vitro* d'anticorps permettant de discriminer des anticorps dirigés contre *M. bovis* BCG et *M. bovis* d'anticorps dirigés contre *M. tuberculosis* dans un échantillon biologique, comprenant les étapes suivantes :
a) mise en contact de l'échantillon biologique avec au moins un produit d'expression des séquences nucléotidiques absentes des génomes de *M. bovis* BCG et de *M. bovis* et présentes dans le génome de *M. tuberculosis* choisies parmi les ORFs et gènes suivants : Rv2346c, Rv2347c, Rv2348c, *plcC, plcB, plcA,* Rv2352c, Rv2353c, Rv3425, Rv3426, Rv3427c, Rv3428c, Rv1964, Rv1965, *mce3,* Rv1967, Rv1968, Rv1969, *lprM,* Rv1971, Rv1972, Rv1973, Rv1974, Rv1975, Rv1976c, Rv1977, *ephA,* Rv3618, Rv3619c, Rv3620c, Rv3621c, Rv3622c, *lpqG, cobL,* Rv2073c, Rv2074, Rv2075c, *echAl,* Rv0223c, tels que définis dans le tableau 1 ou présentes dans les génomes de *M. bovis* BCG et de *M. bovis* et absentes du génome de *M. tuberculosis* choisies parmi les ORFs et gènes suivants : RvD1-ORF1, RvD1-ORF2, RvD1-Rv2024c, RvD2-*plcD,* RvD2-ORF1, RvD2-ORF2, RvD2-ORF3, RvD2-Rv1758, la séquence nucléotidique de RvD1 ayant été déposée sous le n° Y18605 et la séquence nucléotidique de RvD2 ayant été déposée sous le n° Y18606 dans la base de données EMBL,
b) mise en évidence du complexe antigène - anticorps formé.

9. Procédé de détection discriminante selon la revendication 8, **caractérisé en ce que** les séquences nucléotidiques telles que définies à l'étape a) dudit procédé sont regroupées en régions nucléotidiques RD5 à RD10 et RvD1 et RvD2 selon la répartition suivante :
- RD5: Rv2346c, Rv2347c, Rv2348c, *plcC, plcB, plcA,* Rv2352c, Rv2353c, tels que définis dans le tableau 1,
- RD6 : Rv3425, Rv3426, Rv3427c, Rv3428c, tels que définis dans le tableau 1,
- RD7: Rv1964, Rv1965, *mce3,* Rv1967, Rv1968, Rv1969, *lprM,* Rv1971, Rv1972, Rv1973, Rv1974, Rv1975, Rv1976c, Rv1977, tels que définis dans le tableau 1,
- RD8 : *ephA,* Rv3618, Rv3619c, Rv3620c, Rv3621c, Rv3622c, *lpqG,* tels que définis dans le tableau 1,
- RD9 : *cobL,* Rv2073c, Rv2074, Rv2075c, tels que définis dans le tableau 1,
- RD10: *echAl,* Rv0223c, tels que définis dans le tableau 1,
- RvD1 : RvD1-ORF1, RvD1-ORF2, RvD1-Rv2024c, la séquence nucléotidique de RvD1 ayant été déposée sous le n° Y18605 dans la base de données EMBL
- RvD2: RvD2-plcD, RvD2-ORF1, RvD2-ORF2, RvD2-ORF3, RvD2-Rv1758, la séquence nucléotidique de RvD2 ayant été déposée sous le n° Y18606 dans la base de données EMBL.

10. Procédé de détection *in vitro* permettant de discriminer une vaccination avec *M. bovis* BCG d'une infection par *M. tuberculosis* chez un mammifère, comprenant les étapes suivantes :
a) incubation de cellules dudit mammifère, plus particulièrement des cellules du système immunitaire dudit mammifère et plus particulièrement encore des cellules T avec au moins un produit d'expression des séquences nucléotidiques absentes des génomes de *M. bovis* BCG et de *M. bovis* et présentes dans *M. tuberculosis* choisies parmi les ORFs et gènes suivants : Rv2346c, Rv2347c, Rv2348c, *plcC, plcB, plcA,* Rv2352c, Rv2353c, Rv3425, Rv3426, Rv3427c, Rv3428c, Rv1964, Rv1965, *mce3,* Rv1967, Rv1968, Rv1969, *lprM,* Rv1971, Rv1972, Rv1973, Rv1974, Rv1975, Rv1976c, Rv1977, *ephA,* Rv3618, Rv3619c, Rv3620c, Rv3621c, Rv3622c, *lpqG, cobL,* Rv2073c, Rv2074, Rv2075c, *echAl,* Rv0223c, tels que définis dans le tableau 1 ou présentes dans les génomes de *M. bovis* BCG et de *M. bovis* et absentes du génome de *M. tuberculosis* choisies parmi les ORFs et gènes suivants: RvD1-ORF1, RvD1-ORF2, RvD1-Rv2024c, RvD2-plcD, RvD2-ORF1, RvD2-ORF2, RvD2-ORF3, RvD2-Rv1758, la séquence nucléotidique de RvD1 ayant été déposée sous le n° Y18605 et la séquence nucléotidique de RvD2 ayant été déposée sous le n° Y18606 dans la base de données EMBL,
b) détection d'une réaction cellulaire indiquant une sensibilisation préalable du mammifère audit produit, notamment la prolifération cellulaire et/ou la synthèse de protéines telles que l'interféron gamma.

11. Procédé de détection discriminante selon la revendication 10, **caractérisé en ce que** les séquences nucléotidiques telles que définies à l'étape a) dudit procédé sont regroupées en régions nucléotidiques RD5 à RD10 et RvD1 et RvD2 selon la répartition suivante :
- RD5: Rv2346c, Rv2347c, Rv2348c, *plcC, plcB, plcA,* Rv2352c, Rv2353c, tels que définis dans le tableau 1,
- RD6 : Rv3425, Rv3426, Rv3427c, Rv3428c, tels que définis dans le tableau 1,
- RD7: Rv1964, Rv1965, *mce3,* Rv1967, Rv1968, Rv1969, *lprM,* Rv1971, Rv1972, Rv1973, Rv1974, Rv1975, Rv1976c, Rv1977, tels que définis dans le tableau 1,
- RD8 : *ephA,* Rv3618, Rv3619c, Rv3620c, Rv3621c, Rv3622c, *lpgG,* tels que définis dans le tableau 1,
- RD9 : *cobL,* Rv2073c, Rv2074, Rv2075c, tels que définis dans le tableau 1,
- RD10 : *echAl,* Rv0223c, tels que définis dans le tableau 1,
- RvD1: RvD1-ORF1, RvD1-ORF2, RvD1-Rv2024c, la séquence nucléotidique de RvD1 ayant été déposée sous le n° Y18605 dans la base de données EMBL,
- RvD2: RvD2-plcD, RvD2-ORF1, RvD2-ORF2, RvD2-ORF3, RvD2-Rv1758, la séquence nucléotidique de RvD2 ayant été déposée sous le n° Y18606 dans la base de données EMBL.

12. Kit pour le diagnostic *in vitro* d'une infection par *M. tuberculosis* chez un mammifère éventuellement préalablement vacciné avec *M. bovis* BCG, comprenant :
a) un produit d'expression des séquences nucléotidiques absentes des génomes de *M. bovis* BCG et de *M. bovis* et présentes dans *M. tuberculosis* choisies parmi les ORFs et gènes suivants : Rv2346c, Rv2347c, Rv2348c, *plcC, plcB, plcA,* Rv2352c, Rv2353c, Rv3425, Rv3426, Rv3427c, Rv3428c, Rv1964, Rv1965, *mce3,* Rv1967, Rv1968, Rv1969, *lprM,* Rv1971, Rv1972, Rv1973, Rv1974, Rv1975, Rv1976c, Rv1977, *ephA,* Rv3618, Rv3619c, Rv3620c, Rv3621c, Rv3622c, *lpqG, cobL,* Rv2073c, Rv2074, Rv2075c, *echAl,* Rv0223c, tels que définis dans le tableau 1 ou présentes dans les génomes de *M. bovis* BCG et de *M. bovis* et absentes du génome de *M. tuberculosis* choisies parmi les ORFs et gènes suivants : RvD1-ORF1, RvD1-ORF2, RvD1-Rv2024c, RvD2-plcD, RvD2-ORF1, RvD2-ORF2, RvD2-ORF3, RvD2-Rv1758, la séquence nucléotidique de RvD1 ayant été déposée sous le n° Y18605 et la séquence nucléotidique de RvD2 ayant été déposée sous le n° Y18606 dans la base de données EMBL.
b) le cas échéant, les réactifs pour la constitution du milieu propice à la réaction immunologique,
c) les réactifs permettant la détection des complexes antigène - anticorps produits par la réaction immunologique,
d) le cas échéant, un échantillon biologique de référence (témoin négatif) dépourvu d'anticorps reconnus par ledit produit,
e) le cas échéant, un échantillon biologique de référence (témoin positif) contenant une quantité prédéterminée d'anticorps reconnus par ledit produit.

13. Kit selon la revendication 12, **caractérisé en ce que** les séquences nucléotidiques telles que définies dans la partie a) dudit kit sont regroupées en régions nucléotidiques RD5 à RD10 et RvD1 et RvD2 selon la répartition suivante :
- RD5 : Rv2346c, Rv2347c, Rv2348c, *plcC, plcB, plcA,* Rv2352c, Rv2353c, tels que définis dans le tableau 1,
- RD6 : Rv3425, Rv3426, Rv3427c, Rv3428c, tels que définis dans le tableau 1,
- RD7 : Rav1964, Rv1965, *mce3,* Rv1967, Rv1968, Rv1969, *lprM,* Rv1971, Rv1972, Rv1973, Rv1974, Rv1975, Rv1976c, Rv1977, tels que définis dans le tableau 1,
- RD8 : *ephA,* Rv3618, Rv3619c, Rv3620c, Rv3621c, Rv3622c, *lpqG,* tels que définis dans le tableau 1,
- RD9 : *cobL,* Rv2073c, Rv2074, Rv2075c, tels que définis dans le tableau 1,
- RD10 : *echAl,* Rv0223c, tels que définis dans le tableau 1,
- RvD1 : RvD1-ORF1, RvD1-ORF2, RvD1-Rv2024c, la séquence nucléotidique de RvD1: ayant été déposée sous le n° Y18605 dans la base de données EMBL
- RvD2: RvD2-plcD, RvD2-ORF1, RvD2-ORF2, RvD2-ORF3, RvD2-Rv1758, la séquence nucléotidique de RvD2 ayant été déposée sous le n° Y18606 dans la base de données EMBL.

14. Anticorps mono- ou polyclonaux, leurs fragments ou anticorps chimériques, **caractérisés en ce qu'**ils sont capables de reconnaître spécifiquement un produit d'expression des séquences nucléotidiques absentes des génomes de *M. bovis* BCG et de *M. bovis* et présentes dans *M. tuberculosis* choisies parmi les ORFs et gènes suivants : Rv2346c, Rv2347c, Rv2348c, *plcC, plcB, plcA,* Rv2352c, Rv2353c, Rv3425, Rv3426, Rv3427c, Rv3428c, Rv1964, Rv1965, *mce3,* Rv1967, Rv1968, Rv1969, *lprM,* Rv1971, Rv1972, Rv1973, Rv1974, Rv1975, Rv1976c, Rv1977, *ephA,* Rv3618, Rv3619c, Rv3620c, Rv3621c, Rv3622c, *lpqG, cobL,* Rv2073c, Rv2074, Rv2075c, *echAl,* Rv0223c, tels que définis dans le tableau 1 ou présentes dans les génomes de *M. bovis* BCG et de *M. bovis* et absentes du génome de *M. tuberculosis* choisies parmi les ORFs et gènes suivants: RvD1-ORF1, RvD1-ORF2, RvD1-Rv2024c, RvD2-*plcD,* RvD2-ORF1, RvD2-ORF2, RvD2-ORF3, RvD2-Rv1758, la séquence nucléotidique de RvD1 ayant été déposée sous le n° Y18605 et la séquence nucléotidique de RvD2 ayant été déposée sous le n° Y18606 dans la base de données EMBL.

15. Anticorps mono- ou polyclonaux, leurs fragments ou anticorps chimériques selon la revendication 14, **caractérisés en ce que** lesdites séquences nucléotidiques sont regroupées en régions nucléotidiques RD5 à RD10 et RvD1 et RvD2 selon la répartition suivante :
- RD5: Rv2346c, Rv2347c, Rv2348c, *plcC, plcB, plcA,* Rv2352c, Rv2353c, tels que définis dans le tableau 1,
- RD6 : Rv3425, Rv3426, Rv3427c, Rv3428c, tels que définis dans le tableau 1,
- RD7: Rv1964, Rv1965, *mce3,* Rv1967, Rv1968, Rv1969, /*prM,* Rv1971, Rv1972, Rv1973, Rv1974, Rv1975, Rv1976c, Rv1977, tels que définis dans le tableau 1,
- RD8 : *ephA,* Rv3618, Rv3619c, Rv3620c, Rv3621c, Rv3622c, *lpgG,* tels que définis dans le tableau 1,
- RD9: *cobL,* Rv2073c, Rv2074, Rv2075c, tels que définis dans le tableau 1,
- RD10 : *echAl,* Rv0223c, tels que définis dans le tableau 1,
- RvD1 : RvD1-ORF1, RvD1-ORF2, RvD1-Rv2024c, la séquence nucléotidique de RvD1 ayant été déposée sous le n° Y18605 dans la base de données EMBL,
- RvD2: RvD2-plcD, RvD2-ORF1, RvD2-ORF2, RvD2-ORF3, RvD2-Rv1758, la séquence nucléotidique de RvD2 ayant été déposée sous le n° Y18606 dans la base de données EMBL.

16. Procédé pour la détection de la présence d'un antigène permettant de discriminer un antigène de *M. bovis BCG* et *M. bovis* d'un antigène de *M. tuberculosis* dans un échantillon biologique comprenant les étapes suivantes :
a) mise en contact de l'échantillon biologique avec un anticorps selon la revendication 14 ou 15,
a) mise en évidence du complexe antigène - anticorps formé.

17. Kit pour la détection discriminante de la présence d'un antigène de *M. bovis* BCG et *M. bovis* par rapport à un antigène de *M. tuberculosis* dans un échantillon biologique comprenant :
a) un anticorps tel que défini dans la revendication 14 ou 15,
b) les réactifs pour la constitution du milieu propice à la réaction immunologique,
c) les réactifs permettant la détection des complexes antigène - anticorps produits par la réaction immunologique.

18. Composition immunogène **caractérisée en ce qu'**elle consiste en au moins un produit d'expression des séquences nucléotidiques absentes des génomes de *M. bovis* BCG et de *M. bovis* et présentes dans *M. tuberculosis* choisies parmi les ORFs et gènes suivants : Rv2346c, Rv2347c, Rv2348c, *plcC, plcb, plcA,* Rv2352c, Rv2353c, Rv3425, Rv3426, Rv3427c, Rv3428c, Rv1964, Rv1965, *mce3,* Rv1967, Rv1968, Rv1969, /*prM,* Rv1971, Rv1972, Rv1973, Rv1974, Rv1975, Rv1976c, Rv1977, *ephA,* Rv3618, Rv3619c, Rv3620c, Rv3621c, Rv3622c, *lpgG, cobL,* Rv2073c, Rv2074, Rv2075c, *echAl,* Rv0223c, tels que définis dans le tableau 1 ou présentes dans les génomes de *M.* bovis BCG et de *M. bovis* et absentes du génome de *M. tuberculosis* choisies parmi les ORFs et gènes suivants : RvD1-ORF1, RvD1-ORF2, RvD1-Rv2024c, RvD2-*plcD,* RvD2-ORF1, RvD2-ORF2, RvD2-ORF3, RvD2-Rv1758, la séquence nucléotidique de RvD1 ayant été déposée sous le n° Y18605 et la séquence nucléotidique de RvD2 ayant été déposée sous le n° Y18606 dans la base de données EMBL.

19. Composition immunogène telle que définie à la revendication 18, **caractérisée en ce que** ledit au moins un produit d'expression est en association avec un véhicule pharmaceutiquement acceptable et, éventuellement, avec un ou plusieurs adjuvants de l'immunité.

20. Composition immunogène selon la revendication 18, **caractérisée en ce** ' que lesdites séquences nucléotides sont regroupées en régions nucléotidiques RD5 à RD10 et RvD1 et RvD2 selon la répartition suivante :
- RD5 : Rv2346c, Rv2347c, Rv2348c, *plcC, plcB, plcA,* Rv2352c, Rv2353c, tels que définis dans le tableau 1,
- RD6 : Rv3425, Rv3426, Rv3427c, Rv3428c, tels que définis dans le tableau 1,
- RD7 : Rv1964, Rv1965, *mce3,* Rv1967, Rv1968, Rv1969, *lprM,* Rv1971, Rv1972, Rv1973, Rv1974, Rv1975, Rv1976c, Rv1977, tels que définis, dans le tableau 1,
- RD8 : *ephA,* Rv3618, Rv3619c, Rv3620c, Rv3621c, Rv3622c, *lpqG,* tels que définis dans le tableau 1,
- RD9 : *cobL,* Rv2073c, Rv2074, Rv2075c, tels que définis dans le tableau 1,
- RD10 : *echAl,* Rv0223c, tels que définis dans le tableau 1,
- RvD1: RvD1-ORF1, RvD1-ORF2, RvD1-Rv2024c, la séquence nucléotidique de RvD1 ayant été déposée sous le n° Y18605 dans la base de données EMBL,
- RvD2: RvD2-plcD, RvD2-ORF1, RvD2-ORF2, RvD2-ORF3, RvD2-Rv1758, la séquence nucléotidique de RvD2 ayant été déposée sous le n° Y18606 dans la base de données EMBL.

## Claims

1. Detection and identification method for discriminating *M. bovis* BCG and *M. bovis* from *M. tuberculosis* in a biological sample, comprising the following steps:
a) isolation of the DNA from the biological sample to be analyzed or production of a cDNA from the RNA of the biological sample,
b) detection of the DNA sequences of the mycobacterium present in said biological sample,
c) comparison of said sequences with the nucleotide sequences absent from the genomes of *M. bovis* BCG and *M. bovis* and present in the genome of *M. tuberculosis* chosen from the following ORFs and genes: Rv2346c, Rv2347c, Rv2348c, *plcC, plcB, plcA,* Rv2352c, Rv2353c, Rv3425, Rv3426, Rv3427c, Rv3428c, Rv1964, Rv1965, *mce3,* Rv1967, Rv1968, Rv1969, *lprM,* Rv1971, Rv1972, Rv1973, Rv1974, Rv1975, Rv1976c, Rv1977, *ephA,* Rv3618, Rv3619c, Rv3620c, Rv3621c, Rv3622c, *lpqG, cobL,* Rv2073c, Rv2074, Rv2075c, *echAl*, Rv0223c, as defined in Table 1.
d) comparison of said sequences with the nucleotide sequences present in the genomes of *M. bovis* BCG and *M. bovis* and absent from the genome of *M. tuberculosis* chosen from the following ORFs and genes: RvD1-ORF1, RvD1-ORF2, RvD1-Rv2024c, RvD2-plcD, RvD2-ORF1, RvD2-ORF2, RvD2-ORF3, RvD2-Rv1758, the nucleotide sequence of RvD1 having been deposited under the No. Y18605 and the nucleotide sequence of RvD2 having been deposited under the No. Y18606 in the EMBL database.

2. Detection and identification method according to Claim 1, **characterized in that** the nucleotide sequences as defined in steps c) and d) are grouped together in nucleotide regions RD5 to RD10 and RvD1 and RvD2 according to the following distribution:
- RD5: Rv2346c, Rv2347c, Rv2348c, *plcC, plcB,* plcA, Rv2352c, Rv2353c,
- RD6: Rv3425, Rv3426, Rv3427c, Rv3428c,
- RD7: Rv1964, Rv1965, *mce3,* Rv1967, Rv1968, Rv1969, *lprM,* Rv1971, Rv1972, Rv1973, Rv1974, Rv1975, Rv1976c, Rv1977,
- RD8: ephA, Rv3618, Rv3619c, Rv3620c, Rv3621c, Rv3622c, *lpqG*,
- RD9: *cobL,* Rv2073c, Rv2074, Rv2075c,
- RD10: *echAl*, Rv0223c,
- RvD1: RvD1-ORF1, RvD1-ORF2, RvD1-Rv2024c
- RvD2: RvD2-plcD, RvD2-ORF1, RvD2-ORF2, RvD2-ORF3, RvD2-Rv1758.

3. Method according to Claim 1 or 2, in which the detection of the mycobacterial DNA sequences is carried out using nucleotide sequences complementary to said DNA sequences.

4. Method according to Claim 3, in which the detection of the mycobacterial DNA sequences is carried out by amplification of these sequences using primers.

5. Method according to Claim 4, in which the primers have a nucleotide sequence chosen from the group comprising SEQ ID No. 3, SEQ ID No. 4, SEQ ID No. 5, SEQ ID No. 6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17, and SEQ ID No. 18 with:
- the pair SEQ ID No. 3/SEQ ID No. 4 specific for RD5,
- the pair SEQ ID No. 5/SEQ ID No. 6 specific for RD6,
- the pair SEQ ID No. 7/SEQ ID No. 8 specific for RD7,
- the pair SEQ ID No. 9/SEQ ID No. 10 specific for RD8,
- the pair SEQ ID No. 11/SEQ ID No. 12 specific for RD9,
- the pair SEQ ID No. 13/SEQ ID No. 14 specific for RD10,
- the pair SEQ ID No. 15/SEQ ID No. 16 specific for RvD1, and
- the pair SEQ ID No. 17/SEQ ID No. 18 specific for RvD2.

6. Detection and identification kit for discriminating *M. bovis* BCG and *M*. *bovis* from *M*. *tuberculosis* in a biological sample comprising the following elements:
a) at least one pair of primers as defined in Claim 5,
b) the reagents necessary to carry out a DNA amplification reaction,
c) optionally, the necessary components which make it possible to verify or compare the sequence and/or the size of the amplified fragment, for example reagents necessary for electrophoresis or chromatography, or probes necessary for molecular hybridization.

7. Use of at least one pair of primers as defined in Claim 5 for the amplification of a DNA sequence from *M. bovis* BCG, *M. bovis* or *M. tuberculosis.*

8. *In vitro* antibody detection method for discriminating antibodies directed against *M. bovis* BCG and *M. bovis* from antibodies directed against *M. tuberculosis* in a biological sample, comprising the following steps:
a) bringing the biological sample into contact with at least one product of expression of the nucleotide sequences absent from the genomes of *M. bovis* BCG and *M. bovis* and present in the genome of *M. tuberculosis* chosen from the following ORFs and genes: Rv2346c, Rv2347c, Rv2348c, *plcC, plcB, plcA,* Rv2352c, Rv2353c, Rv3425, Rv3426, Rv3427c, Rv3428c, Rv1964, Rv1965, *mce*3, Rv1967, Rv1968, Rv1969, *lprM,* Rv1971, Rv1972, Rv1973, Rv1974, Rv1975, Rv1976c, Rv1977, *ephA*, Rv3618, Rv3619c, Rv3620c, Rv3621c, Rv3622c, *lpqG, cobL,* Rv2073c, Rv2074, Rv2075c, *echAl*, Rv0223c, as defined in Table 1 or present in the genomes of *M. bovis* BCG and *M. bovis* and absent from the genome of *M. tuberculosis* chosen from the following ORFs and genes: RvD1-ORF1, RvD1-ORF2, RvD1-Rv2024c, RvD2-plcD, RvD2-ORF1, RvD2-ORF2, RvD2-ORF3, RvD2-Rv1758, the nucleotide sequence of RvD1 having been deposited under the No. Y18605 and the nucleotide sequence of RvD2 having been deposited under the No. Y18606 in the EMBL database,
b) detecting the antigen-antibody complex formed.

9. Method of discriminatory detection according to Claim 8, **characterized in that** the nucleotide sequences as defined in step a) of said method are grouped together in nucleotide regions RD5 to RD10 and RvD1 and RvD2 according to the following distribution:
- RD5: Rv2346c, Rv2347c, Rv2348c, *plcC, plcB, pleA,* Rv2352c, Rv2353c, as defined in Table 1,
- RD6: Rv3425, Rv3426, Rv3427c, Rv3428c, as defined in Table 1,
- RD7: Rv1964, Rv1965, *mce3,* Rv1967, Rv1968, Rv1969, *lprM,* Rv1971, Rv1972, Rv1973, Rv1974, Rv1975, Rv1976c, Rv1977, as defined in Table 1,
- RD8: *ephA,* Rv3618, Rv3619c, Rv3620c, Rv3621c, Rv3622c, *lpqG,* as defined in Table 1,
- RD9: *cobL,* Rv2073c, Rv2074, Rv2075c, as defined in Table 1,
- RD10: *echAl*, Rv0223c, as defined in Table 1,
- RvD1: RvD1-ORF1, RvD1-ORF2, RvD1-Rv2024c, the nucleotide sequence of RvD1 having been deposited under the No. Y18605 in the EMBL database,
- RvD2: RvD2-plcD, RvD2-ORF1, RvD2-ORF2, RvD2-ORF3, RvD2-Rv1758, the nucleotide sequence of RvD2 having been deposited under the No. Y18606 in the EMBL database.

10. *In vitro* detection method for discriminating a vaccination with *M. bovis* BCG from an infection by *M. tuberculosis* in a mammal, comprising the following steps:
a) incubation of cells of said mammal, more particularly cells of the immune system of said mammal and more particularly still T cells with at least one product of expression of the nucleotide sequences absent from the genomes of *M. bovis* BCG and *M. bovis* and present in *M. tuberculosis* chosen from the following ORFs and genes: Rv2346c, Rv2347c, Rv2348c, *plcC, plcB, plcA,* Rv2352c, Rv2353c, Rv3425, Rv3426, Rv3427c, Rv3428c, Rv1964, Rv1965, *mce3,* Rv1967, Rv1968, Rv1969, *lprM,* Rv1971, Rv1972, Rv1973, Rv1974, Rv1975, Rv1976c, Rv1977, *ephA,* Rv3618, Rv3619c, Rv3620c, Rv3621c, Rv3622c, *lpqG, cobL,* Rv2073c, Rv2074, Rv2075c, *echAl*, Rv0223c, as defined in Table 1 or present in the genomes of *M. bovis* BCG and *M. bovis* and absent from the genome of *M. tuberculosis* chosen from the following ORFs and genes: RvD1-ORF1, RvD1-ORF2, RvD1-Rv2024c, RvD2-plcD, RvD2-ORF1, RvD2-ORF2, RvD2-ORF3, RvD2-Rv1758, the nucleotide sequence of RvD1 having been deposited under the No. Y18605 and the nucleotide sequence of RvD2 having been deposited under the No. Y18606 in the EMBL database,
b) detection of a cellular reaction indicating prior sensitization of the mammal to said product, in particular cell proliferation and/or synthesis of proteins such as gamma-interferon.

11. Method of discriminatory detection according to Claim 10, **characterized in that** the nucleotide sequences as defined in step a) of said method are grouped together in nucleotide regions RD5 to RD10 and RvD1 and RvD2 according to the following distribution:
- RD5: Rv2346c, Rv2347c, Rv2348c, *plcC, plcB, plcA,* Rv2352c, Rv2353c, as defined in Table 1,
- RD6: Rv3425, Rv3426, Rv3427c, Rv3428c, as defined in Table 1,
- RD7: Rv1964, Rv1965, *mce3,* Rv1967, Rv1968, Rv1969, *lprM,* Rv1971, Rv1972, Rv1973, Rv1974, Rv1975, Rv1976c, Rv1977, as defined in Table 1,
- RD8: *ephA*, Rv3618, Rv3619c, Rv3620c, Rv3621c, Rv3622c, *lpqG,* as defined in Table 1,
- RD9: *cobL,* Rv2073c, Rv2074, Rv2075c, as defined in Table 1,
- RD10: *echAl,* Rv0223c, as defined in Table 1,
- RvD1: RvD1-ORF1, RvD1-ORF2, RvD1-Rv2024c, the nucleotide sequence of RvD1 having been deposited under the No. Y18605 in the EMBL database,
- RvD2: RvD2-plcD, RvD2-ORF1, RvD2-ORF2, RvD2-ORF3, RvD2-Rv1758, the nucleotide sequence of RvD2 having been deposited under the No. Y18606 in the EMBL database.

12. Kit for the *in vitro* diagnosis of an *M. tuberculosis* infection in a mammal optionally vaccinated beforehand with *M. bovis* BCG, comprising:
a) a product of expression of the nucleotide sequences absent from the genomes of *M. bovis* BCG and *M. bovis* and present in *M. tuberculosis* chosen from the following ORFs and genes: Rv2346c, Rv2347c, Rv2348c, *plcC, plcB, plcA,* Rv2352c, Rv2353c, Rv3425, Rv3426, Rv3427c, Rv3428c, Rv1964, Rv1965, *mce3,* Rv1967, Rv1968, Rv1969, *lprM*, Rv1971, Rv1972, Rv1973, Rv1974, Rv1975, Rv1976c, Rv1977, *ephA,* Rv3618, Rv3619c, Rv3620c, Rv3621c, Rv3622c, *lpqG, cobL,* Rv2073c, Rv2074, Rv2075c, *echAl,* Rv0223c, as defined in Table 1 or present in the genomes of *M. bovis* BCG and *M*. *bovis* and absent from the genome of *M. tuberculosis* chosen from the following ORFs and genes: RvD1-ORF1, RvD1-ORF2, RvD1-Rv2024c, RvD2-plcD, RvD2-ORF1, RvD2-ORF2, RvD2-ORF3, RvD2-Rv1758, the nucleotide sequence of RvD1 having been deposited under the No. Y18605 and the nucleotide sequence of RvD2 having been deposited under the No. Y18606 in the EMBL database,
b) where appropriate, the reagents for the constitution of the medium suitable for the immunological reaction,
c) the reagents allowing the detection of the antigen-antibody complexes produced by the immunological reaction,
d) where appropriate, a reference biological sample (negative control) free of antibodies recognized by said product,
e) where appropriate, a reference biological sample (positive control) containing a predetermined quantity of antibodies recognized by said product.

13. Kit according to Claim 12, **characterized in that** the nucleotide sequences as defined in part a) of said kit are grouped together in nucleotide regions RD5 to RD10 and RvD1 and RvD2 according to the following distribution:
- RD5: Rv2346c, Rv2347c, Rv2348c, *plcC, plcB, plcA,* Rv2352c, Rv2353c, as defined in Table 1,
- RD6: Rv3425, Rv3426, Rv3427c, Rv3428c, as defined in Table 1,
- RD7: Rv1964, Rv1965, *mce3,* Rv1967, Rv1968, Rv1969, *lprM,* Rv1971, Rv1972, Rv1973, Rv1974, Rv1975, Rv1976c, Rv1977, as defined in Table 1,
- RD8: *ephA,* Rv3618, Rv3619c, Rv3620c, Rv3621c, Rv3622c, *lpqG,* as defined in Table 1,
- RD9: *cobL,* Rv2073c, Rv2074, Rv2075c, as defined in Table 1,
- RD10: *echA1,* Rv0223c, as defined in Table 1,
- RvD1: RvD1-ORF1, RvD1-ORF2, RvD1-Rv2024c, the nucleotide sequence of RvD1 having been deposited under the No. Y18605 in the EMBL database,
- RvD2: RvD2-plcD, RvD2-ORF1, RvD2-ORF2, RvD2-ORF3, RvD2-Rv1758, the nucleotide sequence of RvD2 having been deposited under the No. Y18606 in the EMBL database.

14. Mono- or polyclonal antibodies, their fragments or chimeric antibodies, **characterized in that** they are capable of specifically recognizing a product of expression of the nucleotide sequences absent from the genomes of *M*. *bovis* BCG and *M*. *bovis* and present in *M*. *tuberculosis* chosen from the following ORFs and genes: Rv2346c, Rv2347c, Rv2348c, *plcC, plcB, plcA,* Rv2352c, Rv2353c, Rv3425, Rv3426, Rv3427c, Rv3428c, Rv1964, Rv1965, mce3, Rv1967, Rv1968, Rv1969, *lprM,* Rv1971, Rv1972, Rv1973, Rv1974, Rv1975, Rv1976c, Rv1977, *ephA,* Rv3618, Rv3619c, Rv3620c, Rv3621c, Rv3622c, *lpqG, cobL,* Rv2073c, Rv2074, Rv2075c, *echA1,* Rv0223c, as defined in Table 1 or present in the genomes of *M. bovis* BCG and *M. bovis* and absent from the genome of *M. tuberculosis* chosen from the following ORFs and genes: RvD1-ORF1, RvD1-ORF2, RvD1-Rv2024c, RvD2-plcD, RvD2-ORF1, RvD2-ORF2, RvD2-ORF3, RvD2-Rv1758, the nucleotide sequence of RvD1 having been deposited under the No. Y18605 and the nucleotide sequence of RvD2 having been deposited under the No. Y18606 in the EMBL database.

15. Mono- or polyclonal antibodies, their fragments or chimeric antibodies according to Claim 14, **characterized in that** said nucleotide sequences are grouped together in nucleotide regions RD5 to RD10 and RvD1 and RvD2 according to the following distribution:
- RD5: Rv2346c, Rv2347c, Rv2348c, *plcC, plcB, plcA,* Rv2352c, Rv2353c, as defined in Table 1,
- RD6: Rv3425, Rv3426, Rv3427c, Rv3428c, as defined in Table 1,
- RD7: Rv1964, Rv1965, *mce3,* Rv1967, Rv1968, Rv1969, *lprM,* Rv1971, Rv1972, Rv1973, Rv1974, Rv1975, Rv1976c, Rv1977, as defined in Table 1,
- RD8: *ephA,* Rv3618, Rv3619c, Rv3620c, Rv3621c, Rv3622c, *lpqG,* as defined in Table 1,
- RD9: *cobL*, Rv2073c, Rv2074, Rv2075c, as defined in Table 1,
- RD10: *echA1,* Rv0223c, as defined in Table 1,
- RvD1: RvD1-ORF1, RvD1-ORF2, RvD1-Rv2024c, the nucleotide sequence of RvD1 having been deposited under the No. Y18605 in the EMBL database,
- RvD2: RvD2-plcD, RvD2-ORF1, RvD2-ORF2, RvD2-ORF3, RvD2-Rv1758, the nucleotide sequence of RvD2 having been deposited under the No. Y18606 in the EMBL database.

16. Method for the detection of the presence of an antigen making it possible to discriminate an antigen of *M. bovis* BCG and *M. bovis* from an antigen of *M. tuberculosis* in a biological sample comprising the following steps:
a) bringing the biological sample into contact with an antibody according to Claim 14 or 15,
b) detecting the antigen-antibody complex formed.

17. Kit for the discriminatory detection of the presence of an antigen of *M. bovis* BCG and *M. bovis* in relation to an antigen of *M. tuberculosis* in a biological sample comprising:
a) an antibody as defined in Claim 14 or 15,
b) the reagents for constituting the medium suitable for the immunological reaction,
c) the reagents allowing the detection of the antigen-antibody complexes produced by the immunological reaction.

18. Immunological composition, **characterized in that** it consists of at least one product of expression of the nucleotide sequences absent from the genomes of *M. bovis* BCG and *M. bovis* and present in *M. tuberculosis* chosen from the following ORFs and genes: Rv2346c, Rv2347c, Rv2348c, *plcC, plcB, plcA,* Rv2352c, Rv2353c, Rv3425, Rv3426, Rv3427c, Rv3428c, Rv1964, Rv1965, *mce3,* Rv1967, Rv1968, Rv1969, *lprM,* Rv1971, Rv1972, Rv1973, Rv1974, Rv1975, Rv1976c, Rv1977, *ephA,* Rv3618, Rv3619c, Rv3620c, Rv3621c, Rv3622c, *lpqG, cobL,* Rv2073c, Rv2074, Rv2075c, *echA1,* Rv0223c, as defined in Table 1 or present in the genomes of *M. bovis* BCG and *M. bovis* and absent from the genome of *M. tuberculosis* chosen from the following ORFs and genes: RvD1-ORF1, RvD1-ORF2, RvD1-Rv2024c, RvD2-*plcD,* RvD2-ORF1, RvD2-ORF2, RvD2-ORF3, RvD2-Rv1758, the nucleotide sequence of RvD1 having been deposited under the No. Y18605 and the nucleotide sequence of RvD2 having been deposited under the No. Y18606 in the EMBL database.

19. Immunological composition as defined in Claim 18, **characterized in that** said at least one product of expression is in combination with a pharmaceutically acceptable vehicle and, optionally, with one or more immunity adjuvants.

20. Immunological composition according to Claim 18, **characterized in that** said nucleotide sequences are grouped together in nucleotide regions RD5 to RD10 and RvD1 and RvD2 according to the following distribution:
- RD5: Rv2346c, Rv2347c, Rv2348c, *plcC, plcB, plcA,* Rv2352c, Rv2353c, as defined in Table 1,
- RD6: Rv3425, Rv3426, Rv3427c, Rv3428c, as defined in Table 1,
- RD7: Rv1964, Rv1965, *mce3,* Rv1967, Rv1968, Rv1969, *lprM,* Rv1971, Rv1972, Rv1973, Rv1974, Rv1975, Rv1976c, Rv1977, as defined in Table 1,
- RD8: *ephA,* Rv3618, Rv3619c, Rv3620c, Rv3621c, Rv3622c, *lpqG,* as defined in Table 1,
- RD9: *cobL,* Rv2073c, Rv2074, Rv2075c, as defined in Table 1,
- RD10: *echA1,* Rv0223c, as defined in Table 1,
- RvD1: RvD1-ORF1, RvD1-ORF2, RvD1-Rv2024c, the nucleotide sequence of RvD1 having been deposited under the No. Y18605 in the EMBL database,
- RvD2: RvD2-plcD, RvD2-ORF1, RvD2-ORF2, RvD2-ORF3, RvD2-Rv1758, the nucleotide sequence of RvD2 having been deposited under the No. Y18606 in the EMBL database.

## Patentansprüche

1. Detektions- und Identifizierungsverfahren, das es ermöglicht, in einer biologischen Probe *M. bovis* BCG und *M. bovis* von *M. tuberculosis* zu unterscheiden, und das die folgenden Schritte umfasst:
a) Isolierung der DNA aus der zu analysierenden biologischen Probe oder Gewinnung einer cDNA aus der RNA der biologischen Probe,
b) Detektion der DNA-Sequenzen des Mykobakteriums, das in der biologischen Probe vorkommt,
c) Abgleich der Sequenzen mit den Nukleotidsequenzen, die in den Genomen von *M*. *bovi s* BCG und von *M. bovi s* fehlen und im Genom von *M*. *tuberculosis* vorhanden sind und die aus den folgenden ORFs und Genen gewählt sind: Rv2346c, Rv2347c, Rv2348c, *plcC, plcB, plcA,* Rv2352c, Rv2353c, Rv3425, Rv3426, Rv3427c, Rv3428c, Rv1964, Rv1965, *mce3*, Rv1967, Rv1968, Rv1969, *lprM,* Rv1971, Rv1972, Rv1973, Rv1974, Rv1975, Rv1976c, Rv1977, *ephA,* Rv3618, Rv3619c, Rv3620c, Rv3621c, Rv3622c, *lpgG, cobL,* Rv2073c, Rv2074, Rv2075c, *echA1,* Rv0223c, gemäß den Begriffsbestimmungen in Tabelle 1.
d) Abgleich der Sequenzen mit den Nukleotidsequenzen, die in den Genomen von *M*. *bovis BCG* und von *M. bovis* vorhanden sind und im Genom von *M. tuberculosis* fehlen und die aus den folgenden ORFs und Genen gewählt sind: RvD1-ORF1, RvD1-ORF2, RvD1-Rv2024c, RvD2-*plcD*, RvD2-ORF1, RvD2-ORF2, RvD2-ORF3, RvD2-Rv1758, wobei die Nukleotidsequenz von RvD1 unter der Nr. Y18605 und die Nukleotidsequenz von RvD2 unter der Nr. Y18606 bei der EMBL-Datenbank eingereicht wurden.

2. Detektions- und Identifikationsverfahren nach Anspruch 1, **dadurch gekennzeichnet, dass** die Nukleotidsequenzen gemäß den Begriffsbestimmungen in den Schritten c) und d) nach der folgenden Einteilung zu Nukleotidregionen mit den Bezeichnungen RD5 bis RD10 sowie RvD1 und RvD2 zusammengefasst werden:
- RD5: Rv2346c, Rv2347c, Rv2348c, *plcC, plcB, plcA,* Rv2352c, Rv2353c,
- RD6: Rv3425, Rv3426, Rv3427c, Rv3428c,
- RD7: Rv1964, Rv1965, *mce3,* Rv1967, Rv1968, Rv1969, *lprM,* Rv1971, Rv1972, Rv1973, Rv1974, Rv1975, Rv1976c, Rv1977,
- RD8: *ephA,* Rv3618, Rv3619c, Rv3620c, Rv3621c, Rv3622c, *lpqG,*
- RD9: *cobL,* Rv2073c, Rv2074, Rv2075c,
- RD10: *echA1,* Rv0223c,
- RvD1: RvD1-ORF1, RvD1-ORF2, RvD1-Rv2024c,
- RvD2: RvD2-plcD, RvD2-ORF1, RvD2-ORF2, RvD2-ORF3, RvD2-Rv1758.

3. Verfahren nach den Ansprüchen 1 oder 2, wobei die Detektion der DNA-Sequenzen des Mykobakteriums mittels Nukleotidsequenzen durchgeführt wird, die zu diesen DNA-Sequenzen komplementär sind.

4. Verfahren nach Anspruch 3, wobei die Detektion der DNA-Sequenzen des Mykobakteriums durch Verstärkung dieser Sequenzen mit Hilfe von Primern erfolgt.

5. Verfahren nach Anspruch 4, wobei die Primer eine Nukleotidsequenz aufweisen, die aus der Gruppe gewählt ist, welche die SEQ-ID-Nr. 3, SEQ-ID-Nr. 4, SEQ-ID-Nr. 5, SEQ-ID-Nr. 6, SEQ-ID-Nr. 7, SEQ-ID-Nr. 8, SEQ-ID-Nr. 9, SEQ-ID-Nr. 10, SEQ-ID-Nr. 11, SEQ-ID-Nr. 12, SEQ-ID-Nr. 13, SEQ-ID-Nr. 14, SEQ-ID-Nr. 15, SEQ-ID-Nr. 16, SEQ-ID-Nr. 17 und SEQ-ID-Nr. 18 umfasst, wobei:
- das Paar SEQ-ID-Nr. 3 / SEQ-ID-Nr. 4 für RD5 spezifisch ist,
- das Paar SEQ-ID-Nr. 5 / SEQ-ID-Nr. 6 für RD6 spezifisch ist,
- das Paar SEQ-ID-Nr. 7 / SEQ-ID-Nr. 8 für RD7 spezifisch ist,
- das Paar SEQ-ID-Nr. 9 / SEQ-ID-Nr. 10 für RD8 spezifisch ist,
- das Paar SEQ-ID-Nr. 11 / SEQ-ID-Nr. 12 für RD9 spezifisch ist,
- das Paar SEQ-ID-Nr. 13 / SEQ-ID-Nr. 14 für RD10 spezifisch ist,
- das Paar SEQ-ID-Nr. 15 / SEQ-ID-Nr. 16 für RvD1 spezifisch ist und
- das Paar SEQ-ID-Nr. 17 / SEQ-ID-Nr. 18 für RvD2 spezifisch ist.

6. Anwendungseinheit für die Detektion und die Identifizierung, welche es ermöglicht, in einer biologischen Probe *M. bovis* BCG und *M*. *bovis* von *M. tuberculosis* zu unterscheiden, und welche die folgenden Elemente umfasst:
a) mindestens ein Primerpaar gemäß der Begriffsbestimmung in Anspruch 5,
b) die Reagenzien, die zur Durchführung einer DNA-Verstärkungsreaktion erforderlich sind,
c) möglicherweise diejenigen Elemente, die dazu erforderlich sind, die Sequenz und/oder die Größe des verstärkten Fragments überprüfen oder abgleichen zu können, wobei es sich beispielsweise um Reagenzien handelt, die für eine Elektrophorese oder eine Chromatografie erforderlich sind, oder aber um Sonden, die für eine molekulare Hybridisierung erforderlich sind.

7. Verwendung mindestens eines Primerpaares gemäß der Begriffsbestimmung in Anspruch 5 zur Verstärkung der DNA-Sequenz von *M. bovis* BCG, *M. bovis* oder *M. tuberculosis.*

8. Verfahren zur in-vitro-Detektion von Antikörpern, welches es ermöglicht, in einer biologischen Probe Antikörper, die gegen *M*. *bovis* BCG et *M*. *bovis* gerichtet sind, von Antikörpern zu unterscheiden, die gegen *M*. *tuberculosis* gerichtet sind, und welches die folgenden Schritte umfasst:
a) Inkontaktbringen der biologischen Probe mit mindestens einem Expressionsprodukt der Nukleotidsequenzen, die in den Genomen von *M. bovis* BCG und von *M. bovis* fehlen und im Genom von *M. tuberculosis* vorhanden sind und die aus den folgenden ORFs und Genen gewählt sind: Rv2346c, Rv2347c, Rv2348c, *plcC, plcB, plcA,* Rv2352c, Rv2353c, Rv3425, Rv3426, Rv3427c, Rv3428c, Rv1964, Rv1965, *mce3,* Rv1967, Rv1968, Rv1969, *lprM,* Rv1971, Rv1972, Rv1973, Rv1974, Rv1975, Rv1976c, Rv1977, *ephA,* Rv3618, Rv3619c, Rv3620c, Rv3621c, Rv3622c, *lpqG, cobL*, Rv2073c, Rv2074, Rv2075c, *echA1,* Rv0223c, gemäß den Begriffsbestimmungen in Tabelle 1, oder die in den Genomen von *M*. *bovi s* BCG und von *M*. *bovis* vorhanden sind und im Genom von *M*. *tuberculosis* fehlen und die aus den folgenden ORFs und Genen gewählt sind: RvD1-ORF1, RvD1-ORF2, RvD1-Rv2024c, RvD2-plCD, RvD2-ORF1, RvD2-ORF2, RvD2-ORF3, RvD2-Rv1758, wobei die Nukleotidsequenz von RvD1 unter der Nr. Y18605 und die Nukleotidsequenz von RvD2 unter der Nr. Y18606 bei der EMBL-Datenbank eingereicht wurden,
b) Nachweis des gebildeten Antigen-Antikörper-Komplexes.

9. Verfahren zur unterscheidenden Detektion nach Anspruch 8, **dadurch gekennzeichnet, dass** bei diesem Verfahren die Nukleotidsequenzen gemäß den Begriffsbestimmungen in Schritt a) nach der folgenden Einteilung zu Nukleotidregionen mit den Bezeichnungen RD5 bis RD10 sowie RvD1 und RvD2 zusammengefasst werden:
- RD5: Rv2346c, Rv2347c, Rv2348c, *plcC, plcB, plcA,* Rv2352c, Rv2353c, gemäß den Begriffsbestimmungen in Tabelle 1,
- RD6: Rv3425, Rv3426, Rv3427c, Rv3428c, gemäß den Begriffsbestimmungen in Tabelle 1,
- RD7: Rv1964, Rv1965, *mce3,* Rv1967, Rv1968, Rv1969, *lprM,* Rv1971, Rv1972, Rv1973, Rv1974, Rv1975, Rv1976c, Rv1977, gemäß den Begriffsbestimmungen in Tabelle 1,
- RD8: *ephA,* Rv3618, Rv3619c, Rv3620c, Rv3621c, Rv3622c, *lpgG,* gemäß den Begriffsbestimmungen in Tabelle 1,
- RD9: *cobL,* Rv2073c, Rv2074, Rv2075c, gemäß den Begriffsbestimmungen in Tabelle 1,
- RD10: *echA1,* Rv0223c, gemäß den Begriffsbestimmungen in Tabelle 1,
- RvD1: RvD1-ORF1, RvD1-ORF2, RvD1-Rv2024c, wobei die Nukleotidsequenz von RvD1 unter der Nr. Y18605 bei der EMBL-Datenbank eingereicht wurde,
- RvD2: RvD2-plcD, RvD2-ORF1, RvD2-ORF2, RvD2-ORF3, RvD2-Rv1758, wobei die Nukleotidsequenz von RvD2 unter der Nr. Y18606 bei der EMBL-Datenbank eingereicht wurde.

10. Verfahren zur *in-vitro*-Detektion, welches es ermöglicht, bei einem Säugetier eine Impfung mit *M. bovis* BCG von einer Infektion mit *M. tuberculosis* zu unterscheiden, und welches die folgenden Schritte umfasst:
a) Inkubation von Zellen des Säugetiers, insbesondere von Zellen des Immunsystems dieses Säugetiers und ganz besonders von T-Zellen, mit mindestens einem Expressionsprodukt der Nukleotidsequenzen, die in den Genomen von *M*. *bovis* BCG und von *M. bovis* fehlen und in *M. tuberculosis* vorhanden sind und die aus den folgenden ORFs und Genen gewählt sind: Rv2346c, Rv2347c, Rv2348c, *plcC, plcB, plcA,* Rv2352c, Rv2353c, Rv3425, Rv3426, Rv3427c, Rv3428c, Rv1964, Rv1965, mce3, Rv1967, Rv1968, Rv1969, *lprM,* Rv1971, Rv1972, Rv1973, Rv1974, Rv1975, Rv1976c, Rv1977, *ephA,* Rv3618, Rv3619c, Rv3620c, Rv3621c, Rv3622c, *lpqG, cobL*, Rv2073c, Rv2074, Rv2075c, *echA1,* Rv0223c, gemäß den Begriffsbestimmungen in Tabelle 1, oder die in den Genomen von *M. bovis BCG* und von *M. bovis* vorhanden sind und im Genom von *M. tuberculosis* fehlen und die aus den folgenden ORFs und Genen gewählt sind: RvD1-ORF1, RvD1-ORF2, RvD1-Rv2024c, *RvD2-plcD,* RvD2-ORF1, RvD2-ORF2, RvD2-ORF3, RvD2-Rv1758, wobei die Nukleotidsequenz von RvD1 unter der Nr. Y18605 und die Nukleotidsequenz von RvD2 unter der Nr. Y18606 bei der EMBL-Datenbank eingereicht wurden,
b) Detektion einer Zellreaktion, die eine vorherige Sensibilisierung des Säugetiers gegenüber dem Produkt anzeigt, wobei es sich insbesondere um die Zellvermehrung und/oder um die Synthese von Proteinen wie etwa von gamma-Interferon handelt.

11. Verfahren zur unterscheidenden Detektion nach Anspruch 10, **dadurch gekennzeichnet, dass** bei diesem Verfahren die Nukleotidsequenzen gemäß den Begriffsbestimmungen in Schritt a) nach der folgenden Einteilung zu Nukleotidregionen mit den Bezeichnungen RD5 bis RD10 sowie RvD1 und RvD2 zusammengefasst werden:
- RD5: Rv2346c, Rv2347c, Rv2348c, *plcC, plcB, plcA*, Rv2352c, Rv2353c, gemäß den Begriffsbestimmungen in Tabelle 1,
- RD6: Rv3425, Rv3426, Rv3427c, Rv3428c, gemäß den Begriffsbestimmungen in Tabelle 1,
- RD7: Rv1964, Rv1965, *mce3,* Rv1967, Rv1968, Rv1969, *lprM,* Rv1971, Rv1972, Rv1973, Rv1974, Rv1975, Rv1976c, Rv1977, gemäß den Begriffsbestimmungen in Tabelle 1,
- RD8: *ephA,* Rv3618, Rv3619c, Rv3620c, Rv3621c, Rv3622c, *lpqG,* gemäß den Begriffsbestimmungen in Tabelle 1,
- RD9: *cobL*, Rv2073c, Rv2074, Rv2075c, gemäß den Begriffsbestimmungen in Tabelle 1,
- RD10: *echA1,* Rv0223c, gemäß den Begriffsbestimmungen in Tabelle 1,
- RvD1: RvD1-ORF1, RvD1-ORF2, RvD1-Rv2024c, wobei die Nukleotidsequenz von RvD1 unter der Nr. Y18605 bei der EMBL-Datenbank eingereicht wurde,
- RvD2: RvD2-plcD, RvD2-ORF1, RvD2-ORF2, RvD2-ORF3, RvD2-Rv1758, wobei die Nukleotidsequenz von RvD2 unter der Nr. Y18606 bei der EMBL-Datenbank eingereicht wurde.

12. Anwendungseinheit zur in-vitro-Diagnose einer Infektion mit *M*. *tuberculosis* bei einem Säugetier, das möglicherweise zuvor mit *M*. *bovis* BCG geimpft wurde, umfassend:
a) ein Expressionsprodukt der Nukleotidsequenzen, die in den Genomen von *M*. *bovis BCG* und von *M*. *bovis* fehlen und in *M*. *tuberculosis* vorhanden sind und die aus den folgenden ORFs und Genen gewählt sind: Rv2346c, Rv2347c, Rv2348c, *plcC, plcB, plcA,* Rv2352c, Rv2353c, Rv3425, Rv3426, Rv3427c, Rv3428c, Rv1964, Rv1965, *mce3,* Rv1967, Rv1968, Rv1969, *lprM,* Rv1971, Rv1972, Rv1973, Rv1974, Rv1975, Rv1976c, Rv1977, *ephA*, Rv3618, Rv3619c, Rv3620c, Rv3621c, Rv3622c, *lpqG, cobL,* Rv2073c, Rv2074, Rv2075c, *echA1,* Rv0223c, gemäß den Begriffsbestimmungen in Tabelle 1, oder die in den Genomen von *M. bovis BCG* und von *M. bovis* vorhanden sind und im Genom von *M. tuberculosis* fehlen und die aus den folgenden ORFs und Genen gewählt sind: RvD1-ORF1, RvD1-ORF2, RvD1-Rv2024c, RvD2-*plcD*, RvD2-ORF1, RvD2-ORF2, RvD2-ORF3, RvD2-Rv1758, wobei die Nukleotidsequenz von RvD1 unter der Nr. Y18605 und die Nukleotidsequenz von RvD2 unter der Nr. Y18606 bei der EMBL-Datenbank eingereicht wurden.
b) gegebenenfalls Reagenzien zur Herstellung eines Milieus, das für eine immunologische Reaktion geeignet ist,
c) Reagenzien, die es ermöglichen, Antigen-Antikörper-Komplexe, welche durch die immunologische Reaktion entstanden sind, zu detektieren,
d) gegebenenfalls eine biologische Vergleichsprobe (Negativkontrolle), die frei von Antikörpern ist, welche von dem Produkt erkannt werden,
e) gegebenenfalls eine biologische Vergleichsprobe (Positivkontrolle), die eine zuvor festgelegte Menge an Antikörpern enthält, welche von dem Produkt erkannt werden.

13. Anwendungseinheit nach Anspruch 12, **dadurch gekennzeichnet, dass** bei dieser Anwendungseinheit die Nukleotidsequenzen gemäß den Begriffsbestimmungen in Schritt a) nach der folgenden Einteilung zu Nukleotidregionen mit den Bezeichnungen RD5 bis RD10 sowie RvD1 und RvD2 zusammengefasst werden:
- RD5: Rv2346c, Rv2347c, Rv2348c, *plcC, plcB, plcA,* Rv2352c, Rv2353c, gemäß den Begriffsbestimmungen in Tabelle 1,
- RD6: Rv3425, Rv3426, Rv3427c, Rv3428c, gemäß den Begriffsbestimmungen in Tabelle 1,
- RD7: Rv1964, Rv1965, *mce3,* Rv1967, Rv1968, Rv1969, *lprM,* Rv1971, Rv1972, Rv1973, Rv1974, Rv1975, Rv1976c, Rv1977, gemäß den Begriffsbestimmungen in Tabelle 1,
- RD8: *ephA,* Rv3618, Rv3619c, Rv3620c, Rv3621c, Rv3622c, *lpqG*, gemäß den Begriffsbestimmungen in Tabelle 1,
- RD9: *cobL,* Rv2073c, Rv2074, Rv2075c, gemäß den Begriffsbestimmungen in Tabelle 1,
- RD10: *echA1,* Rv0223c, gemäß den Begriffsbestimmungen in Tabelle 1,
- RvD1: RvD1-ORF1, RvD1-ORF2, RvD1-Rv2024c, wobei die Nukleotidsequenz von RvD1 unter der Nr. Y18605 bei der EMBL-Datenbank eingereicht wurde,
- RvD2: RvD2-plcD, RvD2-ORF1, RvD2-ORF2, RvD2-ORF3, RvD2-Rv1758, wobei die Nukleotidsequenz von RvD2 unter der Nr. Y18606 bei der EMBL-Datenbank eingereicht wurde.

14. Mono- oder polyklonale Antikörper, deren Fragmente oder chimärische Antikörper, **dadurch gekennzeichnet, dass** sie dazu befähigt sind, spezifisch ein Expressionsprodukt derjenigen Nukleotidsequenzen zu erkennen, welche in den Genomen von *M*. *bovis BCG* und von *M. bovis* fehlen und in *M. tuberculosis* vorhanden sind und welche aus den folgenden ORFs und Genen gewählt sind: Rv2346c, Rv2347c, Rv2348c, *plcC, plcB, plcA,* Rv2352c, Rv2353c, Rv3425, Rv3426, Rv3427c, Rv3428c, Rv1964, Rv1965, *mce3,* Rv1967, Rv1968, Rv1969, *lprM,* Rv1971, Rv1972, Rv1973, Rv1974, Rv1975, Rv1976c, Rv1977, *ephA,* Rv3618, Rv3619c, Rv3620c, Rv3621c, Rv3622c, *lpqG, cobL,* Rv2073c, Rv2074, Rv2075c, *echA1,* Rv0223c, gemäß den Begriffsbestimmungen in Tabelle 1, oder die in den Genomen von *M*. *bovis BCG* und von *M. bovis* vorhanden sind und im Genom von *M*. *tuberculosis* fehlen und die aus den folgenden ORFs und Genen gewählt sind: RvD1-ORF1, RvD1-ORF2, RvD1-Rv2024c, RvD2-plcD, RvD2-ORF1, RvD2-ORF2, RvD2-ORF3, RvD2-Rv1758, wobei die Nukleotidsequenz von RvD1 unter der Nr. Y18605 und die Nukleotidsequenz von RvD2 unter der Nr. YI8606 bei der EMBL-Datenbank eingereicht wurden.

15. Mono- oder polyklonale Antikörper, deren Fragmente oder chimärische Antikörper nach Anspruch 14, **dadurch gekennzeichnet, dass** die Nukleotidsequenzen nach der folgenden Einteilung zu Nukleotidregionen mit den Bezeichnungen RD5 bis RD10 sowie RvD1 und RvD2 zusammengefasst werden:
- RD5: Rv2346c, Rv2347c, Rv2348c, *plcC, plcB, plcA,* Rv2352c, Rv2353c, gemäß den Begriffsbestimmungen in Tabelle 1,
- RD6: Rv3425, Rv3426, Rv3427c, Rv3428c, gemäß den Begriffsbestimmungen in Tabelle 1,
- RD7: Rv1964, Rv1965, *mce3,* Rv1967, Rv1968, Rv1969, *lprM,* Rv1971, Rv1972, Rv1973, Rv1974, Rv1975, Rv1976c, Rv1977, gemäß den Begriffsbestimmungen in Tabelle 1,
- RD8: *ephA,* Rv3618, Rv3619c, Rv3620c, Rv3621c, Rv3622c, *lpqG,* gemäß den Begriffsbestimmungen in Tabelle 1,
- RD9: *cobL,* Rv2073c, Rv2074, Rv2075c, gemäß den Begriffsbestimmungen in Tabelle 1,
- RD10: *echA1,* Rv0223c, gemäß den Begriffsbestimmungen in Tabelle 1,
- RvD1: RvD1-ORF1, RvD1-ORF2, RvD1-Rv2024c, wobei die Nukleotidsequenz von RvD1 unter der Nr. Y18605 bei der EMBL-Datenbank eingereicht wurde,
- RvD2: RvD2-plcD, RvD2-ORF1, RvD2-ORF2, RvD2-ORF3, RvD2-Rv1758, wobei die Nukleotidsequenz von RvD2 unter der Nr. Y18606 bei der EMBL-Datenbank eingereicht wurde.

16. Verfahren zum Nachweis eines Antigens, welches es ermöglicht, in einer biologischen Probe ein Antigen von *M*. *bovis BCG* und *M*. *bovis* von einem Antigen von *M*. *tuberculosis* zu unterscheiden, und welches die folgenden Schritte umfasst:
a) Inkontaktbringen der biologischen Probe mit einem Antikörper gemäß den Ansprüchen 14 oder 15,
a) Nachweis des gebildeten Antigen-Antikörper-Komplexes.

17. Anwendungseinheit für den Nachweis, in einer biologischen Probe, eines Antigens von *M*. *bovis BCG* und *M*. *bovis* im Unterschied zu einem Antigen von *M*. *tuberculosis,* umfassend:
a) einen Antikörper gemäß der Begriffsbestimmung in den Ansprüchen 14 oder 15,
b) Reagenzien zur Herstellung eines Milieus, das für eine immunologische Reaktion geeignet ist,
c) Reagenzien, die es ermöglichen, Antigen-Antikörper-Komplexe, welche durch die immunologische Reaktion entstanden sind, zu detektieren.

18. Immunogene Zusammensetzung, **dadurch gekennzeichnet, dass** sie aus mindestens einem Expressionsprodukt der Nukleotidsequenzen besteht, welche in den Genomen von *M*. *bovis BCG* und von *M. bovis* fehlen und in *M*. *tuberculosis* vorhanden sind und welche aus den folgenden ORFs und Genen gewählt sind: Rv2346c, Rv2347c, Rv2348c, *plcC, plcB, plcA,* Rv2352c, Rv2353c, Rv3425, Rv3426, Rv3427c, Rv3428c, Rv1964, Rv1965, *mce3,* Rv1967, Rv1968, Rv1969, *lprM,* Rv1971, Rv1972, Rv1973, Rv1974, Rv1975, Rv1976c, Rv1977, *ephA,* Rv3618, Rv3619c, Rv3620c, Rv3621c, Rv3622c, *lpqG, cobL,* Rv2073c, Rv2074, Rv2075c, *echA1,* Rv0223c, gemäß den Begriffsbestimmungen in Tabelle 1, oder die in den Genomen von *M. bovis BCG* und von *M. bovis* vorhanden sind und im Genom von *M. tuberculosis* fehlen und die aus den folgenden ORFs und Genen gewählt sind: RvD1-ORF1, RvD1-ORF2, RvD1-Rv2024c, RvD2-*plcD*, RvD2-ORF1, RvD2-ORF2, RvD2-ORF3, RvD2-Rv1758, wobei die Nukleotidsequenz von RvD1 unter der Nr. Y18605 und die Nukleotidsequenz von RvD2 unter der Nr. Y18606 bei der EMBL-Datenbank eingereicht wurden.

19. Immunogene Zusammensetzung gemäß der Begriffsbestimmung in Anspruch 18, **dadurch gekennzeichnet, dass** mindestens ein Expressionsprodukt in Verbindung mit einem pharmazeutisch annehmbaren Trägerstoff und, möglicherweise, mit einem oder mehreren Immunitäts-Adjuvantien vorliegt.

20. Immunogene Zusammensetzung gemäß dem Anspruch 18, **dadurch gekennzeichnet, dass** die Nukleotidsequenzen nach der folgenden Einteilung zu Nukleotidregionen mit den Bezeichnungen RD5 bis RD10 sowie RvD1 und RvD2 zusammengefasst werden:
- RD5: Rv2346c, Rv2347c, Rv2348c, *plcC*, *plcB, plcA*, Rv2352c, Rv2353c, gemäß den Begriffsbestimmungen in Tabelle 1,
- RD6: Rv3425, Rv3426, Rv3427c, Rv3428c, gemäß den Begriffsbestimmungen in Tabelle 1,
- RD7: Rv1964, Rv1965, *mce3*, Rv1967, Rv1968, Rv1969, *lprM,* Rv1971, Rv1972, Rv1973, Rv1974, Rv1975, Rv1976c, Rv1977, gemäß den Begriffsbestimmungen in Tabelle 1,
- RD8: *ephA,* Rv3618, Rv3619c, Rv3620c, Rv3621c, Rv3622c, *lpqG*, gemäß den Begriffsbestimmungen in Tabelle 1,
- RD9: *cobL*, Rv2073c, Rv2074, Rv2075c, gemäß den Begriffsbestimmungen in Tabelle 1,
- RD10: *echA1,* Rv0223c, gemäß den Begriffsbestimmungen in Tabelle 1,
- RvD1: RvD1-ORF1, RvD1-ORF2, RvD1-Rv2024c, wobei die Nukleotidsequenz von RvD1 unter der Nr. Y18605 bei der EMBL-Datenbank eingereicht wurde,
- RvD2: RvD2-plcD, RvD2-ORF1, RvD2-ORF2, RvD2-ORF3, RvD2-Rv1758, wobei die Nukleotidsequenz von RvD2 unter der Nr. Y18606 bei der EMBL-Datenbank eingereicht wurde.


### LISTAGE DE SÉQUENCE

<110> INSTITUT PASTEUR
<120> Séquences délétées chez M. bovis BCG/M. bovis ou M. tuberculosis, procédé de détection des mycobactéries utilisant ces séquences et vaccins.
<130> D18014
<160> 38
<170> PatentIn Vers. 2.0
<210> 1
   <211> 24
   <212> ADN
   <213> Mycobacterium tuberculosis
<220>
   <223> Y277-32F
<400> 1
   gacatgtacg agagacggca tgag 24
<210> 2
   <211> 21
   <212> ADN
   <213> Mycobacterium tuberculosis
<220>
   <223> Y277-32R
<400> 2
   aatccaacac gcagcaacca g 21
<210> 3
   <211> 20
   <212> ADN
   <213> Mycobacterium tuberculosis
<220>
   <223> plcC-B.5P
<400> 3
   ggattcctgg actggcgttg 20
<210> 4
   <211> 19
   <212> ADN
   <213> Mycobacterium tuberculosis
<220>
   <223> plcC-B.3P
<400> 4
   cccacccaag aaaccgcac 19
<210> 5
   <211> 20
   <212> ADN
   <213> Mycobacterium tuberculosis
<220>
   <223> Y78-dell
<400> 5
   aacaaaatcg cctcgtcgcc 20
<210> 6
   <211> 24
   <212> ADN
   <213> Mycobacterium tuberculosis
<220>
   <223> Y78-del2
<400> 6
   aacctgtatt cgtcgttgct gacc 24
<210> 7
   <211> 20
   <212> ADN
   <213> Mycobacterium tuberculosis
<220>
   <223> Rv420-flankl.F
<400> 7
   tggtaatcgt ggccgacaag 20
<210> 8
   <211> 19
   <212> ADN
   <213> Mycobacterium tuberculosis
<220>
   <223> RV420-flank2.R
<400> 8
   tcttgcggcc caatgaatc 19
<210> 9
   <211> 22
   <212> ADN
   <213> Mycobacterium tuberculosis
<220>
   <223> RD8-ephA.F
<400> 9
   ggtgtgattt ggtgagacga tg 22
<210> 10
   <211> 23
   <212> ADN
   <213> Mycobacterium tuberculosis
<220>
   <223> RD8-ephA.R
<400> 10
   agttcctcct gactaatcca ggc 23
<210> 11
   <211> 19
   <212> ADN
   <213> Mycobacterium tuberculosis
<220>
   <223> TB2329.5F
<400> 11
   tctgcccgtc gtgcgcgaa 19
<210> 12
   <211> 19
   <212> ADN
   <213> Mycobacterium tuberculosis
<220>
   <223> TB2332.5R
<400> 12
   cagtggctcg gcacgcaca 19
<210> 13
   <211> 22
   <212> ADN
   <213> Mycobacterium tuberculosis
<220>
   <223> RD10-264F
<400> 13
   tcgcgaaaga ggtcatctaa ac 22
<210> 14
   <211> 20
   <212> ADN
   <213> Mycobacterium tuberculosis
<220>
   <223> RD10-267R
<400> 14
   agatgctcaa gccgtgcacc 20
<210> 15
   <211> 22
   <212> ADN
   <213> Mycobacterium bovis
<220>
   <223> TBoli2268469.F
<400> 15
   cgcgccacaa acgtactatc tc 22
<210> 16
   <211> 20
   <212> ADN
   <213> Mycobacterium bovis
<220>
   <223> TBoli2269064.R
<400> 16
   agtttcaccg gctgtcgttc 20
<210> 17
   <211> 22
   <212> ADN
   <213> Mycobacterium bovis
<220>
   <223> Y28-IS6110B.5'
<400> 17
   cccacaccgc aggattggca ag 22
<210> 18
   <211> 24
   <212> ADN
   <213> Mycobacterium bovis
<220>
   <223> Y28-RHS.2
<400> 18
   atcgagtgca tgaacgcaac cgag 24
<210> 19
   <211> 21
   <212> ADN
   <213> Mycobacterium bovis BCG
<220>
   <223> TB16.0F
<400> 19
   gagccaacga tgatgatgac c 21
<210> 20
   <211> 19
   <212> ADN
   <213> Mycobacterium bovis BCG
<220>
   <223> TB16.5F
<400> 20
   ggtcacggtc ggtgtcgtc 19
<210> 21
   <211> 20
   <212> ADN
   <213> Mycobacterium bovis BCG
<220>
   <223> TB4398.7R
<400> 21
   cagaactgca ggggtggtac 20
<210> 22
   <211> 21
   <212> ADN
   <213> Mycobacterium bovis BCG
<220>
   <223> TB3689.5
<400> 22
   ctagttgttc agccgcgtct t 21
<210> 23
   <211> 19
   <212> ADN
   <213> Mycobacterium bovis BCG
<220>
   <223> TB3591.0R
<400> 23
   accggggtgt cggccagtt 19
<210> 24
   <211> 19
   <212> ADN
   <213> Mycobacterium bovis BCG
<220>
   <223> TB3689.9F
<400> 24
   tcgcggccac cgtgcgtaa 19
<210> 25
   <211> 20
   <212> ADN
   <213> Mycobacterium bovis BCG
<220>
   <223> TB3591.5R
<400> 25
   ggcgcctatg actgataccc 20
<210> 26
   <211> 19
   <212> ADN
   <213> Mycobacterium bovis BCG
<220>
   <223> TB3608.0F
<400> 26
   gaacagggtc gcggagtct 19
<210> 27
   <211> 20
   <212> ADN
   <213> Mycobacterium bovis BCG
<220>
   <223> TB3672.0R
<400> 27
   tcgaggaggt cgagtcctgt 20
<210> 28
   <211> 20
   <212> ADN
   <213> Mycobacterium bovis BCG
<220>
   <223> TB3671.7R
<400> 28
   gggttcatga ggtgctaggg 20
<210> 29
   <211> 22
   <212> ADN
   <213> Mycobacterium bovis BCG
<220>
   <223> RvD5-intF
<400> 29
   gggttcacgt tcattactgt tc 22
<210> 30
   <211> 20
   <212> ADN
   <213> Mycobacterium bovis BCG
<220>
   <223> RvD5-intR
<400> 30
   cctgcgctta tctctagcgg 20
<210> 31
   <211> 19
   <212> ADN
   <213> Mycobacterium bovis BCG
<220>
   <223> TB4411.0F
<400> 31
   ccggccactc actgccttc 19
<210> 32
   <211> 20
   <212> ADN
   <213> Mycobacterium bovis BCG
<220>
   <223> TB0.3R
<400> 32
   acggtagtgt cgtcggcttc 20
<210> 33
   <211> 21
   <212> ADN
   <213> Mycobacterium bovis BCG
<220>
   <223> TB3675.0F
<400> 33
   ccaacaccgt caactactcg a 21
<210> 34
   <211> 20
   <212> ADN
   <213> Mycobacterium bovis BCG
<220>
   <223> TB3675.5R
<400> 34
   atcgcagaac tccggcgaca 20
<210> 35
   <211> 19
   <212> ADN
   <213> Mycobacterium bovis BCG
<220>
   <223> TB1.2F
<400> 35
   cgatctgatc gccgacgcc 19
<210> 36
   <211> 19
   <212> ADN
   <213> Mycobacterium bovis BCG
<220>
   <223> TB1.5F
<400> 36
   tccgtcagcg ctccaagcg 19
<210> 37
   <211> 20
   <212> ADN
   <213> Mycobacterium bovis BCG
<220>
   <223> TB1.8F
<400> 37
   gtccccaaac tgcacaccct 20
<210> 38
   <211> 21
   <212> ADN
   <213> Mycobacterium bovis BCG
<220>
   <223> TB2.2R
<400> 38
   aatccggaaa tcgtcagacc g 21